# EUROPEAN PATENT SPECIFICATION

(11) **EP 3 071 691 B1**
(45) Date of publication and mention of the grant of the patent: **23.10.2019**
(21) Application number: 14812038.9
(22) Date of filing: 13.11.2014
(51) Int. Cl.: C12N 9/26, C11D 3/386

(54) **VARIANT ALPHA-AMYLASES HAVING REDUCED SUSCEPTIBILITY TO PROTEASE CLEAVAGE, AND METHODS OF USE, THEREOF**
VARIANTE ALPHA-AMYLASEN MIT VERRINGERTER SUSZEPTIBILITÄT GEGENÜBER PROTEASESPALTUNG UND VERFAHREN ZUR VERWENDUNG DAVON
VARIANTS D'ALPHA-AMYLASES AYANT UNE SENSIBILITÉ RÉDUITE AU CLIVAGE PROTÉASIQUE, ET LEURS PROCÉDÉS D'UTILISATION

(30) Priority: 20.11.2013 US 201361906617 P
(43) Date of publication of application: 28.09.2016
(73) Proprietor: Danisco US Inc., Palo Alto, California 94304 (US)
(72) Inventor: FINAN, Dina, Palo Alto, California 94304 (US)
(74) Representative: Mewburn Ellis LLP
(86) International application number: PCT/US2014/065522
(87) International publication number: WO 2015/077126

(56) References cited:
- WO-A1-2014/099523
- WO-A2-2009/061379
- REDDY N S ET AL: "AN OVERVIEW OF THE MICROBIAL ALPHA-AMYLASE FAMILY", AFRICAN JOURNAL OF BIOTECHNOLOGY, ACADEMIC PRESS, US, vol. 2, no. 12, 1 December 2003 (2003-12-01), pages 645-648, XP008043284, ISSN: 1684-5315
- DATABASE UniProt [Online] 2 November 2010 (2010-11-02), Lucas S et al.: "SubName: Full=Glucan 1,4-alpha-maltohexaosidase {ECO:0000313|EMBL:EFM12522.1}; EC=3.2.1.98 {ECO:0000313|EMBL:EFM12522.1};", retrieved from EBI accession no. UNIPROT:E0I4J1 Database accession no. E0I4J1

## Description

### FIELD OF THE INVENTION

### CROSS REFERENCE TO RELATED APPLICATIONS

This application claims benefit of priority from USSN 61/906,617, filed 20 November 2013.

Disclosed are compositions and methods relating to variant α-amylase enzymes comprising mutations that reduce their susceptibility to proteolytic digestion and improve their perfomance. The variants α-amylases are particularly useful in application that require α-amylases and proteases to be present in the same solution.

### BACKGROUND

Starch consists of a mixture of amylose (15-30% w/w) and amylopectin (70-85% w/w). Amylose consists of linear chains of α-1,4-linked glucose units having a molecular weight (MW) from about 60,000 to about 800,000. Amylopectin is a branched polymer containing α-1,6 branch points every 24-30 glucose units; its MW may be as high as 100 million.

α-amylases hydrolyze starch, glycogen, and related polysaccharides by cleaving internal α-1,4-glucosidic bonds at random. α-amylases, particularly from *Bacilli,* have been used for a variety of different purposes, including starch liquefaction and saccharification, textile desizing, starch modification in the paper and pulp industry, brewing, baking, production of syrups for the food industry, production of feedstocks for fermentation processes, and in animal feed to increase digestability. α-amylases have also been used to remove starchy soils and stains during dishwashing and laundry washing.

In some applications, α-amylases are used in combination with other enzymes, including proteases. While some α-amylases are relatively stable in the presence of proteases, others are susceptible to proteolytic cleavage and are quickly inactivated in the presence of protease. Accordingly, many α-amylases that have excellent starch hydrolysis activity cannot be used in the presence of protease.

### SUMMARY

The present compositions and methods relate to variant α-amylase enzymes that have reduced susceptibility to proteolytic cleavage and improved performance, and methods of use, thereof. Aspects and embodiments of the present compositions and methods are summarized in the following separately-numbered paragraphs:
1. In a first aspect, a method for reducing the susceptibility of an α-amylase having a TIM barrel structure to proteolytic cleavage is provided, comprising substituting a non-canonical, surface-exposed amino acid residue present in the loop linking the 7^{th} strand of the β-barrel and the 7^{th} helix to a different amino acid residue to produce a variant α-amylase, wherein the variant α-amylase has reduced susceptibility to cleavage in the loop linking the 7^{th} strand of the β-barrel and the 7^{th} helix by protease compared to the parent α-amylase, and wherein SEQ ID NO: 3 is used for position numbering.
2. In some embodiments of the method of paragraph 1, the non-canonical, surface-exposed amino acid residue is present at a position corresponding to position 333 in the parent α-amylase, wherein SEQ ID NO: 3 is used for position numbering.
3. In some embodiments, the method of paragraph 2 further comprises substituting the amino acid residue present at a position corresponding to position 335 in the parent α-amylase to a different amino acid residue, wherein SEQ ID NO: 3 is used for position numbering.
4. In some embodiments of the method of preceding paragraphs 2 or 3, the parent α-amylase has an amino acid residue other than glycine or glutamine at the position corresponding to position 333.
5. In some embodiments of the method of any of preceding paragraphs 2-4, the parent α-amylase has a threonine at the position corresponding to position 333.
6. In some embodiments of the method of any of preceding paragraphs 2-5, the amino acid residue at the position corresponding to position 333 is substituted to a glycine.
7. In some embodiments of the method of any of preceding paragraphs 3-6, the parent α-amylase has an amino acid residue other than serine at the position corresponding to position 335.
8. In some embodiments of the method of any of preceding paragraphs 3-7, the amino acid residue at the position corresponding to position 335 is substituted to a serine.
9. In some embodiments, the method of any of the preceding paragraphs further comprises making one or more of the following mutations in the parent α-amylase: (i) deleting of one or more amino acid residues at positions corresponding to positions 177, 178, 179, and 180; (ii) substituting the amino acid residue present at a position corresponding to position 186; or (iii) substituting the amino acid residue present at a position corresponding to position 472; wherein the resulting variant has increased detergent stability and/or increased cleaning performance in a detergent composition compared to the parent, and wherein SEQ ID NO: 3 is used for position numbering.
10. In some embodiments, the method of any of the preceding paragraphs further comprises making one or more of the following mutations in the parent α-amylase: (i) deleting the amino acid residues at positions corresponding to positions 177 and 178; (ii) substituting the amino acid residue present at a position corresponding to position 186 to a proline; (iii) substituting the amino acid residue present at a position corresponding to position 472 to an arginine or a lysine; wherein the resulting variant has increased detergent stability and/or increased cleaning performance in a detergent composition compared to the parent, and wherein SEQ ID NO: 3 is used for position numbering.
11. In some embodiments, the method of any of paragraphs 1-10 further comprises making one or more mutations at positions corresponding to N125, F152, N205, and G473, wherein SEQ ID NO: 3 is used for position numbering.
12. In some embodiments of the method of paragraph 11, the variants comprise a combination of mutations selected from the group consisting of:
   (i) N125Y + E186P + T333G + A335S + Q337E + G472K;
   (ii) N125Y + F152W + E186P + T333G + A335S + Q337E + G472K;
   (iii) N125Y + F152W + E186P + T333G + A335S + Q337E + G472R + G473R;
   (iv) N125Y + F152W + E186P + N205D + T333G + A335S + Q337E + G472K;
   (v) N125Y + E186P + T333G + A335S + G472K;
   (vi) N125Y + F152W + E186P + T333G + A335S + G472K;
   (vii) N125Y + F152W + E186P + T333G + A335S + G472R + G473R;
   (viii) N125Y + F152W + E186P + N205D + T333G + A335S + G472K;
   (ix) N125Y + E186P + T333G + G472K;
   (x) N125Y + F152W + E186P + T333G + G472K;
   (xi) N125Y + F152W + E186P + T333G + G472R + G473R; and
   (xii) N125Y + F152W + E186P + N205D + T333G + G472K.
13. In some embodiments of the method of any of the preceding paragraphs, the parent or the variant α-amylase has an amino acid sequence having at least 70%, at least 80%, or at least 90% amino acid sequence identity to SEQ ID NO: 3.
14. In another aspect, a variant α-amylase prepared by the method of any of the preceding paragraphs is provided.
15. In another aspect, a variant of a parent α-amylase is provided, wherein the variant comprises a substitution of the amino acid residue present at a position corresponding to position 333 in the parent α-amylase to a different amino acid residue, wherein the variant α-amylase has reduced susceptibility to cleavage in the loop linking the 7^{th} strand of the β-barrel and the 7^{th} helix by a protease compared to the parent α-amylase, and wherein SEQ ID NO: 3 is used for position numbering.
16. In some embodiments, the variant of paragraph 15 further comprises a substitution of the amino acid residue present in the parent α-amylase at a position corresponding to position 335 to a different amino acid residue to further reduce the susceptibility to cleavage, wherein SEQ ID NO: 3 is used for position numbering.
17. In some embodiments of the variant of preceding paragraphs 15 or 16, the parent α-amylase has an amino acid residue other than glycine or glutamine at the position corresponding to position 333.
18. In some embodiments of the variant of any of preceding paragraphs 15-17, the parent α-amylase has a threonine at the position corresponding to position 333.
19. In some embodiments of the variant of any of preceding paragraphs 15-18, the variant α-amylase has a substitution to glycine at the position corresponding to position 333.
20. In some embodiments of the variant of any of preceding paragraphs 15-19, the parent α-amylase has an amino acid residue other than serine at the position corresponding to position 335.
21. In some embodiments of the variant of any of preceding paragraphs 16-20, the variant α-amylase has a substitution to serine at the position corresponding to position 335.
22. In some embodiments, the variant of any of preceding paragraphs 15-21 comprises one or more of the following mutations in relation to the parent: (i) the deletion of one or more amino acid residues at positions corresponding to positions 177, 178, 179, and 180; (ii) the substitution of the amino acid residue present at a position corresponding to position 186; and (iii) the substitution of the amino acid residue present at a position corresponding to position 472; wherein the variant has increased detergent stability and/or increased cleaning performance in a detergent composition compared to the parent, and wherein the position numbering refers to SEQ ID NO: 3.
23. In some embodiments, the variant of any of preceding paragraphs 15-22 further comprises one or more of the following mutations in relation to the parent: (i) the deletion of the amino acid residues at positions corresponding to positions 177 and 178; (ii) the substitution of the amino acid residue present at a position corresponding to position 186 to a proline; (iii) the substitution of the amino acid residue present at a position corresponding to position 472 to arginine or lysine; wherein the variant has increased detergent stability and/or increased cleaning performance in a detergent composition compared to the parent, and wherein the position numbering refers to SEQ ID NO: 3.
24. In some embodiments, the variant of any of preceding paragraphs 15-23 further comprises a mutation at a position selected from the group consisting of N125, F152, N205, and G473.
25. In some embodiments, the variant of any of preceding paragraphs 15-24 comprises the deletion of one or more amino acid residues at positions corresponding to positions 177, 178, 179, and 180, and further comprises a combination of mutations selected from the group consisting of:
   (i) N125Y + E186P + T333G + A335S + Q337E + G472K;
   (ii) N125Y + F152W + E186P + T333G + A335S + Q337E + G472K;
   (iii) N125Y + F152W + E186P + T333G + A335S + Q337E + G472R + G473R;
   (iv) N125Y + F152W + E186P + N205D + T333G + A335S + Q337E + G472K;
   (v) N125Y + E186P + T333G + A335S + G472K;
   (vi) N125Y + F152W + E186P + T333G + A335S + G472K;
   (vii) N125Y + F152W + E186P + T333G + A335S + G472R + G473R;
   (viii) N125Y + F152W + E186P + N205D + T333G + A335S + G472K;
   (ix) N125Y + E186P + T333G + G472K;
   (x) N125Y + F152W + E186P + T333G + G472K;
   (xi) N125Y + F152W + E186P + T333G + G472R + G473R; and
   (xii) N125Y + F152W + E186P + N205D + T333G + G472K.
26. In some embodiments of the variant of any of preceding paragraphs 15-24, the parent or the variant has an amino acid sequence having at least 70%, at least 80%, or at least 90% amino acid sequence identity to SEQ ID NO: 3.
27. In another aspect, a composition comprising the α-amylase of any of paragraphs 14-26 is provided.
28. In some embodiments, the composition of paragraph 27 further comprises a surfactant.
29. In some embodiments, the composition of paragraph 27 or 28 is a laundry detergent, a laundry detergent additive, or a manual or automatic dishwashing detergent.
30. In some embodiments, the composition of any of paragraphs 27-29 further comprises one or more additional enzymes selected from the group consiting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase and an α-amylase other than PcuAmy1, or a variant thereof.
31. In some embodiments, the composition of paragraph 27 is for saccharifying a composition comprising starch, for SSF post liquefaction, or for direct SSF without prior liquefaction or for producing a fermented beverage or a baked food product.
32. In some embodiments, the composition of paragraph 27 or 28 is for textile desizing.
33. In another aspect, a recombinant polynucleotide encoding a polypeptide of any of paragraphs 14-26 is provided.
34. In some embodiments, the polynucleotide of paragraph 33 has at least 80% nucleic acid sequence identity to the polynucleotide of SEQ ID NOs: 1, or hybridizes under stringent conditions to a polynucleotide complementary to the polynucleotide of SEQ ID NOs: 1.
35. In another aspect, an expression vector comprising the polynucleotide of paragraph 33 or 34 is provided.
36. In another aspect, a host cell comprising the expression vector of paragraph 35 is provided
37. In another aspect, use of the α-amylase of any of paragraphs 16-26 in the production of a composition comprising glucose, in the production of a liquefied starch, in the production of a foodstuff or beverage, in cleaning starchy stains, or in textile desizing is provided.
38. In another aspect, a method for removing a starchy stain or soil from a surface is provided, comprising: contacting the surface with a composition comprising an effective amount of the variant α-amylase of any of paragraphs 16-26 and optionally a surfactant; and allowing the α-amylase to hydrolyze starch components present in the starchy stain to produce smaller starch-derived molecules that dissolve in aqueous solution; thereby removing the starchy stain from the surface; wherein the composition further optionally comprises at least one additional enzymes selected from the group consiting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase, and an α-amylase other than PcuAmy1, or a variant thereof.
39. In another aspect, a method for desizing a textile is provided, comprising: contacting a sized textile with an effective amount of a variant α-amylase of any of paragraphs 14-26; and allowing the α-amylase to hydrolyze starch components in the size to produce smaller starch-derived molecules that dissolve in aqueous solution; thereby removing the size from the textile.
40. In another aspect, a method for saccharifying a composition comprising starch to produce a composition comprising glucose is provided, the method comprising: contacting the composition comprising starch with effective amount of a variant α-amylase of any of paragraphs 14-26; and saccharifying the composition comprising starch to produce the composition comprising glucose; wherein the α-amylase catalyzes the saccharification of the starch solution to glucose.
41. In some embodiments, of the method of paragraph 40, the composition comprising starch comprises liquefied starch, gelatinized starch, or granular starch.
42. In another aspect, a method for preparing a foodstuff or beverage is provided, comprising: contacting a foodstuff or beverage comprising starch with an α-amylase of any of paragraphs 14-26; and allowing the α-amylase to hydrolyze the starch to produce smaller starch-derived molecules; wherein the method further optionally comprises contacting the foodstuff or beverage with glucoamylase, hexokinase, xylanase, glucose isomerase, xylose isomerase, phosphatase, phytase, pullulanase, β-amylase, α-amylase that is not the variant α-amylase, protease, cellulase, hemicellulase, lipase, cutinase, isoamylase, redox enzyme, esterase, transferase, pectinase, α-glucosidase, beta-glucosidase, or a combination thereof.
43. In some embodiments of the method of any one of paragraphs 40-42, the α-amylase is expressed and secreted by a host cell.
44. In some embodiments of the method of paragraph 43, the composition comprising starch is contacted with the host cell.
45. In some embodiments of the method of paragraph 43 or 44, the host cell further expresses and secretes a glucoamylase or other enzyme.
46. In some embodiments of the method of any one of paragraphs 43-45, the host cell is capable of fermenting the composition.

These and other aspects and embodiments of the compositions and methods will be apparent from the present description and drawings.

### BRIEF DESCRIPTION OF THE DRAWINGS

Figure 1A-1C is an amino acid sequence alignment of a number of α-amylases described for use in industrial applications. The alignment was created using Clustal W with default parameters.
Figure 2 is an image of an SDS/PAGE gel showing the cleavage of PcuAmy1-v1 in the presence of increasing amounts of GG36 protease. The letters on the right side of the gel indicate (A) intact full-length PcuAmy1-v1, (B) a first cleavage product of PcuAmy1-v1, (C) GG36 protease, (D) a contaminant in the GG36 protein preparation, and (E) a second cleavage product of PcuAmy1-v1.
Figure 3 is a graph showing the residual α-amylase activty of PcuAmy1 and several engineered variants following incubation with GG36 protease.
Figure 4 is an image of an SDS/PAGE gel showing the proteolytic cleavage of PcuAmy1 and several engineered variants following incubation with GG36 protease.
Figure 5 is a graph showing the stability of PcuAmy1-v1 and several engineered variants following incubation with GG36 protease in MIFA Total detergent for up to 14 days at 37°C.
Figure 6 is a graph showing the stability of PcuAmy1-v1 and several engineered variants following incubation with GG36 protease in MIFA Total detergent for 3 or 14 days at 37°C.
Figure 7 is a graph showing the stability of PcuAmy1-v1 and several engineered variants following incubation with GG36 protease in Unilever Omo detergent for up to 14 days at 37°C.
Figure 8 is a graph showing the stability of PcuAmy1-v1 and several engineered variants following incubation with GG36 protease in Unilever Omo detergent for 3 or 14 days at 37°C.
Figure 9 is a graph showing the dose-dependent cleaning perfomance PcuAmy1-3B and PcuAmy1-3L in Persil Universal Gel Gold detergent compared to two commercial benchmarks.
Figure 10 is a graph showing the thermal stability of PcuAmy1-3B and PcuAmy1-3L in Persil Universal Gel Gold detergent compared to two commercial benchmarks.

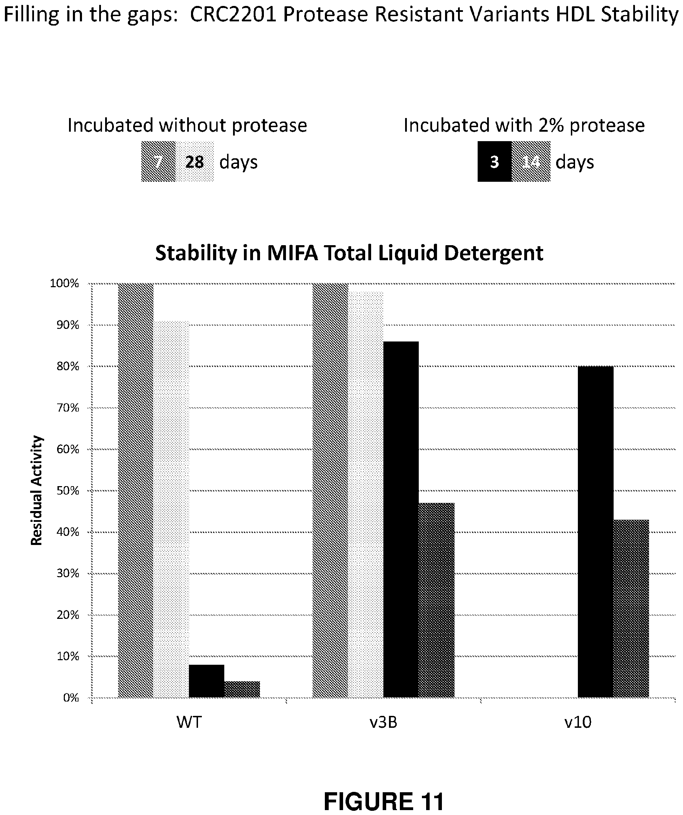

Figure 11 is a graph showing the stability of PcuAmy1-v1 and several engineered variants following incubation with GG36 protease in MIFA Total detergent for 3 or 14 days at 37°C.
Figure 12 is a graph showing the relative cleaning performance of PcuAmy1 variants v1, v6, v8, and v16 compared to STAINZYME® and ACE-QK in buffer at pH 8.0. Enzyme doses are noted on the x-axis.
Figure 13 is a graph showing the relative thermal stability of PcuAmy1 variants v1, v6, v8, and v16 compared to STAINZYME® in buffer at the temperatures indicated. 5 ppm of PcuAmy1 variants and 10 ppm of STAINZYME® were used.

### DETAILED DESCRIPTION

Described are compositions and methods relating to variant α-amylase enzymes that have reduced susceptibility to proteolytic cleavage. Exemplary applications for the variant α-amylases are starch liquefaction and saccharification, cleaning starchy stains in laundry and dish washing, desizing in textile processing, baking and brewing, and animal feed, particularly where proteases are known or suspected of being present. These and other aspects of the compositions and methods are described in detail, below.

Prior to describing the various aspects and embodiments of the present compositions and methods, the following definitions and abbreviations are described.

### 1. Definitions and Abbreviations

In accordance with this detailed description, the following abbreviations and definitions apply. Note that the singular forms "a," "an," and "the" include plural referents unless the context clearly dictates otherwise. Thus, for example, reference to "an enzyme" includes a plurality of such enzymes, and reference to "the dosage" includes reference to one or more dosages and equivalents thereof known to those skilled in the art, and so forth.

The present document is organized into a number of sections for ease of reading; however, the reader will appreciate that statements made in one section may apply to other sections. In this manner, the headings used for different sections of the disclosure should not be construed as limiting.

Unless defined otherwise, all technical and scientific terms used herein have the same meaning as commonly understood by one of ordinary skill in the art. The following terms are provided below.

### 1.1. Abbreviations and Acronyms

The following abbreviations/acronyms have the following meanings unless otherwise specified:
- ABTS: 2,2-azino-bis-3-ethylbenzothiazoline-6-sulfonic acid
- AE or AEO: alcohol ethoxylate
- AES or AEOS: alcohol ethoxysulfate
- AkAA: *Aspergillus kawachii* α-amylase
- AnGA: *Aspergillus niger* glucoamylase
- AOS: α-olefinsulfonate
- AS: alkyl sulfate
- cDNA: complementary DNA
- CMC: carboxymethylcellulose
- DE: dextrose equivalent
- DNA: deoxyribonucleic acid
- DPn: degree of saccharide polymerization having n subunits
- ds or DS: dry solids
- DTMPA: diethylenetriaminepentaacetic acid
- EC: Enzyme Commission
- EDTA: ethylenediaminetetraacetic acid
- EO: ethylene oxide (polymer fragment)
- EOF: End of Fermentation
- GA: glucoamylase
- GAU/g ds: glucoamylase activity unit/gram dry solids
- HFCS: high fructose corn syrup
- HgGA: *Humicola grisea* glucoamylase
- IPTG: isopropyl β-D-thiogalactoside
- IRS: insoluble residual starch
- kDa: kiloDalton
- LAS: linear alkylbenzenesulfonate
- LAT, BLA: *B. licheniformis* amylase
- MW: molecular weight
- MWU: modified Wohlgemuth unit; 1.6x10⁻⁵ mg/MWU = unit of activity
- NCBI: National Center for Biotechnology Information
- NOBS: nonanoyloxybenzenesulfonate
- NTA: nitriloacetic acid
- OxAm: Purastar HPAM 5000L (Danisco US Inc.)
- PAHBAH: p-hydroxybenzoic acid hydrazide
- PEG: polyethyleneglycol
- pI: isoelectric point
- PI: performance index
- ppm: parts per million, *e.g.,* µg protein per gram dry solid
- PVA: poly(vinyl alcohol)
- PVP: poly(vinylpyrrolidone)
- RCF: relative centrifugal/centripetal force (*i.e*., x gravity)
- RNA: ribonucleic acid
- SAS: alkanesulfonate
- SDS-PAGE: sodium dodecyl sulfate polyacrylamide gel electrophoresis
- SSF: simultaneous saccharification and fermentation
- SSU/g solid: soluble starch unit/gram dry solids
- sp.: species
- TAED: tetraacetylethylenediamine
- Tm: melting temperature
- TrGA: *Trichoderma reesei* glucoamylase
- w/v: weight/volume
- w/w: weight/weight
- v/v: volume/volume
- wt%: weight percent
- °C: degrees Centigrade
- H₂O: water
- dH₂O or DI: deionized water
- dIH₂O: deionized water, Milli-Q filtration
- g or gm: grams
- µg: micrograms
- mg: milligrams
- kg: kilograms
- µL and µl: microliters
- mL and ml: milliliters
- mm: millimeters
- µm: micrometer
- M: molar
- mM: millimolar
- µM: micromolar
- U: units
- sec: seconds
- min(s): minute/minutes
- hr(s): hour/hours
- DO: dissolved oxygen
- Ncm: Newton centimeter
- ETOH: ethanol
- eq.: equivalents
- N: normal
- MWCO: molecular weight cut-off
- SSRL: Stanford Synchrotron Radiation Lightsource
- PDB: Protein Database
- CAZy: Carbohydrate-Active Enzymes database
- Tris-HCl: tris(hydroxymethyl)aminomethane hydrochloride
- HEPES: 4-(2-hydroxyethyl)-1-piperazineethanesulfonic acid
- mS/cm: milli-Siemens/cm
- CV: column volumes

### 1.2. Definitions

The terms "amylase" or "amylolytic enzyme" refer to an enzyme that is, among other things, capable of catalyzing the degradation of starch. α-amylases are hydrolases that cleave the α-D-(1→4) O-glycosidic linkages in starch. Generally, α-amylases (EC 3.2.1.1; α-D-(1→4)-glucan glucanohydrolase) are defined as endo-acting enzymes cleaving α-D-(1→4) O-glycosidic linkages within the starch molecule in a random fashion yielding polysaccharides containing three or more (1-4)-α-linked D-glucose units. In contrast, the exo-acting amylolytic enzymes, such as β-amylases (EC 3.2.1.2; α-D-(1→4)-glucan maltohydrolase) and some product-specific amylases like maltogenic α-amylase (EC 3.2.1.133) cleave the polysaccharide molecule from the non-reducing end of the substrate. β-amylases, α-glucosidases (EC 3.2.1.20; α-D-glucoside glucohydrolase), glucoamylase (EC 3.2.1.3; α-D-(1→4)-glucan glucohydrolase), and product-specific amylases like the maltotetraosidases (EC 3.2.1.60) and the maltohexaosidases (EC 3.2.1.98) can produce malto-oligosaccharides of a specific length or enriched syrups of specific maltooligosaccharides.

The terms "protease" and "proteinase" refer to an enzyme protein that has the ability to perform "proteolysis" or "proteolytic cleavage" which refers to hydrolysis of peptide bonds that link amino acids together in a peptide or polypeptide chain forming the protein. This activity of a protease as a protein-digesting enzyme is referred to as "proteolytic activity." Many well known procedures exist for measuring proteolytic activity (*See e.g*., Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (*See e.g*., WO 99/34011 and U.S. Pat. No. 6,376,450. The pNA assay (*See e.g*., Del Mar et al., Anal. Biochem. 99:316-320 [1979]) also finds use in determining the active enzyme concentration for fractions collected during gradient elution. This assay measures the rate at which p-nitroaniline is released as the enzyme hydrolyzes a soluble synthetic peptide substrate, such as succinyl-alanine-alanine-proline-phenylalanine-p-nitroanilide (suc-AAPF-pNA), and cleavage occurs between the C-terminal amino acid (phenylalanine) and the p-NA, causing the production of yellow color from the hydrolysis reaction, which is measured at 410 nm on a spectrophotometer and is proportional to the active enzyme concentration. Measurement of the color change allows calculation of the rate of the reaction. In addition, absorbance measurements at 280 nanometers (nm) can be used to determine the total protein concentration. The active enzyme/total protein ratio gives the enzyme purity when a reference standard is used.

The terms "serine protease" refers to enzymes that cleave peptide bonds in proteins, in which enzymes serine serves as the nucleophilic amino acid at the enzyme active site. Serine proteases fall into two broad categories based on their structure: chymotrypsin-like (trypsin-like) or subtilisin-like. Most commonly used laundry and dishwashing detergents are serine protease, particularly subtlisins.

The term "TIM barrel" refers to a three dimensional polypeptide structure that include eight α-helices and eight parallel β-strands that alternate along the peptide backbone.

The term "surface-exposed" with respect to an amino acid residue in a polypeptide refers to a residue that is on the exterior surface of a polypeptide when the polypeptide is intact and properly folded, *i.e*., not denatured or fragmented. In the case of an α-amylase, the structure is refered to as a TIM barrel.

The term "non-canonical" with reference to an amino acid residue in a polypeptide refers to a residue that is not normally found at a given position based on amino acid sequence alignments of similar molecules using Clustal W with default parameter. In some cases, the particular residue is found at a given position in only 1 in 10, 1 in 20, 1 in 30, 1 in 50, or even 1 in 100 similar molecules.

"Enzyme units" herein refer to the amount of product formed per time under the specified conditions of the assay. For example, a "glucoamylase activity unit" (GAU) is defined as the amount of enzyme that produces 1 g of glucose per hour from soluble starch substrate (4% DS) at 60°C, pH 4.2. A "soluble starch unit" (SSU) is the amount of enzyme that produces 1 mg of glucose per minute from soluble starch substrate (4% DS) at pH 4.5, 50°C. DS refers to "dry solids."

The term "starch" refers to any material comprised of the complex polysaccharide carbohydrates of plants, comprised of amylose and amylopectin with the formula (C₆H₁₀O₅)ₓ, wherein X can be any number. The term includes plant-based materials such as grains, cereal, grasses, tubers and roots, and more specifically materials obtained from wheat, barley, corn, rye, rice, sorghum, brans, cassava, millet, milo, potato, sweet potato, and tapioca. The term "starch" includes granular starch. The term "granular starch" refers to raw, *i.e*., uncooked starch, *e.g*., starch that has not been subject to gelatinization.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

Reference to the wild-type polypeptide is understood to include the mature form of the polypeptide. A "mature" polypeptide or variant, thereof, is one in which a signal sequence is absent, for example, cleaved from an immature form of the polypeptide during or following expression of the polypeptide.

The term "variant," with respect to a polypeptide, refers to a polypeptide that differs from a specified wild-type, parental, or reference polypeptide in that it includes one or more man-made substitutions, insertions, or deletions of an amino acid. The identity of the wild-type, parental, or reference polypeptide or polynucleotide will be apparent from context. Mutations are indicated by standard nomenclature, *e*.*g*., X₁NX₂, where X₁ is the starting amino acid residue, N is the amino acid position number, and X₂ is the final amino acid residue. Deletions are represented by "del" or Δ, followed by one or more amino acid position numbers. Combinations of mutations in a single α-amylase polypeptide, as in the case of "combinatorial variants," may be indicated by separating individual mutations with dashes (-), by separating individual mutations with plus signs (+),by separating individual mutations with slashes (/), or by appropriate textual context, without distinction.

In the case of the present α-amylases, "activity" refers to α-amylase activity, which can be measured as described, herein.

The term "recombinant," when used in reference to a subject cell, nucleic acid, protein or vector, indicates that the subject has been modified from its native state. Thus, for example, recombinant cells express genes that are not found within the native (non-recombinant) form of the cell, or express native genes at different levels or under different conditions than found in nature. Recombinant nucleic acids differ from a native sequence by one or more nucleotides and/or are operably linked to heterologous sequences, *e*.*g*., a heterologous promoter in an expression vector. Recombinant proteins may differ from a native sequence by one or more amino acids and/or are fused with heterologous sequences. A vector comprising a nucleic acid encoding an amylase is a recombinant vector.

The terms "recovered," "isolated," and "separated," refer to a compound, protein (polypeptides), cell, nucleic acid, amino acid, or other specified material or component that is removed from at least one other material or component with which it is naturally associated as found in nature. An "isolated" polypeptides, thereof, includes, but is not limited to, a culture broth containing secreted polypeptide expressed in a heterologous host cell.

The term "purified" refers to material (*e*.*g*., an isolated polypeptide or polynucleotide) that is in a relatively pure state, *e.g*., at least about 90% pure, at least about 95% pure, at least about 98% pure, or even at least about 99% pure.

The term "enriched" refers to material (*e*.*g*., an isolated polypeptide or polynucleotide) that is in about 50% pure, at least about 60% pure, at least about 70% pure, or even at least about 70% pure.

The terms "thermostable" and "thermostability," with reference to an enzyme, refer to the ability of the enzyme to retain activity after exposure to an elevated temperature. The thermostability of an enzyme, such as an amylase enzyme, is measured by its half-life (t_{1/2}) given in minutes, hours, or days, during which half the enzyme activity is lost under defined conditions. The half-life may be calculated by measuring residual α-amylase activity following exposure to (*i.e*., challenge by) an elevated temperature.

A "pH range," with reference to an enzyme, refers to the range of pH values under which the enzyme exhibits catalytic activity.

The terms "pH stable" and "pH stability," with reference to an enzyme, relate to the ability of the enzyme to retain activity over a wide range of pH values for a predetermined period of time (*e.g*., 15 min., 30 min., 1 hour).

The term "amino acid sequence" is synonymous with the terms "polypeptide," "protein," and "peptide," and are used interchangeably. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e*., N→C).

The term "nucleic acid" encompasses DNA, RNA, heteroduplexes, and synthetic molecules capable of encoding a polypeptide. Nucleic acids may be single stranded or double stranded, and may be chemical modifications. The terms "nucleic acid" and "polynucleotide" are used interchangeably. Because the genetic code is degenerate, more than one codon may be used to encode a particular amino acid, and the present compositions and methods encompass nucleotide sequences that encode a particular amino acid sequence. Unless otherwise indicated, nucleic acid sequences are presented in 5'-to-3' orientation.

"Hybridization" refers to the process by which one strand of nucleic acid forms a duplex with, *i.e*., base pairs with, a complementary strand, as occurs during blot hybridization techniques and PCR techniques. Stringent hybridization conditions are exemplified by hybridization under the following conditions: 65°C and 0.1X SSC (where 1X SSC = 0.15 M NaCl, 0.015 M Na₃ citrate, pH 7.0). Hybridized, duplex nucleic acids are characterized by a melting temperature (Tₘ), where one half of the hybridized nucleic acids are unpaired with the complementary strand. Mismatched nucleotides within the duplex lower the Tₘ.

A "synthetic" molecule is produced by *in vitro* chemical or enzymatic synthesis rather than by an organism.

The terms "transformed," "stably transformed," and "transgenic," used with reference to a cell means that the cell contains a non-native (*e*.*g*., heterologous) nucleic acid sequence integrated into its genome or carried as an episome that is maintained through multiple generations.

The term "introduced" in the context of inserting a nucleic acid sequence into a cell, means "transfection", "transformation" or "transduction," as known in the art.

A "host strain" or "host cell" is an organism into which an expression vector, phage, virus, or other DNA construct, including a polynucleotide encoding a polypeptide of interest *(e.g.,* an amylase) has been introduced. Exemplary host strains are microorganism cells (*e.g*., bacteria, filamentous fungi, and yeast) capable of expressing the polypeptide of interest and/or fermenting saccharides. The term "host cell" includes protoplasts created from cells.

The term "heterologous" with reference to a polynucleotide or protein refers to a polynucleotide or protein that does not naturally occur in a host cell.

The term "endogenous" with reference to a polynucleotide or protein refers to a polynucleotide or protein that occurs naturally in the host cell.

The term "expression" refers to the process by which a polypeptide is produced based on a nucleic acid sequence. The process includes both transcription and translation.

A "selective marker" or "selectable marker" refers to a gene capable of being expressed in a host to facilitate selection of host cells carrying the gene. Examples of selectable markers include but are not limited to antimicrobials (*e*.*g*., hygromycin, bleomycin, or chloramphenicol) and/or genes that confer a metabolic advantage, such as a nutritional advantage on the host cell.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" refers to a DNA construct comprising a DNA sequence encoding a polypeptide of interest, which coding sequence is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

The term "operably linked" means that specified components are in a relationship (including but not limited to juxtaposition) permitting them to function in an intended manner. For example, a regulatory sequence is operably linked to a coding sequence such that expression of the coding sequence is under control of the regulatory sequences.

A "signal sequence" is a sequence of amino acids attached to the N-terminal portion of a protein, which facilitates the secretion of the protein outside the cell. The mature form of an extracellular protein lacks the signal sequence, which is cleaved off during the secretion process.

"Biologically active" refer to a sequence having a specified biological activity, such an enzymatic activity.

The term "specific activity" refers to the number of moles of substrate that can be converted to product by an enzyme or enzyme preparation per unit time under specific conditions. Specific activity is generally expressed as units (U)/mg of protein.

As used herein, "water hardness" is a measure of the minerals (*e*.*g*., calcium and magnesium) present in water.

As used herein, an "effective amount of amylase," or similar expressions, refers to an amount of amylase sufficient to produce a visible, or otherwise measurable amount of starch hydrolysis in an particular application. Starch hydrolysis may result in, *e.g*., a visible cleaning of fabrics or dishware, reduced viscosity of a starch slurry or mash, and the like.

A "swatch" is a piece of material such as a fabric that has a stain applied thereto. The material can be, for example, fabrics made of cotton, polyester or mixtures of natural and synthetic fibers. The swatch can further be paper, such as filter paper or nitrocellulose, or a piece of a hard material such as ceramic, metal, or glass. For amylases, the stain is starch based, but can include blood, milk, ink, grass, tea, wine, spinach, gravy, chocolate, egg, cheese, clay, pigment, oil, or mixtures of these compounds.

A "smaller swatch" is a section of the swatch that has been cut with a single hole punch device, or has been cut with a custom manufactured 96-hole punch device, where the pattern of the multi-hole punch is matched to standard 96-well microtiter plates, or the section has been otherwise removed from the swatch. The swatch can be of textile, paper, metal, or other suitable material. The smaller swatch can have the stain affixed either before or after it is placed into the well of a 24-, 48- or 96-well microtiter plate. The smaller swatch can also be made by applying a stain to a small piece of material. For example, the smaller swatch can be a stained piece of fabric 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived of to deliver simultaneously swatches to any format plate, including but not limited to 24-well, 48-well, and 96-well plates. In another conceivable method, the soiled test platform can be a bead made of metal, plastic, glass, ceramic, or another suitable material that is coated with the soil substrate. The one or more coated beads are then placed into wells of 96-, 48-, or 24-well plates or larger formats, containing suitable buffer and enzyme.

"A cultured cell material comprising an amylase" or similar language, refers to a cell lysate or supernatant (including media) that includes an amylase as a component. The cell material may be from a heterologous host that is grown in culture for the purpose of producing the amylase.

"Percent sequence identity" means that a particular sequence has at least a certain percentage of amino acid residues identical to those in a specified reference sequence, when aligned using the CLUSTAL W algorithm with default parameters. *See* Thompson *et al*. (1994) *Nucleic Acids Res.* 22:4673-4680. Default parameters for the CLUSTAL W algorithm are:

| | |
|---|---|
| Gap opening penalty: | 10.0 |
| Gap extension penalty: | 0.05 |
| Protein weight matrix: | BLOSUM series |
| DNA weight matrix: | IUB |
| Delay divergent sequences %: | 40 |
| Gap separation distance: | 8 |
| DNA transitions weight: | 0.50 |
| List hydrophilic residues: | GPSNDQEKR |
| Use negative matrix: | OFF |
| Toggle Residue specific penalties: | ON |
| Toggle hydrophilic penalties: | ON |
| Toggle end gap separation penalty | OFF. |

Deletions are counted as non-identical residues, compared to a reference sequence. Deletions occurring at either termini are included. For example, a variant 500-amino acid residue polypeptide with a deletion of five amino acid residues from the C-terminus would have a percent sequence identity of 99% (495/500 identical residues × 100) relative to the parent polypeptide. Such a variant would be encompassed by the language, "a variant having at least 99% sequence identity to the parent."

"Fused" polypeptide sequences are connected, *i.e*., operably linked, via a peptide bond between two subject polypeptide sequences.

The term "filamentous fungi" refers to all filamentous forms of the subdivision Eumycotina, particulary Pezizomycotina species.

The term "degree of polymerization" (DP) refers to the number (n) of anhydro-glucopyranose units in a given saccharide. Examples of DP1 are the monosaccharides glucose and fructose. Examples of DP2 are the disaccharides maltose and sucrose. The term "DE," or "dextrose equivalent," is defined as the percentage of reducing sugar, *i.e*., D-glucose, as a fraction of total carbohydrate in a syrup.

The term "dry solids content" (ds) refers to the total solids of a slurry in a dry weight percent basis. The term "slurry" refers to an aqueous mixture containing insoluble solids.

The phrase "simultaneous saccharification and fermentation (SSF)" refers to a process in the production of biochemicals in which a microbial organism, such as an ethanologenic microorganism, and at least one enzyme, such as an amylase, are present during the same process step. SSF includes the contemporaneous hydrolysis of starch substrates (granular, liquefied, or solubilized) to saccharides, including glucose, and the fermentation of the saccharides into alcohol or other biochemical or biomaterial in the same reactor vessel.

An "ethanologenic microorganism" refers to a microorganism with the ability to convert a sugar or oligosaccharide to ethanol.

The term "fermented beverage" refers to any beverage produced by a method comprising a fermentation process, such as a microbial fermentation, *e*.*g*., a bacterial and/or fungal fermentation. "Beer" is an example of such a fermented beverage, and the term "beer" is meant to comprise any fermented wort produced by fermentation/brewing of a starch-containing plant material. Often, beer is produced exclusively from malt or adjunct, or any combination of malt and adjunct.

The term "malt" refers to any malted cereal grain, such as malted barley or wheat.

The term "adjunct" refers to any starch and/or sugar containing plant material that is not malt, such as barley or wheat malt. Examples of adjuncts include common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, cassava and syrups, such as corn syrup, sugar cane syrup, inverted sugar syrup, barley and/or wheat syrups, and the like.

The term "mash" refers to an aqueous slurry of any starch and/or sugar containing plant material, such as grist, *e*.*g*., comprising crushed barley malt, crushed barley, and/or other adjunct or a combination thereof, mixed with water later to be separated into wort and spent grains.

The term "wort" refers to the unfermented liquor run-off following extracting the grist during mashing.

"Iodine-positive starch" or "IPS" refers to (1) amylose that is not hydrolyzed after liquefaction and saccharification, or (2) a retrograded starch polymer. When saccharified starch or saccharide liquor is tested with iodine, the high DPn amylose or the retrograded starch polymer binds iodine and produces a characteristic blue color. The saccharide liquor is thus termed "iodine-positive saccharide," "blue saccharide," or "blue sac."

The terms "retrograded starch" or "starch retrogradation" refer to changes that occur spontaneously in a starch paste or gel on ageing.

The term "about" refers to ± 15% to the referenced value.

### 2. Variant α-Amylases having reduced susceptibility to proteases

An aspect of the present compositions and methods relates to variant α-amylase enzymes having reduced susceptibility to proteolytic cleavage compared to the parental α-amylase from which they are derived. Without being limited to a theory, it is believed that certain α-amylases include amino acid sequence in surface-exposed loops that are susceptible to proteolytic cleavage. Such sequences may have evolved to make the α-amylases labile in the presence of protease, *e.g*., to regulate their activity under certain conditions. Alternatively, such sequences may have evolved because the parental α-amylases were not typically exposed to an environment where proteases were present, in which case there was no selective pressure to promote resistance to proteases. Regardless of the reason for the existence of such sequences, it has been observed that some α-amylases that exhibit excellent starch hydrolysis activity and performance in industrial applications are not commercially useful only because of their susceptibility to proteases. The present compositions and methods allow such α-amylases to be used in applications where proteases are known or expected to be present.

As described in detail in the appended Examples, an α-amylase from *Paenibacillus curdlanolyticus* (PcuAmy1) was shown to have high levels of starch hydrolysis activity and engineered variants outperformed commercial benchmarks in cleaning assays. However, the parental α-amylase and its initial variants proved to be highly protease susceptible, making them unsuitable for use in combination with proteases, as would be the case if used in premium laundry and dishwashing detergents, which almost invariably include proteases, such as subtilisin proteases. Following incubation in the presence of *Bacillus lentis* subtilisin (GG36, DuPont Industrial Biosciences, Palo Alto, CA, USA), mass spectroscopy analysis of the reaction products were consistent with hydrolysis of PcuAmy1 occurring between residues Q334 and L336.

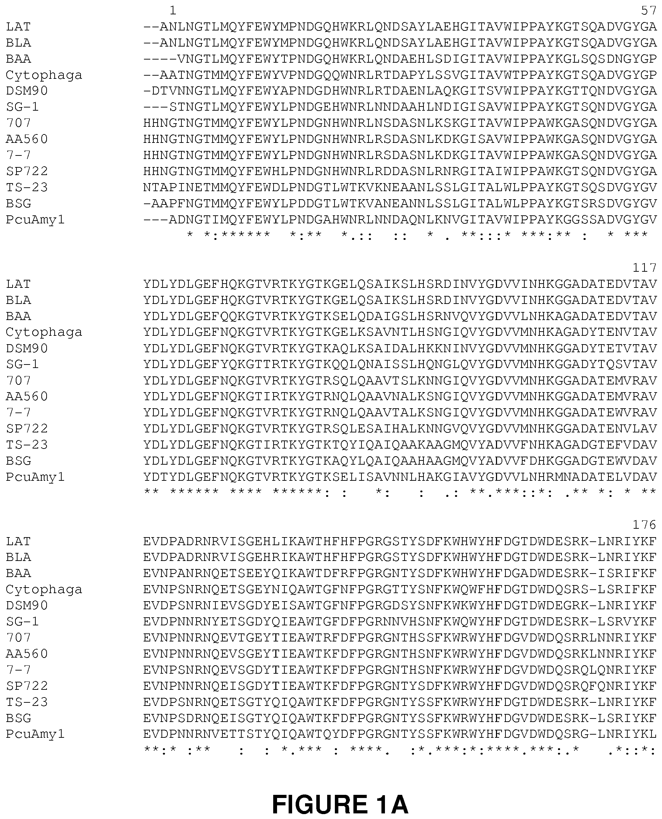

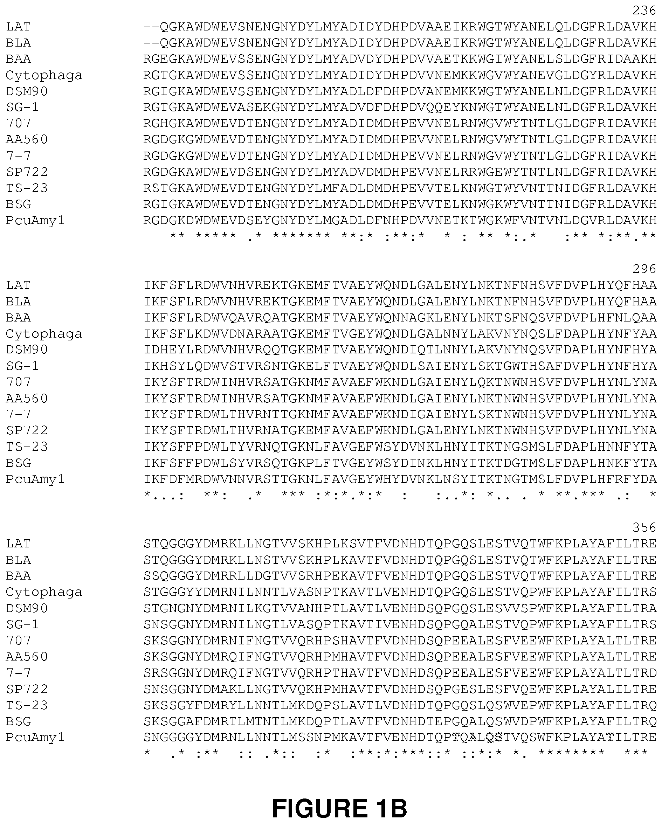

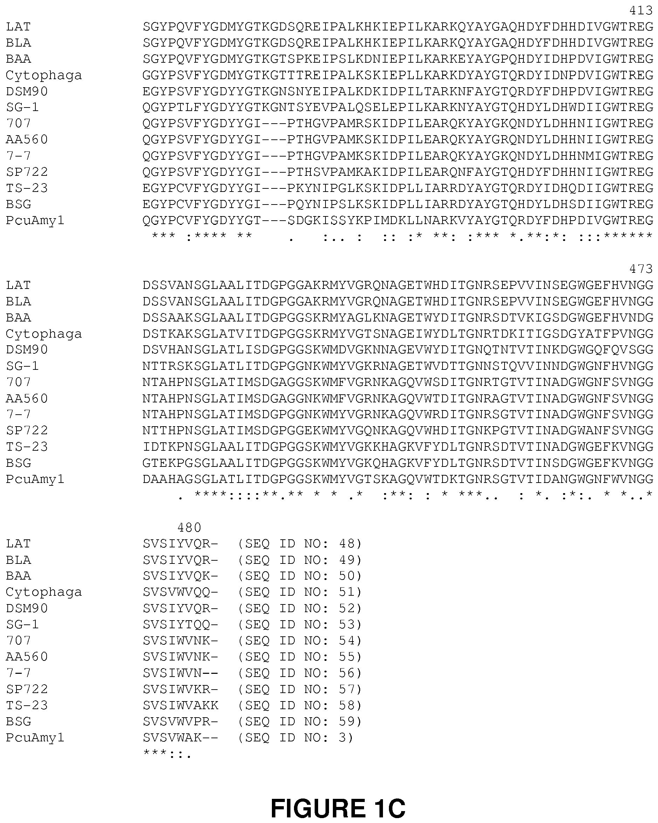

Figure 1A-1C shown an amino acid sequence alignment using Clustal W (default parameters) of several α-amylases previously described for use in commercial applications, including, the α-amylases from *Bacillus* sp. 707 (SEQ ID NO: 54), *Bacillus* sp. AA560 (SEQ ID NO: 55), *Bacillus* sp. 7-7 (SEQ ID NO: 56), *Bacillus* sp. SP722 (SEQ ID NO: 57), *Bacillus liecheniformis* [LAT (SEQ ID NO: 48); BLA(SEQ ID NO: 49)], *Bacillus amyloliquifaciens* (BAA; SEQ ID NO: 50), *Cytophaga* sp. (SEQ ID NO: 51), *Bacillus* sp. DSM90 (SEQ ID NO: *52), Bacillus* sp. SG-1 (SEQ ID NO: 53), *Bacillus* sp. TS-23 (SEQ ID NO: 58), *Bacillus sterothermophilus* (BSG; SEQ ID NO: 59), as well as PcuAmy1 (SEQ ID NO: 3). LAT and BLA are both *B. liecheniformis* α-amylases but differ in amino acid sequence at a few positions. Amino acid numbers used in Figure 1A-1C and throughout are with reference to PcuAmy1 (SEQ ID NO: 3) unless otherwise stated. Residues 333, 335, 337, 338, and 251 are in bold in PcuAmy1.

The primary amino amino acid sequence between poistions 322 and 355 is highly conserved among α-amylases, with these sequences forming part of the 7^{th} strand of the β-barrel, the 7^{th} helix, and the loop linking these structures in the TIM barrel structure of the molecule. Residues 333 and 335 are in a surface exposed portion of the aforementioned loop. The residue at position 333 is usually glycine but is sometimes a glutamic acid. The residue at position 337 is usually serine or alanine. In *P. curdlanolyticus* α-amylase, the residues that occupy these positions are threonine and alanine, respectively. The presence of a threonine at position 333 is particularly unusual within α-amylases.

As described in the appended Examples, substitution of the threonine present at position 333 of *P. curdlanolyticus* α-amylase with a canonical glycine dramatically reduced the susceptibility of the α-amylase to proteolytic cleavage. The further substitution of the alanine present at position 335 further reduced the susceptibility of the α-amylase to proteolytic cleavage, although the substitution at position 333 was clearly most important. The ability to reduce the susceptibility of the *P. curdlanolyticus* α-amylase to proteolytic cleavage allows variants of this α-amylase to be used in commercial applications where proteases are known or suspected to be present.

While the mutations responsible for reducing protease susceptibility were identified using a particular α-amylase, the present mutations and methods can be applied to other protease-susceptible α-amylases that include non-canonical, surface-exposed residues in the loop linking the 7^{th} strand of the β-barrel and the 7^{th} helix in the TIM barrel structure. Such α-amylases can be identified by amino acid sequence alignment, as illustrated in Figure 1A-1C. The precise beginning and end of the loop linking the 7^{th} strand of the β-barrel and the 7^{th} helix may vary slightly for different α-amylases but can be determined accurately by modeling a protease-susceptible α-amylase based on the structure of commonly used industrial α-amylases, such as those from *Bacillus liecheniformis, sterothermophilus*, and *amyloliquifaciens*, the structures of which are available via the Research Collaboratory for Structural Bioinformatics (RCSB) Protein Data Bank (PDB). The surface-exposed residues in the loop can be identified in the same manner.

In view of the described discovery, an aspect of the present compositions and methods is a method for reducing the susceptibility of an α-amylase to proteolytic cleavage by substituting a non-canonical, surface-exposed residue in the loop linking the 7^{th} strand of the β-barrel and the 7^{th} helix in the TIM barrel structure of a parent α-alpha-amylase with a different amino acid residue to produce a variant alpha-amylase, wherein the variant alpha-amylase has reduced susceptibility to cleavage in the loop linking the 7^{th} strand of the β-barrel and the 7^{th} helix by a protease compared to the parent alpha-amylase. In some embodiments, the parent α-amylase has an amino acid residue other than glycine or glutamine at the position corresponding to position 333. In particular embodiments, the parent α-amylase has a threonine at the position corresponding to position 333. In some embodiments, the amino acid residue at the position corresponding to position 333 is substituted to a glycine or glutamic acid.

In some embodiments, the method further includes substituting the amino acid residue present at the position corresponding to position 335 in a parent alpha-amylase with a different amino acid residue. In some embodiments, the parent alpha-amylase has an amino acid residue other than serine at the position corresponding to position 335. In some embodiments, the substitution is to a serine.

In some embodiments, the protease to which the α-amylase is susceptible is a serine protease. In particular embodiments, the protease is a subtilisin-like serine protease, including any of the myriad variants described for use in laundry and dishwashing compositions, including variants having improved cold-water cleaning performance.

Another aspect of the present compositions and methods is a variant α-amylase produced by the aforementioned method. Such variants may be based on any CAZy Family 13 α-amylases that are susceptible to proteolytic cleavage as a result of having a non-canonical amino acid residue at a surface-exposed residue in the loop linking the 7^{th} strand of the β-barrel and the 7^{th} helix in the TIM barrel structure. In some embodiments, the variant α-amylases have a defined degree of amino acid sequence identity to *P. curdlanolyticus* α-amylase (SEQ ID NO: 3) for example, at least 60%, at least 70%, at least 80%, at least 85%, at least 90%, at least 95%, at least 98%, or even at least 99%, amino acid sequence identity. In some embodiments, the present α-amylase are derived from a parental amylase having a defined degree of amino acid sequence identity to *P. curdlanolyticus* α-amylase, for example, at least 60%, at least 70%, at least 80%, at least 85%, at least 90%, at least 95%, at least 98%, or even at least 99%, amino acid sequence identity.

In some embodiments, the variant α-amylases comprise conservative substitution of one or several amino acid residues relative to the parent α-amylase. Exemplary conservative amino acid substitutions are listed in the Table 1Some conservative mutations can be produced by genetic manpulation, while others are produced by introducing synthetic amino acids into a polypeptide other means. **Table 1**. Conservative amino acid substitutions

| *For Amino Acid* | *Code* | *Replace with any of* |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | c | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, b-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

In some embodiments, the present α-amylases comprises a deletion, substitution, insertion, or addition of one or a few amino acid residues relative to the amino acid sequence of the parent α-amylase. Exemplary deletions, substitutions, and insertions correspond to those that have been made in CAZy Family 13 α-amylases. In some embodiments, the present α-amylases are derived from the amino acid sequence of a parent α-amylase by deletion, substitution, insertion, or addition of one or a few amino acid residues relative to the amino acid sequence of the parent α-amylase. In all cases, the expression "one or a few amino acid residues" refers to 10 or less, *i.e.,* 1, 2, 3, 4, 5, 6, 7, 8, 9, or 10, amino acid residues.

In some embodiments, the variants include mutations that further improve their performance or stability, for example, thermal stability, detergent stability, oxidative stability, starch hydrolysis activity, cold-temperature perfomance, expression, solubility, and the like. Exemplary mutations are those described for other CAZy Family 13 α-amylases. In some embodiments, the mutation is the deletion of one or more amino acid residues at positions corresponding to positions 177, 178, 179, and 180. In particular embodiment, the mutation is the pairwise deletion of the residues at positions 177 and 178 or 179 and 180. In some embodiments, the mutation is the substitution of the amino acid residue present at a position corresponding to position 186 with a different amino acid residue. In particular embodiments, the mutation is equivalent to E186P. In some embodiments, the mutation is the substitution of the amino acid residue present at a position corresponding to position 472 with a different amino acid residue. In particular embodiments, the mutation is G472K or G472R. In some embodiments, the performance mutation is at one or more of positions N125, F152, N205, and G473.

Exemplary combinations of mutations are shown, below:
N125Y + E186P + T333G + A335S + Q337E + G472K;
N125Y + F152W + E186P + T333G + A335S + Q337E + G472K;
N125Y + F152W + E186P + T333G + A335S + Q337E + G472R + G473R;
N125Y + F152W + E186P + N205D + T333G + A335S + Q337E + G472K;
N125Y + E186P + T333G + A335S + G472K;
N125Y + F152W + E186P + T333G + A335S + G472K;
N125Y + F152W + E186P + T333G + A335S + G472R + G473R;
N125Y + F152W + E186P + N205D + T333G + A335S + G472K;
N125Y + E186P + T333G + G472K;
N125Y + F152W + E186P + T333G + G472K;
N125Y + F152W + E186P + T333G + G472R + G473R; and
N125Y + F152W + E186P + N205D + T333G + G472K.

These combinations of mutations can be combined with the aforementioned deletions.

The present variant α-amylases may be "precursor," "immature," or "full-length," in which case they include a signal sequence, or "mature," in which case they lack a signal sequence. Mature forms of the polypeptides are generally the most useful. Unless otherwise noted, the amino acid residue numbering used herein refers to the mature forms of the respective amylase polypeptides. The present amylase polypeptides may also be truncated to remove the N or C-termini, so long as the resulting polypeptides retain amylase activity.

The present amylase may be a "chimeric" or "hybrid" polypeptide, in that it includes at least a portion of a first amylase polypeptide, and at least a portion of a second amylase polypeptide (such chimeric amylases have recently been "rediscovered" as domain-swap amylases). The present amylases may further include heterologous signal sequence, an epitope to allow tracking or purification, or the like. Exemplary heterologous signal sequences are from *B. licheniformis* amylase (LAT), *B. subtilis* (AmyE or AprE), and *Streptomyces* CelA.

In another aspect, nucleic acids encoding an α-amylase polypeptide is provided. In some embodiments, the nucleic acid hybridizes under stringent or very stringent conditions to a nucleic acid complementary to a nucleic acid encoding PcuAmy1 α-amylase, such as SEQ ID NO: 1.

Nucleic acids may encode a "full-length" ("fl" or "FL") amylase, which includes a signal sequence, only the mature form of an amylase, which lacks the signal sequence, or a truncated form of an amylase, which lacks the N or C-terminus of the mature form. Preferrably, the nucleic acids are of sufficient length to encode an active amylase enzyme. In all cases, the nucleic acid encode am α-amylase that includes a mutation correspoinding to a those described, herein, and have reduced susceptibility to protease compared to the corresponding parental α-amylase.

A nucleic acid that encodes an α-amylase can be operably linked to various promoters and regulators in a vector suitable for expressing the α-amylase in host cells. Exemplary promoters are from *B. licheniformis* amylase (LAT), *B. subtilis* (AmyE or AprE), and *Streptomyces* CelA. Such a nucleic acid can also be linked to other coding sequences, *e.g*., to encode a chimeric polypeptide.

### 3. Production of α-Amylases

The present α-amylases can be produced in host cells, for example, by secretion or intracellular expression. A cultured cell material (*e*.*g*., a whole-cell broth) comprising an α-amylase can be obtained following secretion of the α-amylase into the cell medium. Optionally, the α-amylase can be isolated from the host cells, or even isolated from the cell broth, depending on the desired purity of the final α-amylase. A gene encoding an α-amylase can be cloned and expressed according to methods well known in the art. Suitable host cells include bacterial, fungal (including yeast and filamentous fungi), and plant cells (including algae). Particularly useful host cells include *Aspergillus niger, Aspergillus oryzae* or *Trichoderma reesei.* Other host cells include bacterial cells, *e.g., Bacillus subtilis* or *B. licheniformis,* as well as *Streptomyces.*

The host cell further may express a nucleic acid encoding a homologous or heterologous glucoamylase, *i.e*., a glucoamylase that is not the same species as the host cell, or one or more other enzymes. The glucoamylase may be a variant glucoamylase, such as one of the glucoamylase variants disclosed in U.S. Patent No. 8,058,033 (Danisco US Inc.), for example. Additionally, the host may express one or more accessory enzymes, proteins, peptides. These may benefit liquefaction, saccharification, fermentation, SSF, etc processes. Furthermore, the host cell may produce biochemicals in addition to enzymes used to digest the various feedstock(s). Such host cells may be useful for fermentation or simultaneous saccharification and fermentation processes to reduce or eliminate the need to add enzymes.

### 3.1. Vectors

A DNA construct comprising a nucleic acid encoding α-amylases can be constructed to be expressed in a host cell. Because of the well-known degeneracy in the genetic code, different polynucleotides that encode an identical amino acid sequence can be designed and made with routine skill. It is also well-known in the art to optimize codon use for a particular host cell. Nucleic acids encoding α-amylases can be incorporated into a vector. Vectors can be transferred to a host cell using well-known transformation techniques, such as those disclosed below.

The vector may be any vector that can be transformed into and replicated within a host cell. For example, a vector comprising a nucleic acid encoding an α-amylase can be transformed and replicated in a bacterial host cell as a means of propagating and amplifying the vector. The vector also may be transformed into an expression host, so that the encoding nucleic acids can be expressed as a functional amylase. Host cells that serve as expression hosts can include filamentous fungi, for example. The Fungal Genetics Stock Center (FGSC) Catalogue of Strains lists suitable vectors for expression in fungal host cells. *See* FGSC, Catalogue of Strains, University of Missouri, at www.fgsc.net (last modified January 17, 2007). A representative vector is pJG153, a promoterless Cre expression vector that can be replicated in a bacterial host. *See* Harrison et al. (2011) Applied Environ. Microbiol. 77:3916-22. pJG153can be modified with routine skill to comprise and express a nucleic acid encoding an amylase variant.

A nucleic acid encoding an α-amylase can be operably linked to a suitable promoter, which allows transcription in the host cell. The promoter may be any DNA sequence that shows transcriptional activity in the host cell of choice and may be derived from genes encoding proteins either homologous or heterologous to the host cell. Exemplary promoters for directing the transcription of the DNA sequence encoding an α-amylase, especially in a bacterial host, are the promoter of the lac operon of *E. coli*, the *Streptomyces coelicolor* agarase gene dagA or celA promoters, the promoters of the *Bacillus licheniformis* α-amylase gene (amyL), the promoters of the *Bacillus stearothermophilus* maltogenic amylase gene (amyM), the promoters of the *Bacillus amyloliquefaciens* α-amylase (amyQ), the promoters of the *Bacillus subtilis* xylA and xylB genes *etc.* For transcription in a fungal host, examples of useful promoters are those derived from the gene encoding *Aspergillus oryzae* TAKA amylase, *Rhizomucor miehei* aspartic proteinase, *Aspergillus niger* neutral α-amylase, *A*. *niger* acid stable α-amylase, *A*. *niger* glucoamylase, *Rhizomucor miehei* lipase, *A. oryzae* alkaline protease, *A*. *oryzae* triose phosphate isomerase, or *A*. *nidulans* acetamidase. When a gene encoding an amylase is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. *cbh1* is an endogenous, inducible promoter from *T. reesei. See* Liu et al. (2008) "Improved heterologous gene expression in Trichoderma reesei by cellobiohydrolase I gene (cbh1) promoter optimization," Acta Biochim. Biophys. Sin (Shanghai) 40(2): 158-65.

The coding sequence can be operably linked to a signal sequence. The DNA encoding the signal sequence may be the DNA sequence naturally associated with the amylase gene to be expressed or from a different Genus or species. A signal sequence and a promoter sequence comprising a DNA construct or vector can be introduced into a fungal host cell and can be derived from the same source. For example, the signal sequence is the *cbh1* signal sequence that is operably linked to a *cbh1* promoter.

An expression vector may also comprise a suitable transcription terminator and, in eukaryotes, polyadenylation sequences operably linked to the DNA sequence encoding an α-amylase. Termination and polyadenylation sequences may suitably be derived from the same sources as the promoter.

The vector may further comprise a DNA sequence enabling the vector to replicate in the host cell. Examples of such sequences are the origins of replication of plasmids pUC19, pACYC177, pUB110, pE194, pAMB1, and pIJ702.

The vector may also comprise a selectable marker, *e.g*., a gene the product of which complements a defect in the isolated host cell, such as the *dal* genes from *B. subtilis* or *B. licheniformis*, or a gene that confers antibiotic resistance such as, *e.g*., ampicillin, kanamycin, chloramphenicol or tetracycline resistance. Furthermore, the vector may comprise *Aspergillus* selection markers such as *amdS*, *argB*, *niaD* and xx*sC*, a marker giving rise to hygromycin resistance, or the selection may be accomplished by co-transformation, such as known in the art. *See e.g.,* International PCT Application WO 91/17243.

Intracellular expression may be advantageous in some respects, *e*.*g*., when using certain bacteria or fungi as host cells to produce large amounts of amylase for subsequent enrichment or purification. Extracellular secretion of amylase into the culture medium can also be used to make a cultured cell material comprising the isolated amylase.

The expression vector typically includes the components of a cloning vector, such as, for example, an element that permits autonomous replication of the vector in the selected host organism and one or more phenotypically detectable markers for selection purposes. The expression vector normally comprises control nucleotide sequences such as a promoter, operator, ribosome binding site, translation initiation signal and optionally, a repressor gene or one or more activator genes. Additionally, the expression vector may comprise a sequence coding for an amino acid sequence capable of targeting the amylase to a host cell organelle such as a peroxisome, or to a particular host cell compartment. Such a targeting sequence includes but is not limited to the sequence, SKL. For expression under the direction of control sequences, the nucleic acid sequence of the amylase is operably linked to the control sequences in proper manner with respect to expression.

The procedures used to ligate the DNA construct encoding an amylase, the promoter, terminator and other elements, respectively, and to insert them into suitable vectors containing the information necessary for replication, are well known to persons skilled in the art (*see, e.g.,* Sambrook et al., MOLECULAR CLONING: A LABORATORY MANUAL, 2nd ed., Cold Spring Harbor, 1989, and 3rd ed., 2001).

### 3.2. Transformation and Culture of Host Cells

An isolated cell, either comprising a DNA construct or an expression vector, is advantageously used as a host cell in the recombinant production of an amylase. The cell may be transformed with the DNA construct encoding the enzyme, conveniently by integrating the DNA construct (in one or more copies) in the host chromosome. This integration is generally considered to be an advantage, as the DNA sequence is more likely to be stably maintained in the cell. Integration of the DNA constructs into the host chromosome may be performed according to conventional methods, *e*.*g*., by homologous or heterologous recombination. Alternatively, the cell may be transformed with an expression vector as described above in connection with the different types of host cells.

Examples of suitable bacterial host organisms are Gram positive bacterial species such as *Bacillaceae* including *Bacillus subtilis, Bacillus licheniformis, Bacillus lentus, Bacillus brevis, Geobacillus* (formerly *Bacillus*) *stearothermophilus, Bacillus alkalophilus, Bacillus amyloliquefaciens, Bacillus coagulans, Bacillus lautus, Bacillus megaterium,* and *Bacillus thuringiensis*; *Streptomyces* species such as *Streptomyces murinus*; lactic acid bacterial species including *Lactococcus* sp. such as *Lactococcus lactis*; *Lactobacillus* sp. including *Lactobacillus reuteri*; *Leuconostoc* sp.; *Pediococcus* sp.; and *Streptococcus* sp. Alternatively, strains of a Gram negative bacterial species belonging to *Enterobacteriaceae* including *E. coli*, or to *Pseudomonadaceae* can be selected as the host organism.

A suitable yeast host organism can be selected from the biotechnologically relevant yeasts species such as but not limited to yeast species such as *Pichia* sp., *Hansenula* sp., or *Kluyveromyces*, *Yarrowinia*, *Schizosaccharomyces* species or a species of *Saccharomyces*, including *Saccharomyces cerevisiae* or a species belonging to *Schizosaccharomyces* such as, for example, *S. pombe* species. A strain of the methylotrophic yeast species, *Pichia pastoris*, can be used as the host organism. Alternatively, the host organism can be a *Hansenula* species. Suitable host organisms among filamentous fungi include species of *Aspergillus, e.g., Aspergillus niger, Aspergillus oryzae, Aspergillus tubigensis, Aspergillus awamori,* or *Aspergillus nidulans.* Alternatively, strains of a *Fusarium* species, *e.g., Fusarium oxysporum* or of a *Rhizomucor* species such as *Rhizomucor miehei* can be used as the host organism. Other suitable strains include *Thermomyces* and *Mucor* species. In addition, *Trichoderma sp.* can be used as a host. A suitable procedure for transformation of *Aspergillus* host cells includes, for example, that described in EP 238023. An amylase expressed by a fungal host cell can be glycosylated, *i.e*., will comprise a glycosyl moiety. The glycosylation pattern can be the same or different as present in the wild-type amylase. The type and/or degree of glycosylation may impart changes in enzymatic and/or biochemical properties.

It is advantageous to delete genes from expression hosts, where the gene deficiency can be cured by the transformed expression vector. Known methods may be used to obtain a fungal host cell having one or more inactivated genes. Gene inactivation may be accomplished by complete or partial deletion, by insertional inactivation or by any other means that renders a gene nonfunctional for its intended purpose, such that the gene is prevented from expression of a functional protein. Any gene from a *Trichoderma* sp. or other filamentous fungal host that has been cloned can be deleted, for example, *cbh1, cbh2, egl1,* and *egl2* genes. Gene deletion may be accomplished by inserting a form of the desired gene to be inactivated into a plasmid by methods known in the art.

Introduction of a DNA construct or vector into a host cell includes techniques such as transformation; electroporation; nuclear microinjection; transduction; transfection, *e*.*g*., lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g.,* Sambrook *et al*. (2001), *supra.* The expression of heterologous protein in *Trichoderma* is described, for example, in U.S. Patent No. 6,022,725. Reference is also made to Cao et al. (2000) Science 9:991-1001 for transformation of *Aspergillus* strains. Genetically stable transformants can be constructed with vector systems whereby the nucleic acid encoding an amylase is stably integrated into a host cell chromosome. Transformants are then selected and purified by known techniques.

The preparation of *Trichoderma* sp. for transformation, for example, may involve the preparation of protoplasts from fungal mycelia. *See* Campbell et al. (1989) Curr. Genet. 16: 53-56. The mycelia can be obtained from germinated vegetative spores. The mycelia are treated with an enzyme that digests the cell wall, resulting in protoplasts. The protoplasts are protected by the presence of an osmotic stabilizer in the suspending medium. These stabilizers include sorbitol, mannitol, potassium chloride, magnesium sulfate, and the like. Usually the concentration of these stabilizers varies between 0.8 M and 1.2 M, *e.g*., a 1.2 M solution of sorbitol can be used in the suspension medium.

Uptake of DNA into the host *Trichoderma* sp. strain depends upon the calcium ion concentration. Generally, between about 10-50 mM CaCl₂ is used in an uptake solution. Additional suitable compounds include a buffering system, such as TE buffer (10 mM Tris, pH 7.4; 1 mM EDTA) or 10 mM MOPS, pH 6.0 and polyethylene glycol. The polyethylene glycol is believed to fuse the cell membranes, thus permitting the contents of the medium to be delivered into the cytoplasm of the *Trichoderma* sp. strain. This fusion frequently leaves multiple copies of the plasmid DNA integrated into the host chromosome.

Usually transformation of *Trichoderma* sp. uses protoplasts or cells that have been subjected to a permeability treatment, typically at a density of 10⁵ to 10⁷/mL, particularly 2x10⁶/mL. A volume of 100 µL of these protoplasts or cells in an appropriate solution (*e*.*g*., 1.2 M sorbitol and 50 mM CaCl₂) may be mixed with the desired DNA. Generally, a high concentration of PEG is added to the uptake solution. From 0.1 to 1 volume of 25% PEG 4000 can be added to the protoplast suspension; however, it is useful to add about 0.25 volumes to the protoplast suspension. Additives, such as dimethyl sulfoxide, heparin, spermidine, potassium chloride and the like, may also be added to the uptake solution to facilitate transformation. Similar procedures are available for other fungal host cells. *See, e.g.,* U.S. Patent No. 6,022,725.

### 3.3. Expression

A method of producing an amylase may comprise cultivating a host cell as described above under conditions conducive to the production of the enzyme and recovering the enzyme from the cells and/or culture medium.

The medium used to cultivate the cells may be any conventional medium suitable for growing the host cell in question and obtaining expression of an amylase. Suitable media and media components are available from commercial suppliers or may be prepared according to published recipes (*e.g*., as described in catalogues of the American Type Culture Collection).

An enzyme secreted from the host cells can be used in a whole broth preparation. In the present methods, the preparation of a spent whole fermentation broth of a recombinant microorganism can be achieved using any cultivation method known in the art resulting in the expression of an α-amylase. Fermentation may, therefore, be understood as comprising shake flask cultivation, small- or large-scale fermentation (including continuous, batch, fed-batch, or solid state fermentations) in laboratory or industrial fermenters performed in a suitable medium and under conditions allowing the amylase to be expressed or isolated. The term "spent whole fermentation broth" is defined herein as unfractionated contents of fermentation material that includes culture medium, extracellular proteins (*e*.*g*., enzymes), and cellular biomass. It is understood that the term "spent whole fermentation broth" also encompasses cellular biomass that has been lysed or permeabilized using methods well known in the art.

An enzyme secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

The polynucleotide encoding an amylase in a vector can be operably linked to a control sequence that is capable of providing for the expression of the coding sequence by the host cell, *i.e.* the vector is an expression vector. The control sequences may be modified, for example by the addition of further transcriptional regulatory elements to make the level of transcription directed by the control sequences more responsive to transcriptional modulators. The control sequences may in particular comprise promoters.

Host cells may be cultured under suitable conditions that allow expression of an amylase. Expression of the enzymes may be constitutive such that they are continually produced, or inducible, requiring a stimulus to initiate expression. In the case of inducible expression, protein production can be initiated when required by, for example, addition of an inducer substance to the culture medium, for example dexamethasone or IPTG or Sophorose. Polypeptides can also be produced recombinantly in an in vitro cell-free system, such as the TnT™ (Promega) rabbit reticulocyte system.

An expression host also can be cultured in the appropriate medium for the host, under aerobic conditions. Shaking or a combination of agitation and aeration can be provided, with production occurring at the appropriate temperature for that host, *e.g*., from about 25°C to about 75°C (*e.g*., 30°C to 45°C), depending on the needs of the host and production of the desired α-amylase. Culturing can occur from about 12 to about 100 hours or greater (and any hour value there between, *e*.*g*., from 24 to 72 hours). Typically, the culture broth is at a pH of about 4.0 to about 8.0, again depending on the culture conditions needed for the host relative to production of an amylase.

### 3.4. Identification of Amylase Activity

To evaluate the expression of an amylase in a host cell, assays can measure the expressed protein, corresponding mRNA, or α-amylase activity. For example, suitable assays include Northern blotting, reverse transcriptase polymerase chain reaction, and in situ hybridization, using an appropriately labeled hybridizing probe. Suitable assays also include measuring amylase activity in a sample, for example, by assays directly measuring reducing sugars such as glucose in the culture media. For example, glucose concentration may be determined using glucose reagent kit No. 15-UV (Sigma Chemical Co.) or an instrument, such as Technicon Autoanalyzer. α-Amylase activity also may be measured by any known method, such as the PAHBAH or ABTS assays, described below.

### 3.5. Methods for Enriching and Purifying α-Amylases

Fermentation, separation, and concentration techniques are well known in the art and conventional methods can be used in order to prepare a concentrated an α-α-amylase polypeptide-containing solution.

After fermentation, a fermentation broth is obtained, the microbial cells and various suspended solids, including residual raw fermentation materials, are removed by conventional separation techniques in order to obtain an amylase solution. Filtration, centrifugation, microfiltration, rotary vacuum drum filtration, ultrafiltration, centrifugation followed by ultrafiltration, extraction, or chromatography, or the like, are generally used.

It is desirable to concentrate an α-amylase polypeptide-containing solution in order to optimize recovery. Use of unconcentrated solutions requires increased incubation time in order to collect the enriched or purified enzyme precipitate.

The enzyme containing solution is concentrated using conventional concentration techniques until the desired enzyme level is obtained. Concentration of the enzyme containing solution may be achieved by any of the techniques discussed herein. Exemplary methods of enrichment and purification include but are not limited to rotary vacuum filtration and/or ultrafiltration.

The enzyme solution is concentrated into a concentrated enzyme solution until the enzyme activity of the concentrated α-amylase polypeptide-containing solution is at a desired level.

Concentration may be performed using, *e.g*., a precipitation agent, such as a metal halide precipitation agent. Metal halide precipitation agents include but are not limited to alkali metal chlorides, alkali metal bromides and blends of two or more of these metal halides. Exemplary metal halides include sodium chloride, potassium chloride, sodium bromide, potassium bromide and blends of two or more of these metal halides. The metal halide precipitation agent, sodium chloride, can also be used as a preservative.

The metal halide precipitation agent is used in an amount effective to precipitate an amylase. The selection of at least an effective amount and an optimum amount of metal halide effective to cause precipitation of the enzyme, as well as the conditions of the precipitation for maximum recovery including incubation time, pH, temperature and concentration of enzyme, will be readily apparent to one of ordinary skill in the art, after routine testing.

Generally, at least about 5% w/v (weight/volume) to about 25% w/v of metal halide is added to the concentrated enzyme solution, and usually at least 8% w/v. Generally, no more than about 25% w/v of metal halide is added to the concentrated enzyme solution and usually no more than about 20% w/v. The optimal concentration of the metal halide precipitation agent will depend, among others, on the nature of the specific α-amylase polypeptide and on its concentration in the concentrated enzyme solution.

Another alternative way to precipitate the enzyme is to use organic compounds. Exemplary organic compound precipitating agents include: 4-hydroxybenzoic acid, alkali metal salts of 4-hydroxybenzoic acid, alkyl esters of 4-hydroxybenzoic acid, and blends of two or more of these organic compounds. The addition of the organic compound precipitation agents can take place prior to, simultaneously with or subsequent to the addition of the metal halide precipitation agent, and the addition of both precipitation agents, organic compound and metal halide, may be carried out sequentially or simultaneously.

Generally, the organic precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitation agents can be, for example, linear or branched alkyl esters of 4-hydroxybenzoic acid, wherein the alkyl group contains from 1 to 10 carbon atoms, and blends of two or more of these organic compounds. Exemplary organic compounds are linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl esters of 4-hydroxybenzoic acid, butyl ester of 4-hydroxybenzoic acid, ethyl ester of 4-hydroxybenzoic acid and blends of two or more of these organic compounds can also be used. Additional organic compounds also include but are not limited to 4-hydroxybenzoic acid methyl ester (named methyl PARABEN), 4-hydroxybenzoic acid propyl ester (named propyl PARABEN), which also are both amylase preservative agents. For further descriptions, see, *e.g*., U.S. Patent No. 5,281,526.

Addition of the organic compound precipitation agent provides the advantage of high flexibility of the precipitation conditions with respect to pH, temperature, α-amylase concentration, precipitation agent concentration, and time of incubation.

The organic compound precipitation agent is used in an amount effective to improve precipitation of the enzyme by means of the metal halide precipitation agent. The selection of at least an effective amount and an optimum amount of organic compound precipitation agent, as well as the conditions of the precipitation for maximum recovery including incubation time, pH, temperature and concentration of enzyme, will be readily apparent to one of ordinary skill in the art, in light of the present disclosure, after routine testing.

Generally, at least about 0.01% w/v of organic compound precipitation agent is added to the concentrated enzyme solution and usually at least about 0.02% w/v. Generally, no more than about 0.3% w/v of organic compound precipitation agent is added to the concentrated enzyme solution and usually no more than about 0.2% w/v.

The concentrated polypeptide solution, containing the metal halide precipitation agent, and the organic compound precipitation agent, can be adjusted to a pH, which will, of necessity, depend on the enzyme to be enriched or purified. Generally, the pH is adjusted at a level near the isoelectric point of the amylase. The pH can be adjusted at a pH in a range from about 2.5 pH units below the isoelectric point (pI) up to about 2.5 pH units above the isoelectric point.

The incubation time necessary to obtain an enriched or purified enzyme precipitate depends on the nature of the specific enzyme, the concentration of enzyme, and the specific precipitation agent(s) and its (their) concentration. Generally, the time effective to precipitate the enzyme is between about 1 to about 30 hours; usually it does not exceed about 25 hours. In the presence of the organic compound precipitation agent, the time of incubation can still be reduced to less about 10 hours and in most cases even about 6 hours.

Generally, the temperature during incubation is between about 4°C and about 50°C. Usually, the method is carried out at a temperature between about 10°C and about 45°C (*e.g*., between about 20°C and about 40°C). The optimal temperature for inducing precipitation varies according to the solution conditions and the enzyme or precipitation agent(s) used.

The overall recovery of enriched or purified enzyme precipitate, and the efficiency with which the process is conducted, is improved by agitating the solution comprising the enzyme, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the enriched or purified enzyme is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration, or the like. Further enrichment or purification of the enzyme precipitate can be obtained by washing the precipitate with water. For example, the enriched or purified enzyme precipitate is washed with water containing the metal halide precipitation agent, or with water containing the metal halide and the organic compound precipitation agents.

During fermentation, an α-amylase polypeptide accumulates in the culture broth. For the isolation, enrichment, or purification of the desired α-amylase, the culture broth is centrifuged or filtered to eliminate cells, and the resulting cell-free liquid is used for enzyme enrichment or purification. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme-active fraction. For further enrichment or purification, a conventional procedure such as ion exchange chromatography may be used.

Enriched or purified enzymes are useful for laundry and cleaning applications. For example, they can be used in laundry detergents and spot removers. They can be made into a final product that is either liquid (solution, slurry) or solid (granular, powder).

A more specific example of enrichment or purification, is described in Sumitani et al. (2000) "New type of starch-binding domain: the direct repeat motif in the C-terminal region of Bacillus sp. 195 α-amylase contributes to starch binding and raw starch degrading," Biochem. J. 350: 477-484, and is briefly summarized here. The enzyme obtained from 4 liters of a Streptomyces lividans TK24 culture supernatant was treated with (NH4)2SO4 at 80% saturation. The precipitate was recovered by centrifugation at 10,000 × g (20 min. and 4°C) and re-dissolved in 20 mM Tris/HCl buffer (pH 7.0) containing 5 mM CaCl2. The solubilized precipitate was then dialyzed against the same buffer. The dialyzed sample was then applied to a Sephacryl S-200 column, which had previously been equilibrated with 20 mM Tris/HCl buffer, (pH 7.0), 5 mM CaCl2, and eluted at a linear flow rate of 7 mL/hr with the same buffer. Fractions from the column were collected and assessed for activity as judged by enzyme assay and SDS-PAGE. The protein was further purified as follows. A Toyopearl HW55 column (Tosoh Bioscience, Montgomeryville, PA; Cat. No. 19812) was equilibrated with 20 mM Tris/HCl buffer (pH 7.0) containing 5 mM CaCl2 and 1.5 M (NH4)2SO4. The enzyme was eluted with a linear gradient of 1.5 to 0 M (NH4)2SO4 in 20 mM Tris/HCL buffer, pH 7.0 containing 5 mM CaCl2. The active fractions were collected, and the enzyme precipitated with (NH4)2SO4 at 80% saturation. The precipitate was recovered, re-dissolved, and dialyzed as described above. The dialyzed sample was then applied to a Mono Q HR5/5 column (Amersham Pharmacia; Cat. No. 17-5167-01) previously equilibrated with 20 mM Tris/HCl buffer (pH 7.0) containing 5 mM CaCl2, at a flow rate of 60 mL/hour. The active fractions are collected and added to a 1.5 M (NH4)2SO4 solution. The active enzyme fractions were rechromatographed on a Toyopearl HW55 column, as before, to yield a homogeneous enzyme as determined by SDS-PAGE. See Sumitani et al. (2000) Biochem. J. 350: 477-484, for general discussion of the method and variations thereon.

For production scale recovery, α-amylase polypeptides can be enriched or partially purified as generally described above by removing cells via flocculation with polymers. Alternatively, the enzyme can be enriched or purified by microfiltration followed by concentration by ultrafiltration using available membranes and equipment. However, for some applications, the enzyme does not need to be enriched or purified, and whole broth culture can be lysed and used without further treatment. The enzyme can then be processed, for example, into granules.

### 4. Compositions and Uses of α-amylases

The present α-amylases are useful for a variety of industrial applications. For example, α-amylases are useful in a starch conversion process, particularly in a saccharification process of a starch that has undergone liquefaction. The desired end-product may be any product that may be produced by the enzymatic conversion of the starch substrate. For example, the desired product may be a syrup rich in glucose and maltose, which can be used in other processes, such as the preparation of HFCS, or which can be converted into a number of other useful products, such as ascorbic acid intermediates (*e*.*g*., gluconate; 2-keto-L-gulonic acid; 5-keto-gluconate; and 2,5-diketogluconate); 1,3-propanediol; aromatic amino acids (*e*.*g*., tyrosine, phenylalanine and tryptophan); organic acids (*e*.*g*., lactate, pyruvate, succinate, isocitrate, and oxaloacetate); amino acids (*e*.*g*., serine and glycine); antibiotics; antimicrobials; enzymes; vitamins; and hormones.

The starch conversion process may be a precursor to, or simultaneous with, a fermentation process designed to produce alcohol for fuel or drinking (*i.e*., potable alcohol). One skilled in the art is aware of various fermentation conditions that may be used in the production of these end-products. α-amylases are also useful in compositions and methods of food preparation. These various uses of α-amylases are described in more detail below.

### 4.1. Preparation of Starch Substrates

Those of general skill in the art are well aware of available methods that may be used to prepare starch substrates for use in the processes disclosed herein. For example, a useful starch substrate may be obtained from tubers, roots, stems, legumes, cereals or whole grain. More specifically, the granular starch may be obtained from corn, cobs, wheat, barley, rye, triticale, milo, sago, millet, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains 70-72% starch. Specifically contemplated starch substrates are corn starch and wheat starch. The starch from a grain may be ground or whole and includes corn solids, such as kernels, bran and/or cobs. The starch may also be highly refined raw starch or feedstock from starch refinery processes. Various starches also are commercially available. For example, corn starch is available from Cerestar, Sigma, and Katayama Chemical Industry Co. (Japan); wheat starch is available from Sigma; sweet potato starch is available from Wako Pure Chemical Industry Co. (Japan); and potato starch is available from Nakaari Chemical Pharmaceutical Co. (Japan).

The starch substrate can be a crude starch from milled whole grain, which contains non-starch fractions, *e*.*g*., germ residues and fibers. Milling may comprise either wet milling or dry milling or grinding. In wet milling, whole grain is soaked in water or dilute acid to separate the grain into its component parts, *e*.*g*., starch, protein, germ, oil, kernel fibers. Wet milling efficiently separates the germ and meal (*i.e*., starch granules and protein) and is especially suitable for production of syrups. In dry milling or grinding, whole kernels are ground into a fine powder and often processed without fractionating the grain into its component parts. In some cases, oils from the kernels are recovered. Dry ground grain thus will comprise significant amounts of non-starch carbohydrate compounds, in addition to starch. Dry grinding of the starch substrate can be used for production of ethanol and other biochemicals. The starch to be processed may be a highly refined starch quality, for example, at least 90%, at least 95%, at least 97%, or at least 99.5% pure.

### 4.2. Gelatinization and Liquefaction of Starch

As used herein, the term "liquefaction" or "liquefy" means a process by which starch is converted to less viscous and shorter chain dextrins. Generally, this process involves gelatinization of starch simultaneously with or followed by the addition of an α-amylase, although additional liquefaction-inducing enzymes optionally may be added. In some embodiments, the starch substrate prepared as described above is slurried with water. The starch slurry may contain starch as a weight percent of dry solids of about 10-55%, about 20-45%, about 30-45%, about 30-40%, or about 30-35%. α-Amylase (EC 3.2.1.1) may be added to the slurry, with a metering pump, for example. The α-amylase typically used for this application is a thermally stable, bacterial α-amylase, such as a Geobacillus stearothermophilus α-amylase. The α-amylase is usually supplied, for example, at about 1500 units per kg dry matter of starch. To optimize α-amylase stability and activity, the pH of the slurry typically is adjusted to about pH 5.5-6.5 and about 1 mM of calcium (about 40 ppm free calcium ions) can also be added. Geobacillus stearothermophilus variants or other α-amylases may require different conditions. Bacterial α-amylase remaining in the slurry following liquefaction may be deactivated via a number of methods, including lowering the pH in a subsequent reaction step or by removing calcium from the slurry in cases where the enzyme is dependent upon calcium.

The slurry of starch plus the α-amylase may be pumped continuously through a jet cooker, which is steam heated to 105°C. Gelatinization occurs rapidly under these conditions, and the enzymatic activity, combined with the significant shear forces, begins the hydrolysis of the starch substrate. The residence time in the jet cooker is brief. The partly gelatinized starch may be passed into a series of holding tubes maintained at 105-110°C and held for 5-8 min. to complete the gelatinization process ("primary liquefaction"). Hydrolysis to the required DE is completed in holding tanks at 85-95°C or higher temperatures for about 1 to 2 hours ("secondary liquefaction"). These tanks may contain baffles to discourage back mixing. As used herein, the term "minutes of secondary liquefaction" refers to the time that has elapsed from the start of secondary liquefaction to the time that the Dextrose Equivalent (DE) is measured. The slurry is then allowed to cool to room temperature. This cooling step can be 30 minutes to 180 minutes, *e.g.* 90 minutes to 120 minutes. The liquefied starch typically is in the form of a slurry having a dry solids content (w/w) of about 10-50%; about 10-45%; about 15-40%; about 20-40%; about 25-40%; or about 25-35%.

Liquefaction with α-amylases advantageously can be conducted at low pH, eliminating the requirement to adjust the pH to about pH 5.5-6.5. α-amylases can be used for liquefaction at a pH range of 2 to 7, *e.g*., pH 3.0 - 7.5, pH 4.0 - 6.0, or pH 4.5 - 5.8. α-amylases can maintain liquefying activity at a temperature range of about 85°C - 95°C, *e.g*., 85°C, 90°C, or 95°C. For example, liquefaction can be conducted with 800 µg an amylase in a solution of 25% DS corn starch for 10 min at pH 5.8 and 85°C, or pH 4.5 and 95°C, for example. Liquefying activity can be assayed using any of a number of known viscosity assays in the art.

In particular embodiments using the present α-amylases, starch liquifaction is performed at a temperature range of 90-115°C, for the purpose of producing high-purity glucose syrups, HFCS, maltodextrins, etc.

### 4.3. Saccharification

The liquefied starch can be saccharified into a syrup rich in lower DP (*e.g*., DP1 + DP2) saccharides, using α-amylases, optionally in the presence of another enzyme(s). The exact composition of the products of saccharification depends on the combination of enzymes used, as well as the type of granular starch processed. Advantageously, the syrup obtainable using the provided α-amylases may contain a weight percent of DP2 of the total oligosaccharides in the saccharified starch exceeding 30%, *e.g*., 45% - 65% or 55% - 65%. The weight percent of (DPI + DP2) in the saccharified starch may exceed about 70%, *e.g*., 75% - 85% or 80% - 85%. The present amylases also produce a relatively high yield of glucose, *e.g*., DP1 > 20%, in the syrup product.

Whereas liquefaction is generally run as a continuous process, saccharification is often conducted as a batch process. Saccharification typically is most effective at temperatures of about 60-65°C and a pH of about 4.0-4.5, *e.g*., pH 4.3, necessitating cooling and adjusting the pH of the liquefied starch. Saccharification may be performed, for example, at a temperature between about 40°C, about 50°C, or about 55°C to about 60°C or about 65°C. Saccharification is normally conducted in stirred tanks, which may take several hours to fill or empty. Enzymes typically are added either at a fixed ratio to dried solids as the tanks are filled or added as a single dose at the commencement of the filling stage. A saccharification reaction to make a syrup typically is run over about 24-72 hours, for example, 24-48 hours. When a maximum or desired DE has been attained, the reaction is stopped by heating to 85°C for 5 min., for example. Further incubation will result in a lower DE, eventually to about 90 DE, as accumulated glucose re-polymerizes to isomaltose and/or other reversion products via an enzymatic reversion reaction and/or with the approach of thermodynamic equilibrium. When using an amylase, saccharification optimally is conducted at a temperature range of about 30°C to about 75°C, *e.g*., 45°C - 75°C or 47°C - 74°C. The saccharifying may be conducted over a pH range of about pH 3 to about pH 7, *e.g*., pH 3.0 - pH 7.5, pH 3.5 - pH 5.5, pH 3.5, pH 3.8, or pH 4.5.

An amylase may be added to the slurry in the form of a composition. Amylase can be added to a slurry of a granular starch substrate in an amount of about 0.6 - 10 ppm ds, *e.g*., 2 ppm ds. An amylase can be added as a whole broth, clarified, enriched, partially purified, or purified enzyme. The specific activity of the amylase may be about 300 U/mg of enzyme, for example, measured with the PAHBAH assay. The amylase also can be added as a whole broth product.

An amylase may be added to the slurry as an isolated enzyme solution. For example, an amylase can be added in the form of a cultured cell material produced by host cells expressing an amylase. An amylase may also be secreted by a host cell into the reaction medium during the fermentation or SSF process, such that the enzyme is provided continuously into the reaction. The host cell producing and secreting amylase may also express an additional enzyme, such as a glucoamylase. For example, U.S. Patent No. 5,422,267 discloses the use of a glucoamylase in yeast for production of alcoholic beverages. For example, a host cell, *e.g*., Trichoderma reesei or Aspergillus niger, may be engineered to co-express an amylase and a glucoamylase, *e*.*g*., HgGA, TrGA, or a TrGA variant, during saccharification. The host cell can be genetically modified so as not to express its endogenous glucoamylase and/or other enzymes, proteins or other materials. The host cell can be engineered to express a broad spectrum of various saccharolytic enzymes. For example, the recombinant yeast host cell can comprise nucleic acids encoding a glucoamylase, an alpha-glucosidase, an enzyme that utilizes pentose sugar, an α-amylase, a pullulanse, an isoamylase, and/or an isopullulanase. *See, e.g*., WO 2011/153516 A2.

### 4.4. Isomerization

The soluble starch hydrolysate produced by treatment with amylase can be converted into high fructose starch-based syrup (HFSS), such as high fructose corn syrup (HFCS). This conversion can be achieved using a glucose isomerase, particularly a glucose isomerase immobilized on a solid support. The pH is increased to about 6.0 to about 8.0, *e.g*., pH 7.5 (depending on the isomerase), and Ca²⁺ is removed by ion exchange. Suitable isomerases include SWEETZYME®, IT (Novozymes A/S); G-ZYME® IMGI, and G-ZYME® G993, KETOMAX®, G-ZYME® G993, G-ZYME® G993 liquid, and GENSWEET® IGI. Following isomerization, the mixture typically contains about 40-45% fructose, *e.g*., 42% fructose.

### 4.5. Fermentation

The soluble starch hydrolysate, particularly a glucose rich syrup, can be fermented by contacting the starch hydrolysate with a fermenting organism typically at a temperature around 32°C, such as from 30°C to 35°C for alcohol-producing yeast. The temperature and pH of the fermentation will depend upon the fermenting organism. EOF products include metabolites, such as citric acid, lactic acid, succinic acid, monosodium glutamate, gluconic acid, sodium gluconate, calcium gluconate, potassium gluconate, itaconic acid and other carboxylic acids, glucono delta-lactone, sodium erythorbate, lysine and other amino acids, omega 3 fatty acid, butanol, isoprene, 1,3-propanediol and other biomaterials.

Ethanologenic microorganisms include yeast, such as *Saccharomyces cerevisiae* and bacteria, *e.g*., *Zymomonas moblis*, expressing alcohol dehydrogenase and pyruvate decarboxylase. The ethanologenic microorganism can express xylose reductase and xylitol dehydrogenase, which convert xylose to xylulose. Improved strains of ethanologenic microorganisms, which can withstand higher temperatures, for example, are known in the art and can be used. *See* Liu et al. (2011) Sheng Wu Gong Cheng Xue Bao 27:1049-56. Commercial sources of yeast include ETHANOL RED® (LeSaffre); THERMOSACC® (Lallemand); RED STAR® (Red Star); FERMIOL® (DSM Specialties); and SUPERSTART® (Alltech). Microorganisms that produce other metabolites, such as citric acid and lactic acid, by fermentation are also known in the art. *See, e.g.,* Papagianni (2007) Biotechnol. Adv. 25:244-63; John et al. (2009) Biotechnol. Adv. 27:145-52.

The saccharification and fermentation processes may be carried out as an SSF process. Fermentation may comprise subsequent enrichment, purification, and recovery of ethanol, for example. During the fermentation, the ethanol content of the broth or "beer" may reach about 8-18% v/v, *e.g*., 14-15% v/v. The broth may be distilled to produce enriched, *e.g*., 96% pure, solutions of ethanol. Further, CO₂ generated by fermentation may be collected with a CO₂ scrubber, compressed, and marketed for other uses, *e*.*g*., carbonating beverage or dry ice production. Solid waste from the fermentation process may be used as protein-rich products, *e*.*g*., livestock feed.

As mentioned above, an SSF process can be conducted with fungal cells that express and secrete amylase continuously throughout SSF. The fungal cells expressing amylase also can be the fermenting microorganism, *e*.*g*., an ethanologenic microorganism. Ethanol production thus can be carried out using a fungal cell that expresses sufficient amylase so that less or no enzyme has to be added exogenously. The fungal host cell can be from an appropriately engineered fungal strain. Fungal host cells that express and secrete other enzymes, in addition to amylase, also can be used. Such cells may express glucoamylase and/or a pullulanase, phytase, *alpha*-glucosidase, isoamylase, beta-amylase cellulase, xylanase, other hemicellulases, protease, *beta*-glucosidase, pectinase, esterase, redox enzymes, transferase, or other enzyme.

A variation on this process is a "fed-batch fermentation" system, where the substrate is added in increments as the fermentation progresses. Fed-batch systems are useful when catabolite repression may inhibit the metabolism of the cells and where it is desirable to have limited amounts of substrate in the medium. The actual substrate concentration in fed-batch systems is estimated by the changes of measurable factors such as pH, dissolved oxygen and the partial pressure of waste gases, such as CO₂. Batch and fed-batch fermentations are common and well known in the art.

Continuous fermentation is an open system where a defined fermentation medium is added continuously to a bioreactor, and an equal amount of conditioned medium is removed simultaneously for processing. Continuous fermentation generally maintains the cultures at a constant high density where cells are primarily in log phase growth. Continuous fermentation permits modulation of cell growth and/or product concentration. For example, a limiting nutrient such as the carbon source or nitrogen source is maintained at a fixed rate and all other parameters are allowed to moderate. Because growth is maintained at a steady state, cell loss due to medium being drawn off should be balanced against the cell growth rate in the fermentation. Methods of optimizing continuous fermentation processes and maximizing the rate of product formation are well known in the art of industrial microbiology.

### 4.6. Compositions Comprising α-Amylases

α-amylases may be combined with a glucoamylase (EC 3.2.1.3), *e.g*., a *Trichoderma* glucoamylase or variant thereof. An exemplary glucoamylase is *Trichoderma reesei* glucoamylase (TrGA) and variants thereof that possess superior specific activity and thermal stability. *See* U.S. Published Applications Nos. 2006/0094080, 2007/0004018, and 2007/0015266 (Danisco US Inc.). Suitable variants of TrGA include those with glucoamylase activity and at least 80%, at least 90%, or at least 95% sequence identity to wild-type TrGA. α-amylases advantageously increase the yield of glucose produced in a saccharification process catalyzed by TrGA.

Alternatively, the glucoamylase may be another glucoamylase derived from plants (including algae), fungi, or bacteria. For example, the glucoamylases may be *Aspergillus niger* G1 or G2 glucoamylase or its variants (*e.g*., Boel et al. (1984) EMBO J. 3:1097-1102; WO 92/00381; WO 00/04136 (Novo Nordisk A/S)); and A. *awamori* glucoamylase (*e.g*., WO 84/02921 (Cetus Corp.)). Other contemplated *Aspergillus* glucoamylase include variants with enhanced thermal stability, *e.g*., G137A and G139A (Chen et al. (1996) Prot. Eng. 9:499-505); D257E and D293E/Q (Chen et al. (1995) Prot. Eng. 8:575-582); N182 (Chen et al. (1994) Biochem. J. 301:275-281); A246C (Fierobe et al. (1996) Biochemistry, 35: 8698-8704); and variants with Pro residues in positions A435 and S436 (Li et al. (1997) Protein Eng. 10:1199-1204). Other contemplated glucoamylases include *Talaromyces* glucoamylases, in particular derived from *T. emersonii* (*e.g*., WO 99/28448 (Novo Nordisk A/S), *T. leycettanus* (*e.g*., U.S. Patent No. RE 32,153 (CPC International, Inc.)), *T. duponti*, or *T. thermophilus* (*e.g*., U.S. Patent No. 4,587,215). Contemplated bacterial glucoamylases include glucoamylases from the genus *Clostridium,* in particular *C*. *thermoamylolyticum* (*e.g*., EP 135138 (CPC International, Inc.) and *C*. *thermohydrosulfuricum* (*e.g*., WO 86/01831 (Michigan Biotechnology Institute)). Suitable glucoamylases include the glucoamylases derived from *Aspergillus oryzae*, such as a glucoamylase shown in SEQ ID NO:2 in WO 00/04136 (Novo Nordisk A/S). Also suitable are commercial glucoamylases, such as AMG 200L; AMG 300 L; SAN™ SUPER and AMG™ E (Novozymes); OPTIDEX® 300 and OPTIDEX L-400 (Danisco US Inc.); AMIGASE™ and AMIGASE™ PLUS (DSM); G-ZYME® G900 (Enzyme Bio-Systems); and G-ZYME® G990 ZR (*A*. *niger* glucoamylase with a low protease content). Still other suitable glucoamylases include *Aspergillus fumigatus* glucoamylase, *Talaromyces* glucoamylase, *Thielavia* glucoamylase, *Trametes* glucoamylase, *Thermomyces* glucoamylase, *Athelia* glucoamylase, or *Humicola* glucoamylase (*e.g*., HgGA). Glucoamylases typically are added in an amount of about 0.1 - 2 glucoamylase units (GAU)/g ds, *e.g*., about 0.16 GAU/g ds, 0.23 GAU/g ds, or 0.33 GAU/g ds.

Other suitable enzymes that can be used with amylase include a phytase, protease, pullulanase, β-amylase, isoamylase, a different α-amylase, alpha-glucosidase, cellulase, xylanase, other hemicellulases, beta-glucosidase, transferase, pectinase, lipase, cutinase, esterase, redox enzymes, or a combination thereof. For example, a debranching enzyme, such as an isoamylase (EC 3.2.1.68), may be added in effective amounts well known to the person skilled in the art. A pullulanase (EC 3.2.1.41), *e*.*g*., PROMOZYME®, is also suitable. Pullulanase typically is added at 100 U/kg ds. Further suitable enzymes include proteases, such as fungal and bacterial proteases. Fungal proteases include those obtained from *Aspergillus*, such as *A*. *niger*, *A. awamori*, *A. oryzae*; *Mucor* (*e.g*., *M. miehei*); *Rhizopus*; and *Trichoderma.*

β-Amylases (EC 3.2.1.2) are exo-acting maltogenic amylases, which catalyze the hydrolysis of 1,4-α-glucosidic linkages into amylopectin and related glucose polymers, thereby releasing maltose. β-Amylases have been isolated from various plants and microorganisms. *See* Fogarty et al. (1979) in PROGRESS IN INDUSTRIAL MICROBIOLOGY, Vol. 15, pp. 112-115. These β-Amylases have optimum temperatures in the range from 40°C to 65°C and optimum pH in the range from about 4.5 to about 7.0. Contemplated β-amylases include, but are not limited to, β-amylases from barley SPEZYME® BBA 1500, SPEZYME® DBA, OPTIMALT™ ME, OPTIMALT™ BBA (Danisco US Inc.); and NOVOZYM™ WBA (Novozymes A/S).

Compositions comprising the present amylases may be aqueous or non-aqueous formulations, granules, powders, gels, slurries, pastes, etc., which may further comprise any one or more of the additional enzymes listed, herein, along with buffers, salts, preservatives, water, co-solvents, surfactants, and the like. Such compositions may work in combination with endogenous enzymes or other ingredients already present in a slurry, water bath, washing machine, food or drink product, etc, for example, endogenous plant (including algal) enzymes, residual enzymes from a prior processing step, and the like.

### 5. Compositions and Methods for Baking and Food Preparation

The present invention also relates to a "food composition," including but not limited to a food product, animal feed and/or food/feed additives, comprising an amylase, and methods for preparing such a food composition comprising mixing α-amylase with one or more food ingredients, or uses thereof.

Furthermore, the present invention relates to the use of an amylase in the preparation of a food composition, wherein the food composition is baked subsequent to the addition of the polypeptide of the invention. As used herein the term "baking composition" means any composition and/or additive prepared in the process of providing a baked food product, including but not limited to bakers flour, a dough, a baking additive and/or a baked product. The food composition or additive may be liquid or solid.

As used herein, the term "flour" means milled or ground cereal grain. The term "flour" also may mean Sago or tuber products that have been ground or mashed. In some embodiments, flour may also contain components in addition to the milled or mashed cereal or plant matter. An example of an additional component, although not intended to be limiting, is a leavening agent. Cereal grains include wheat, oat, rye, and barley. Tuber products include tapioca flour, cassava flour, and custard powder. The term "flour" also includes ground corn flour, maize-meal, rice flour, whole-meal flour, self-rising flour, tapioca flour, cassava flour, ground rice, enriched flower, and custard powder.

For the commercial and home use of flour for baking and food production, it is important to maintain an appropriate level of α-amylase activity in the flour. A level of activity that is too high may result in a product that is sticky and/or doughy and therefore unmarketable. Flour with insufficient α-amylase activity may not contain enough sugar for proper yeast function, resulting in dry, crumbly bread, or baked products. Accordingly, an amylase, by itself or in combination with another α-amylase(s), may be added to the flour to augment the level of endogenous α-amylase activity in flour.

An amylase can further be added alone or in a combination with other amylases to prevent or retard staling, *i.e*., crumb firming of baked products. The amount of anti-staling amylase will typically be in the range of 0.01-10 mg of enzyme protein per kg of flour, *e.g*., 0.5 mg/kg ds. Additional anti-staling amylases that can be used in combination with an amylase include an endo-amylase, *e.g*., a bacterial endo-amylase from *Bacillus.* The additional amylase can be another maltogenic α-amylase (EC 3.2.1.133), *e.g*., from *Bacillus.* NOVAMYL® is an exemplary maltogenic α-amylase from *B. stearothermophilus* strain NCIB 11837 and is described in Christophersen et al. (1997) Starch 50:39-45. Other examples of anti-staling endo-amylases include bacterial α-amylases derived from *Bacillus,* such as *B. licheniformis* or *B. amyloliquefaciens.* The anti-staling amylase may be an exo-amylase, such as β-amylase, *e.g*., from plant sources, such as soybean, or from microbial sources, such as *Bacillus.*

The baking composition comprising an amylase further can comprise a phospholipase or enzyme with phospholipase activity. An enzyme with phospholipase activity has an activity that can be measured in Lipase Units (LU). The phospholipase may have A₁ or A₂ activity to remove fatty acid from the phospholipids, forming a lysophospholipid. It may or may not have lipase activity, *i.e*., activity on triglyceride substrates. The phospholipase typically has a temperature optimum in the range of 30-90°C., *e.g*., 30-70°C. The added phospholipases can be of animal origin, for example, from pancreas, *e*.*g*., bovine or porcine pancreas, snake venom or bee venom. Alternatively, the phospholipase may be of microbial origin, *e.g*., from filamentous fungi, yeast or bacteria, for example.

The phospholipase is added in an amount that improves the softness of the bread during the initial period after baking, particularly the first 24 hours. The amount of phospholipase will typically be in the range of 0.01-10 mg of enzyme protein per kg of flour, *e*.*g*., 0.1-5 mg/kg. That is, phospholipase activity generally will be in the range of 20-1000 LU/kg of flour, where a Lipase Unit is defined as the amount of enzyme required to release 1 µmol butyric acid per minute at 30°C, pH 7.0, with gum arabic as emulsifier and tributyrin as substrate.

Compositions of dough generally comprise wheat meal or wheat flour and/or other types of meal, flour or starch such as corn flour, cornstarch, rye meal, rye flour, oat flour, oatmeal, soy flour, sorghum meal, sorghum flour, potato meal, potato flour or potato starch. The dough may be fresh, frozen or par-baked. The dough can be a leavened dough or a dough to be subjected to leavening. The dough may be leavened in various ways, such as by adding chemical leavening agents, *e.g*., sodium bicarbonate or by adding a leaven, *i.e*., fermenting dough. Dough also may be leavened by adding a suitable yeast culture, such as a culture of *Saccharomyces cerevisiae* (baker's yeast), *e.g*., a commercially available strain of *S*. *cerevisiae.*

The dough may also comprise other conventional dough ingredients, *e*.*g*., proteins, such as milk powder, gluten, and soy; eggs (*e.g*., whole eggs, egg yolks or egg whites); an oxidant, such as ascorbic acid, potassium bromate, potassium iodate, azodicarbonamide (ADA) or ammonium persulfate; an amino acid such as L-cysteine; a sugar; or a salt, such as sodium chloride, calcium acetate, sodium sulfate or calcium sulfate. The dough further may comprise fat, *e.g*., triglyceride, such as granulated fat or shortening. The dough further may comprise an emulsifier such as mono- or diglycerides, diacetyl tartaric acid esters of mono- or diglycerides, sugar esters of fatty acids, polyglycerol esters of fatty acids, lactic acid esters of monoglycerides, acetic acid esters of monoglycerides, polyoxyethylene stearates, or lysolecithin. In particular, the dough can be made without addition of emulsifiers.

The dough product may be any processed dough product, including fried, deep fried, roasted, baked, steamed and boiled doughs, such as steamed bread and rice cakes. In one embodiment, the food product is a bakery product. Typical bakery (baked) products include bread - such as loaves, rolls, buns, bagels, pizza bases etc. pastry, pretzels, tortillas, cakes, cookies, biscuits, crackers etc.

Optionally, an additional enzyme may be used together with the anti-staling amylase and the phospholipase. The additional enzyme may be a second amylase, such as an amyloglucosidase, a β-amylase, a cyclodextrin glucanotransferase, or the additional enzyme may be a peptidase, in particular an exopeptidase, a transglutaminase, a lipase, a cellulase, a xylanase, a protease, a protein disulfide isomerase, *e*.*g*., a protein disulfide isomerase as disclosed in WO 95/00636, for example, a glycosyltransferase, a branching enzyme (1,4-α-glucan branching enzyme), a 4-α-glucanotransferase (dextrin glycosyltransferase) or an oxidoreductase, *e*.*g*., a peroxidase, a laccase, a glucose oxidase, an amadoriase, a metalloproteinase, a pyranose oxidase, a lipooxygenase, an L-amino acid oxidase or a carbohydrate oxidase. The additional enzyme(s) may be of any origin, including mammalian and plant, and particularly of microbial (bacterial, yeast or fungal) origin and may be obtained by techniques conventionally used in the art.

The xylanase is typically of microbial origin, *e*.*g*., derived from a bacterium or fungus, such as a strain of *Aspergillus.* Xylanases include PENTOPAN® and NOVOZYM 384®, for example, which are commercially available xylanase preparations produced from *Trichoderma reesei.* The amyloglucosidase may be an *A*. *niger* amyloglucosidase (such as AMG®). Other useful amylase products include GRINDAMYL® A 1000 or A 5000 (Grindsted Products, Denmark) and AMYLASE H™ or AMYLASE P™ (DSM). The glucose oxidase may be a fungal glucose oxidase, in particular an *Aspergillus niger* glucose oxidase (such as GLUZYME®). An exemplary protease is NEUTRASE®.

The process may be used for any kind of baked product prepared from dough, either of a soft or a crisp character, either of a white, light or dark type. Examples are bread, particularly white, whole-meal or rye bread, typically in the form of loaves or rolls, such as, but not limited to, French baguette-type bread, pita bread, tortillas, cakes, pancakes, biscuits, cookies, pie crusts, crisp bread, steamed bread, pizza and the like.

An amylase may be used in a pre-mix, comprising flour together with an anti-staling amylase, a phospholipase, and/or a phospholipid. The pre-mix may contain other dough-improving and/or bread-improving additives, *e*.*g*., any of the additives, including enzymes, mentioned above. An amylase can be a component of an enzyme preparation comprising an anti-staling amylase and a phospholipase, for use as a baking additive.

The enzyme preparation is optionally in the form of a granulate or agglomerated powder. The preparation can have a narrow particle size distribution with more than 95% (by weight) of the particles in the range from 25 to 500 µm. Granulates and agglomerated powders may be prepared by conventional methods, *e*.*g*., by spraying an amylase onto a carrier in a fluid-bed granulator. The carrier may consist of particulate cores having a suitable particle size. The carrier may be soluble or insoluble, *e*.*g*., a salt (such as NaCl or sodium sulfate), a sugar (such as sucrose or lactose), a sugar alcohol (such as sorbitol), starch, rice, corn grits, or soy.

Enveloped particles, *i.e.,* α-amylase particles, can comprise an amylase. To prepare enveloped α-amylase particles, the enzyme is contacted with a food grade lipid in sufficient quantity to suspend all of the α-amylase particles. Food grade lipids, as used herein, may be any naturally organic compound that is insoluble in water but is soluble in non-polar organic solvents such as hydrocarbon or diethyl ether. Suitable food grade lipids include, but are not limited to, triglycerides either in the form of fats or oils that are either saturated or unsaturated. Examples of fatty acids and combinations thereof which make up the saturated triglycerides include, but are not limited to, butyric (derived from milk fat), palmitic (derived from animal and plant fat), and/or stearic (derived from animal and plant fat). Examples of fatty acids and combinations thereof which make up the unsaturated triglycerides include, but are not limited to, palmitoleic (derived from animal and plant fat), oleic (derived from animal and plant fat), linoleic (derived from plant oils), and/or linolenic (derived from linseed oil). Other suitable food grade lipids include, but are not limited to, monoglycerides and diglycerides derived from the triglycerides discussed above, phospholipids and glycolipids.

The food grade lipid, particularly in the liquid form, is contacted with a powdered form of the α-amylase particles in such a fashion that the lipid material covers at least a portion of the surface of at least a majority, *e*.*g*., 100% of the α-amylase particles. Thus, each α-amylase particle is individually enveloped in a lipid. For example, all or substantially all of the α-amylase particles are provided with a thin, continuous, enveloping film of lipid. This can be accomplished by first pouring a quantity of lipid into a container, and then slurrying the α-amylase particles so that the lipid thoroughly wets the surface of each α-amylase particle. After a short period of stirring, the enveloped α-amylase particles, carrying a substantial amount of the lipids on their surfaces, are recovered. The thickness of the coating so applied to the particles of α-amylase can be controlled by selection of the type of lipid used and by repeating the operation in order to build up a thicker film, when desired.

The storing, handling and incorporation of the loaded delivery vehicle can be accomplished by means of a packaged mix. The packaged mix can comprise the enveloped α-amylase. However, the packaged mix may further contain additional ingredients as required by the manufacturer or baker. After the enveloped α-amylase has been incorporated into the dough, the baker continues through the normal production process for that product.

The advantages of enveloping the α-amylase particles are two-fold. First, the food grade lipid protects the enzyme from thermal denaturation during the baking process for those enzymes that are heat labile. Consequently, while the α-amylase is stabilized and protected during the proving and baking stages, it is released from the protective coating in the final baked good product, where it hydrolyzes the glucosidic linkages in polyglucans. The loaded delivery vehicle also provides a sustained release of the active enzyme into the baked good. That is, following the baking process, active α-amylase is continually released from the protective coating at a rate that counteracts, and therefore reduces the rate of, staling mechanisms.

In general, the amount of lipid applied to the α-amylase particles can vary from a few percent of the total weight of the α-amylase to many times that weight, depending upon the nature of the lipid, the manner in which it is applied to the α-amylase particles, the composition of the dough mixture to be treated, and the severity of the dough-mixing operation involved.

The loaded delivery vehicle, *i.e*., the lipid-enveloped enzyme, is added to the ingredients used to prepare a baked good in an effective amount to extend the shelf-life of the baked good. The baker computes the amount of enveloped α-amylase, prepared as discussed above, that will be required to achieve the desired anti-staling effect. The amount of the enveloped α-amylase required is calculated based on the concentration of enzyme enveloped and on the proportion of α-amylase to flour specified. A wide range of concentrations has been found to be effective, although, as has been discussed, observable improvements in anti-staling do not correspond linearly with the α-amylase concentration, but above certain minimal levels, large increases in α-amylase concentration produce little additional improvement. The α-amylase concentration actually used in a particular bakery production could be much higher than the minimum necessary to provide the baker with some insurance against inadvertent under-measurement errors by the baker. The lower limit of enzyme concentration is determined by the minimum anti-staling effect the baker wishes to achieve.

A method of preparing a baked good may comprise: a) preparing lipid-coated α-amylase particles, where substantially all of the α-amylase particles are coated; b) mixing a dough containing flour; c) adding the lipid-coated α-amylase to the dough before the mixing is complete and terminating the mixing before the lipid coating is removed from the α-amylase; d) proofing the dough; and e) baking the dough to provide the baked good, where the α-amylase is inactive during the mixing, proofing and baking stages and is active in the baked good.

The enveloped α-amylase can be added to the dough during the mix cycle, *e.g*., near the end of the mix cycle. The enveloped α-amylase is added at a point in the mixing stage that allows sufficient distribution of the enveloped α-amylase throughout the dough; however, the mixing stage is terminated before the protective coating becomes stripped from the α-amylase particle(s). Depending on the type and volume of dough, and mixer action and speed, anywhere from one to six minutes or more might be required to mix the enveloped α-amylase into the dough, but two to four minutes is average. Thus, several variables may determine the precise procedure. First, the quantity of enveloped α-amylase should have a total volume sufficient to allow the enveloped α-amylase to be spread throughout the dough mix. If the preparation of enveloped α-amylase is highly concentrated, additional oil may need to be added to the pre-mix before the enveloped α-amylase is added to the dough. Recipes and production processes may require specific modifications; however, good results generally can be achieved when 25% of the oil specified in a bread dough formula is held out of the dough and is used as a carrier for a concentrated enveloped α-amylase when added near the end of the mix cycle. In bread or other baked goods, particularly those having a low fat content, *e*.*g*., French-style breads, an enveloped α-amylase mixture of approximately 1% of the dry flour weight is sufficient to admix the enveloped α-amylase properly with the dough. The range of suitable percentages is wide and depends on the formula, finished product, and production methodology requirements of the individual baker. Second, the enveloped α-amylase suspension should be added to the mix with sufficient time for complete mixture into the dough, but not for such a time that excessive mechanical action strips the protective lipid coating from the enveloped α-amylase particles.

In a further aspect of the invention, the food composition is an oil, meat, lard, composition comprising an amylase. In this context the term "oil, meat, lard, composition" means any composition, based on, made from and/or containing oil, meat or lard, respectively. Another aspect the invention relates to a method of preparing an oil or meat or lard composition and/or additive comprising an amylase, comprising mixing the polypeptide of the invention with a oil/meat/lard composition and/or additive ingredients.

In a further aspect of the invention, the food composition is an animal feed composition, animal feed additive and/or pet food comprising an amylase and variants thereof. The present invention further relates to a method for preparing such an animal feed composition, animal feed additive composition and/or pet food comprising mixing an amylase and variants thereof with one or more animal feed ingredients and/or animal feed additive ingredients and/or pet food ingredients. Furthermore, the present invention relates to the use of an amylase in the preparation of an animal feed composition and/or animal feed additive composition and/or pet food.

The term "animal" includes all non-ruminant and ruminant animals. In a particular embodiment, the animal is a non-ruminant animal, such as a horse and a mono-gastric animal. Examples of mono-gastric animals include, but are not limited to, pigs and swine, such as piglets, growing pigs, sows; poultry such as turkeys, ducks, chicken, broiler chicks, layers; fish such as salmon, trout, tilapia, catfish and carps; and crustaceans such as shrimps and prawns. In a further embodiment the animal is a ruminant animal including, but not limited to, cattle, young calves, goats, sheep, giraffes, bison, moose, elk, yaks, water buffalo, deer, camels, alpacas, llamas, antelope, pronghorn and nilgai.

In the present context, it is intended that the term "pet food" is understood to mean a food for a household animal such as, but not limited to dogs, cats, gerbils, hamsters, chinchillas, fancy rats, guinea pigs; avian pets, such as canaries, parakeets, and parrots; reptile pets, such as turtles, lizards and snakes; and aquatic pets, such as tropical fish and frogs.

The terms "animal feed composition," "feedstuff' and "fodder" are used interchangeably and may comprise one or more feed materials selected from the group comprising a) cereals, such as small grains (*e*.*g*., wheat, barley, rye, oats and combinations thereof) and/or large grains such as maize or sorghum; b) by products from cereals, such as corn gluten meal, Distillers Dried Grain Solubles (DDGS) (particularly corn based Distillers Dried Grain Solubles (cDDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

### 6. Textile Desizing Compositions and Use

Also contemplated are compositions and methods of treating fabrics (*e.g*., to desize a textile) using an an amylase. Fabric-treating methods are well known in the art (*see, e.g.,* U.S. Patent No. 6,077,316). For example, the feel and appearance of a fabric can be improved by a method comprising contacting the fabric with an an amylase in a solution. The fabric can be treated with the solution under pressure.

An an amylase can be applied during or after the weaving of a textile, or during the desizing stage, or one or more additional fabric processing steps. During the weaving of textiles, the threads are exposed to considerable mechanical strain. Prior to weaving on mechanical looms, warp yarns are often coated with sizing starch or starch derivatives to increase their tensile strength and to prevent breaking. An an amylase can be applied during or after the weaving to remove these sizing starch or starch derivatives. After weaving, an an amylase can be used to remove the size coating before further processing the fabric to ensure a homogeneous and wash-proof result.

An an amylase can be used alone or with other desizing chemical reagents and/or desizing enzymes to desize fabrics, including cotton-containing fabrics, as detergent additives, *e.g.,* in aqueous compositions. An amylase also can be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes to provide softness to the fabric and make the cotton more accessible to the subsequent enzymatic finishing steps. An amylase can be used in methods of finishing denim garments (*e.g*., a "bio-stoning process"), enzymatic desizing and providing softness to fabrics, and/or finishing process.

### 7. Cleaning Compositions

An aspect of the present compositions and methods is a cleaning composition that includes an amylase as a component. An amylase polypeptide can be used as a component in detergent compositions for hand washing, laundry washing, dishwashing, and other hard-surface cleaning.

### 7.1. Overview

Preferably, an amylase is incorporated into detergents at or near a concentration conventionally used for amylase in detergents. For example, an amylase polypeptide may be added in amount corresponding to 0.00001 - 1 mg (calculated as pure enzyme protein) of amylase per liter of wash/dishwash liquor. Exemplary formulations are provided herein, as exemplified by the following:

An amylase polypeptide may be a component of a detergent composition, as the only enzyme or with other enzymes including other amylolytic enzymes. As such, it may be included in the detergent composition in the form of a non-dusting granulate, a stabilized liquid, or a protected enzyme. Non-dusting granulates may be produced, *e.g*., as disclosed in U.S. Patent Nos. 4,106,991 and 4,661,452 and may optionally be coated by methods known in the art. Examples of waxy coating materials are poly(ethylene oxide) products (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Other enzyme stabilizers are known in the art. Protected enzymes may be prepared according to the method disclosed in for example EP 238 216. Polyols have long been recognized as stabilizers of proteins, as well as improving protein solubility.

The detergent composition may be in any useful form, *e.g.,* as powders, granules, pastes, or liquid. A liquid detergent may be aqueous, typically containing up to about 70% of water and 0% to about 30% of organic solvent. It may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); α-olefinsulfonate (AOS); alkyl sulfate (fatty alcohol sulfate) (AS); alcohol ethoxysulfate (AEOS or AES); secondary alkanesulfonates (SAS); α-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide (as described for example in WO 92/06154).

The detergent composition may additionally comprise one or more other enzymes, such as proteases, another amylolytic enzyme, cutinase, lipase, cellulase, pectate lyase, perhydrolase, xylanase, peroxidase, and/or laccase in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (*e.g*., SKS-6 from Hoechst). The detergent may also be unbuilt, *i.e.* essentially free of detergent builder. The enzymes can be used in any composition compatible with the stability of the enzyme. Enzymes generally can be protected against deleterious components by known forms of encapsulation, for example, by granulation or sequestration in hydro gels. Enzymes, and specifically amylases, either with or without starch binding domains, can be used in a variety of compositions including laundry and dishwashing applications, surface cleaners, as well as in compositions for ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a H₂O₂ source such as perborate or percarbonate, which may be combined with a peracid-forming bleach activator such as tetraacetylethylenediamine (TAED) or nonanoyloxybenzenesulfonate (NOBS). Alternatively, the bleaching system may comprise peroxyacids (*e.g*., the amide, imide, or sulfone type peroxyacids). The bleaching system can also be an enzymatic bleaching system, for example, perhydrolase, such as that described in International PCT Application WO 2005/056783.

The enzymes of the detergent composition may be stabilized using conventional stabilizing agents, *e.g.,* a polyol such as propylene glycol or glycerol; a sugar or sugar alcohol; lactic acid; boric acid or a boric acid derivative such as, *e.g.,* an aromatic borate ester; and the composition may be formulated as described in, *e.g.,* WO 92/19709 and WO 92/19708.

The detergent may also contain other conventional detergent ingredients such as *e.g*., fabric conditioners including clays, foam boosters, suds suppressors, anti-corrosion agents, soil-suspending agents, anti-soil redeposition agents, dyes, bactericides, tarnish inhibiters, optical brighteners, or perfumes.

The pH (measured in aqueous solution at use concentration) is usually neutral or alkaline, *e.g.,* pH about 7.0 to about 11.0.

Particular forms of detergent compositions for inclusion of the present α-amylase are described, below.

### 7.2. Heavy Duty Liquid (HDL) laundry detergent composition

Exemplary HDL laundry detergent compositions includes a detersive surfactant (10%-40% wt/wt), including an anionic detersive surfactant (selected from a group of linear or branched or random chain, substituted or unsubstituted alkyl sulphates, alkyl sulphonates, alkyl alkoxylated sulphate, alkyl phosphates, alkyl phosphonates, alkyl carboxylates, and/or mixtures thereof), and optionally non-ionic surfactant (selected from a group of linear or branched or random chain, substituted or unsubstituted alkyl alkoxylated alcohol, for example a C₈-C₁₈ alkyl ethoxylated alcohol and/or C₆-C₁₂ alkyl phenol alkoxylates), wherein the weight ratio of anionic detersive surfactant (with a hydrophilic index (HIc) of from 6.0 to 9) to non-ionic detersive surfactant is greater than 1: 1. Suitable detersive surfactants also include cationic detersive surfactants (selected from a group of alkyl pyridinium compounds, alkyl quarternary ammonium compounds, alkyl quarternary phosphonium compounds, alkyl ternary sulphonium compounds, and/or mixtures thereof); zwitterionic and/or amphoteric detersive surfactants (selected from a group of alkanolamine sulpho-betaines); ampholytic surfactants; semi-polar non-ionic surfactants and mixtures thereof.

The composition may optionally include, a surfactancy boosting polymer consisting of amphiphilic alkoxylated grease cleaning polymers (selected from a group of alkoxylated polymers having branched hydrophilic and hydrophobic properties, such as alkoxylated polyalkylenimines in the range of 0.05 wt%-10 wt%) and/or random graft polymers (typically comprising of hydrophilic backbone comprising monomers selected from the group consisting of: unsaturated C₁-C₆ carboxylic acids, ethers, alcohols, aldehydes, ketones, esters, sugar units, alkoxy units, maleic anhydride, saturated polyalcohols such as glycerol, and mixtures thereof; and hydrophobic side chain(s) selected from the group consisting of: C₄-C₂₅ alkyl group, polypropylene, polybutylene, vinyl ester of a saturated C₁-C₆ mono-carboxylic acid, C₁-C₆ alkyl ester of acrylic or methacrylic acid, and mixtures thereof.

The composition may include additional polymers such as soil release polymers (include anionically end-capped polyesters, for example SRP1, polymers comprising at least one monomer unit selected from saccharide, dicarboxylic acid, polyol and combinations thereof, in random or block configuration, ethylene terephthalate-based polymers and co-polymers thereof in random or block configuration, for example Repel-o-tex SF, SF-2 and SRP6, Texcare SRA100, SRA300, SRN100, SRN170, SRN240, SRN300 and SRN325, Marloquest SL), antiredeposition polymers (0.1 wt% to 10 wt%, include carboxylate polymers, such as polymers comprising at least one monomer selected from acrylic acid, maleic acid (or maleic anhydride), fumaric acid, itaconic acid, aconitic acid, mesaconic acid, citraconic acid, methylenemalonic acid, and any mixture thereof, vinylpyrrolidone homopolymer, and/or polyethylene glycol, molecular weight in the range of from 500 to 100,000 Da); cellulosic polymer (including those selected from alkyl cellulose, alkyl alkoxyalkyl cellulose, carboxyalkyl cellulose, alkyl carboxyalkyl cellulose examples of which include carboxymethyl cellulose, methyl cellulose, methyl hydroxyethyl cellulose, methyl carboxymethyl cellulose, and mixures thereof) and polymeric carboxylate (such as maleate/acrylate random copolymer or polyacrylate homopolymer).

The composition may further include saturated or unsaturated fatty acid, preferably saturated or unsaturated C₁₂-C₂₄ fatty acid (0 wt% to 10 wt%); deposition aids (examples for which include polysaccharides, preferably cellulosic polymers, poly diallyl dimethyl ammonium halides (DADMAC), and co-polymers of DAD MAC with vinyl pyrrolidone, acrylamides, imidazoles, imidazolinium halides, and mixtures thereof, in random or block configuration, cationic guar gum, cationic cellulose such as cationic hydoxyethyl cellulose, cationic starch, cationic polyacylamides, and mixtures thereof.

The composition may further include dye transfer inhibiting agents, examples of which include manganese phthalocyanine, peroxidases, polyvinylpyrrolidone polymers, polyamine N-oxide polymers, copolymers of N-vinylpyrrolidone and N-vinylimidazole, polyvinyloxazolidones and polyvinylimidazoles and/or mixtures thereof; chelating agents, examples of which include ethylene-diamine-tetraacetic acid (EDTA), diethylene triamine penta methylene phosphonic acid (DTPMP), hydroxy-ethane diphosphonic acid (HEDP), ethylenediamine N,N'-disuccinic acid (EDDS), methyl glycine diacetic acid (MGDA), diethylene triamine penta acetic acid (DTPA), propylene diamine tetracetic acid (PDT A), 2-hydroxypyridine-N-oxide (HPNO), or methyl glycine diacetic acid (MGDA), glutamic acid N,N-diacetic acid (N,N-dicarboxymethyl glutamic acid tetrasodium salt (GLDA), nitrilotriacetic acid (NTA), 4,5-dihydroxy-m-benzenedisulfonic acid, citric acid and any salts thereof, N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), and derivatives thereof.

The composition preferably included enzymes (generally about 0.01 wt% active enzyme to 0.03 wt% active enzyme) selected from proteases, amylases, lipases, cellulases, choline oxidases, peroxidases/oxidases, pectate lyases, mannanases, cutinases, laccases, phospholipases, lysophospholipases, acyltransferases, perhydrolases, arylesterases, and any mixture thereof. The composition may include an enzyme stabilizer (examples of which include polyols such as propylene glycol or glycerol, sugar or sugar alcohol, lactic acid, reversible protease inhibitor, boric acid, or a boric acid derivative, *e.g*., an aromatic borate ester, or a phenyl boronic acid derivative such as 4-formylphenyl boronic acid).

The composition optionally include silicone or fatty-acid based suds suppressors; heuing dyes, calcium and magnesium cations, visual signaling ingredients, anti-foam (0.001 wt% to about 4.0 wt%), and/or structurant/thickener (0.01 wt% to 5 wt%, selected from the group consisting of diglycerides and triglycerides, ethylene glycol distearate, microcrystalline cellulose, cellulose based materials, microfiber cellulose, biopolymers, xanthan gum, gellan gum, and mixtures thereof).

The composition can be any liquid form, for example a liquid or gel form, or any combination thereof. The composition may be in any unit dose form, for example a pouch.

### 7.3. Heavy Duty Dry/Solid (HDD) laundry detergent composition

Exemplary HDD laundry detergent compositions includes a detersive surfactant, including anionic detersive surfactants (e.g., linear or branched or random chain, substituted or unsubstituted alkyl sulphates, alkyl sulphonates, alkyl alkoxylated sulphate, alkyl phosphates, alkyl phosphonates, alkyl carboxylates and/or mixtures thereof), non-ionic detersive surfactant (*e*.*g*., linear or branched or random chain, substituted or unsubstituted C₈-C₁₈ alkyl ethoxylates, and/or C₆-C₁₂ alkyl phenol alkoxylates), cationic detersive surfactants (*e*.*g*., alkyl pyridinium compounds, alkyl quaternary ammonium compounds, alkyl quaternary phosphonium compounds, alkyl ternary sulphonium compounds, and mixtures thereof), zwitterionic and/or amphoteric detersive surfactants (*e*.*g*., alkanolamine sulpho-betaines), ampholytic surfactants, semi-polar non-ionic surfactants, and mixtures thereof; builders including phosphate free builders (for example zeolite builders examples which include zeolite A, zeolite X, zeolite P and zeolite MAP in the range of 0 wt% to less than 10 wt%), phosphate builders (for example sodium tri-polyphosphate in the range of 0wt% to less than 10 wt%), citric acid, citrate salts and nitrilotriacetic acid, silicate salt (*e*.*g*., sodium or potassium silicate or sodium meta-silicate in the range of 0 wt% to less than 10 wt%, or layered silicate (SKS-6)); carbonate salt (*e.g*., sodium carbonate and/or sodium bicarbonate in the range of 0 wt% to less than 80 wt%); and bleaching agents including photobleaches (*e.g*., sulfonated zinc phthalocyanines, sulfonated aluminum phthalocyanines, xanthenes dyes, and mixtures thereof) hydrophobic or hydrophilic bleach activators (*e.g*., dodecanoyl oxybenzene sulfonate, decanoyl oxybenzene sulfonate, decanoyl oxybenzoic acid or salts thereof, 3,5,5-trimethy hexanoyl oxybenzene sulfonate, tetraacetyl ethylene diamine-TAED, nonanoyloxybenzene sulfonate-NOBS, nitrile quats, and mixtures thereof), sources of hydrogen peroxide (*e.g*., inorganic perhydrate salts examples of which include mono or tetra hydrate sodium salt of perborate, percarbonate, persulfate, perphosphate, or persilicate), preformed hydrophilic and/or hydrophobic peracids (*e.g*., percarboxylic acids and salts, percarbonic acids and salts, perimidic acids and salts, peroxymonosulfuric acids and salts, and mixtures thereof), and/or bleach catalysts (*e*.*g*., imine bleach boosters (examples of which include iminium cations and polyions), iminium zwitterions, modified amines, modified amine oxides, N-sulphonyl imines, N-phosphonyl imines, N-acyl imines, thiadiazole dioxides, perfluoroimines, cyclic sugar ketones, and mixtures thereof, and metal-containing bleach catalysts (*e.g*., copper, iron, titanium, ruthenium, tungsten, molybdenum, or manganese cations along with an auxiliary metal cations such as zinc or aluminum and a sequestrate such as ethylenediaminetetraacetic acid, ethylenediaminetetra(methylenephosphonic acid), and watersoluble salts thereof).

The composition preferably includes enzymes, *e*.*g*., proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and any mixture thereof.

The composition may optionally include additional detergent ingredients including perfume microcapsules, starch encapsulated perfume accord, hueing agents, additional polymers, including fabric integrity and cationic polymers, dye-lock ingredients, fabric-softening agents, brighteners (for example C.I. Fluorescent brighteners), flocculating agents, chelating agents, alkoxylated polyamines, fabric deposition aids, and/or cyclodextrin.

### 7.4. Automatic dishwashing (ADW) detergent composition

Exemplary ADW detergent composition includes non-ionic surfactants, including ethoxylated non-ionic surfactants, alcohol alkoxylated surfactants, epoxy-capped poly(oxyalkylated) alcohols, or amine oxide surfactants present in amounts from 0 to 10% by weight; builders in the range of 5-60% including phosphate builders (e.g., mono-phosphates, di-phosphates, tri-polyphosphates, other oligomeric-poylphosphates, sodium tripolyphosphate-STPP) and phosphate-free builders (*e*.*g*., amino acid-based compounds including methylglycine-diacetic acid (MGDA) and salts and derivatives thereof, glutamic-N,N-diacetic acid (GLDA) and salts and derivatives thereof, iminodisuccinic acid (IDS) and salts and derivatives thereof, carboxy methyl inulin and salts and derivatives thereof, nitrilotriacetic acid (NTA), diethylene triamine penta acetic acid (DTPA), B-alaninediacetic acid (B-ADA) and their salts, homopolymers and copolymers of poly-carboxylic acids and their partially or completely neutralized salts, monomeric polycarboxylic acids and hydroxycarboxylic acids and their salts in the range of 0.5% to 50% by weight; sulfonated/carboxylated polymers in the range of about 0.1% to about 50% by weight to to provide dimensional stability; drying aids in the range of about 0.1% to about 10% by weight (*e.g*., polyesters, especially anionic polyesters, optionally together with further monomers with 3 to 6 functionalities - typically acid, alcohol or ester functionalities which are conducive to polycondensation, polycarbonate-, polyurethane- and/or polyurea-polyorganosiloxane compounds or precursor compounds, thereof, particularly of the reactive cyclic carbonate and urea type); silicates in the range from about 1% to about 20% by weight (including sodium or potassium silicates for example sodium disilicate, sodium meta-silicate and crystalline phyllosilicates); inorganic bleach (*e.g*., perhydrate salts such as perborate, percarbonate, perphosphate, persulfate and persilicate salts) and organic bleach (*e*.*g*., organic peroxyacids, including diacyl and tetraacylperoxides, especially diperoxydodecanedioc acid, diperoxytetradecanedioc acid, and diperoxyhexadecanedioc acid); bleach activators (*i*.*e*., organic peracid precursors in the range from about 0.1% to about 10% by weight); bleach catalysts (*e.g*., manganese triazacyclononane and related complexes, Co, Cu, Mn, and Fe bispyridylamine and related complexes, and pentamine acetate cobalt(III) and related complexes); metal care agents in the range from about 0.1% to 5% by weight (*e.g*., benzatriazoles, metal salts and complexes, and/or silicates); enzymes in the range from about 0.01 to 5.0 mg of active enzyme per gram of automatic dishwashing detergent composition (*e.g*., proteases, amylases, lipases, cellulases, choline oxidases, peroxidases/oxidases, pectate lyases, mannanases, cutinases, laccases, phospholipases, lysophospholipases, acyltransferase, perhydrolase, arylesterase, and mixtures thereof); and enzyme stabilizer components (*e.g*., oligosaccharides, polysaccharides, and inorganic divalent metal salts).

### 7.5. Additional detergent compositions

Additional exemplary detergent formulations to which the present amylase can be added are described, below, in the numbered paragraphs.
1) A detergent composition formulated as a granulate having a bulk density of at least 600 g/L comprising linear alkylbenzenesulfonate (calculated as acid) about 7% to about 12%; alcohol ethoxysulfate (*e*.*g*., C₁₂₋₁₈ alcohol, 1-2 ethylene oxide (EO)) or alkyl sulfate (*e*.*g*., C₁₆₋₁₈) about 1% to about 4%; alcohol ethoxylate (*e.g*., C₁₄₋₁₅ alcohol, 7 EO) about 5% to about 9%; sodium carbonate (*e*.*g*., Na₂CO₃) about 14% to about 20%; soluble silicate (*e*.*g*., Na₂O, 2SiO₂) about 2 to about 6%; zeolite (*e.g*., NaA1SiO₄) about 15% to about 22%; sodium sulfate (*e*.*g*., Na₂SO₄) 0% to about 6%; sodium citrate/citric acid (*e*.*g*., C₆H₅Na₃O₇/C₆H₈O₇) about 0% to about 15%; sodium perborate (*e.g*., NaBO₃H₂O) about 11% to about 18%; TAED about 2% to about 6%; carboxymethylcellulose (CMC) and 0% to about 2%; polymers (*e.g*., maleic/acrylic acid, copolymer, PVP, PEG) 0-3%; enzymes (calculated as pure enzyme) 0.0001-0.1% protein; and minor ingredients (*e.g*., suds suppressors, perfumes, optical brightener, photobleach) 0-5%.
2) A detergent composition formulated as a granulate having a bulk density of at least 600 g/L comprising linear alkylbenzenesulfonate (calculated as acid) about 6% to about 11%; alcohol ethoxysulfate (*e*.*g*., C₁₂₋₁₈ alcohol, 1-2 EO) or alkyl sulfate (*e*.*g*., C₁₆₋₁₈) about 1% to about 3%; alcohol ethoxylate (*e*.*g*., C₁₄₋₁₅ alcohol, 7 EO) about 5% to about 9%; sodium carbonate (*e*.*g*., Na₂CO₃) about 15% to about 21%; soluble silicate (*e*.*g*., Na₂O, 2SiO₂) about 1% to about 4%; zeolite (*e*.*g*., NaA1SiO₄) about 24% to about 34%; sodium sulfate (*e*.*g*,. Na₂SO₄) about 4% to about 10%; sodium citrate/citric acid (*e*.*g*., C₆H₅Na₃O₇/C₆H₈O₇) 0% to about 15%; carboxymethylcellulose (CMC) 0% to about 2%; polymers (*e.g*., maleic/acrylic acid copolymer, PVP, PEG) 1-6%; enzymes (calculated as pure enzyme protein) 0.0001-0.1%; minor ingredients *(e.g.,* suds suppressors, perfume) 0-5%.
3) A detergent composition formulated as a granulate having a bulk density of at least 600 g/L comprising linear alkylbenzenesulfonate (calculated as acid) about 5% to about 9%; alcohol ethoxylate (*e*.*g*., C₁₂₋₁₅ alcohol, 7 EO) about 7% to about 14%; Soap as fatty acid (*e*.*g*., C₁₆₋₂₂ fatty acid) about 1% to about 3%; sodium carbonate (as Na₂CO₃) about 10% to about 17%; soluble silicate (*e.g*., Na₂O, 2SiO₂) about 3% to about 9%; zeolite (as NaA1SiO₄) about 23% to about 33%; sodium sulfate (*e*.*g*., Na₂SO₄) 0% to about 4%; sodium perborate (*e*.*g*., NaBO₃H₂O) about 8% to about 16%; TAED about 2% to about 8%; phosphonate (*e.g*., EDTMPA) 0% to about 1%; carboxymethylcellulose (CMC) 0% to about 2%; polymers (*e.g*., maleic/acrylic acid copolymer, PVP, PEG) 0-3%; enzymes (calculated as pure enzyme protein) 0.0001-0.1%; minor ingredients (*e.g*., suds suppressors, perfume, optical brightener) 0-5%.
4) A detergent composition formulated as a granulate having a bulk density of at least 600 g/L comprising linear alkylbenzenesulfonate (calculated as acid) about 8% to about 12%; alcohol ethoxylate (*e.g*., C₁₂₋₁₅ alcohol, 7 EO) about 10% to about 25%; sodium carbonate (as Na₂CO₃) about 14% to about 22%; soluble silicate (*e.g*., Na₂O, 2SiO₂) about 1% to about 5%; zeolite (*e*.*g*., NaA1SiO₄) about 25% to about 35%; sodium sulfate (*e*.*g*., Na₂SO₄) 0% to about 10%; carboxymethylcellulose (CMC) 0% to about 2%; polymers (*e.g*., maleic/acrylic acid copolymer, PVP, PEG) 1-3%; enzymes (calculated as pure enzyme protein) 0.0001-0.1%; and minor ingredients (*e.g*., suds suppressors, perfume) 0-5%.
5) An aqueous liquid detergent composition comprising linear alkylbenzenesulfonate (calculated as acid) about 15% to about 21%; alcohol ethoxylate (*e*.*g*., C₁₂₋₁₅ alcohol, 7 EO or C₁₂₋₁₅ alcohol, 5 EO) about 12% to about 18%; soap as fatty acid (*e*.*g*., oleic acid) about 3% to about 13%; alkenylsuccinic acid (C₁₂₋₁₄) 0% to about 13%; aminoethanol about 8% to about 18%; citric acid about 2% to about 8%; phosphonate 0% to about 3%; polymers (*e.g*., PVP, PEG) 0% to about 3%; borate (*e*.*g*., B₄O₇) 0% to about 2%; ethanol 0% to about 3%; propylene glycol about 8% to about 14%; enzymes (calculated as pure enzyme protein) 0.0001-0.1%; and minor ingredients (*e.g*., dispersants, suds suppressors, perfume, optical brightener) 0-5%.
6) An aqueous structured liquid detergent composition comprising linear alkylbenzenesulfonate (calculated as acid) about 15% to about 21%; alcohol ethoxylate (*e*.*g*., C₁₂₋₁₅ alcohol, 7 EO, or C₁₂₋₁₅ alcohol, 5 EO) 3-9%; soap as fatty acid (*e*.*g*., oleic acid) about 3% to about 10%; zeolite (as NaA1SiO₄) about 14% to about 22%; potassium citrate about 9% to about 18%; borate (*e*.*g*., B₄O₇) 0% to about 2%; carboxymethylcellulose (CMC) 0% to about 2%; polymers (*e*.*g*., PEG, PVP) 0% to about 3%; anchoring polymers such as, *e*.*g*., lauryl methacrylate/acrylic acid copolymer; molar ratio 25:1, MW 3800) 0% to about 3%;glycerol 0% to about 5%; enzymes (calculated as pure enzyme protein) 0.0001-0.1%; and minor ingredients (*e.g*., dispersants, suds suppressors, perfume, optical brighteners) 0-5%.
7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate (*e.g*., Na₂CO₃) about 5% to about 10%; Soluble silicate (*e.g*., Na₂O, 2SiO₂) about 1% to about 4%; zeolite (*e*.*g*., NaA1SiO₄) about 20% to about 40%; Sodium sulfate (*e*.*g*., Na₂SO₄) about 2% to about 8%; sodium perborate (e.g., NaBO₃H₂O) about 12% to about 18%; TAED about 2% to about 7%; polymers (*e*.*g*., maleic/acrylic acid copolymer, PEG) about 1% to about 5%; enzymes (calculated as pure enzyme protein) 0.0001-0.1%; and minor ingredients *(e.g.,* optical brightener, suds suppressors, perfume) 0-5%.
8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (e.g., Na₂CO₃) about 4% to about 10%; soluble silicate (Na₂O, 2SiO₂) about 1% to about 4%; zeolite *(e.g.,* NaA1SiO₄) about 30% to about 50%; sodium sulfate *(e.g.,* Na₂SO₄) about 3% to about 11%; sodium citrate (e.g., C₆H₅Na₃O₇) about 5% to about 12%; polymers *(e.g.,* PVP, maleic/acrylic acid copolymer, PEG) about 1% to about 5%; enzymes (calculated as pure enzyme protein) 0.0001-0.1%; and minor ingredients (e.g., suds suppressors, perfume) 0-5%.
9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e*.*g*., Na₂CO₃) about 14% to about 22%; zeolite (*e.g*., NaA1SiO₄) about 18% to about 32%; sodium sulfate (*e*.*g*., Na₂SO₄) about 5% to about 20%; sodium citrate (*e*.*g*., C₆H₅Na₃O₇) about 3% to about 8%; sodium perborate (*e*.*g*., NaBO₃H₂O) about 4% to about 9%; bleach activator (*e*.*g*., NOBS or TAED) about 1% to about 5%; carboxymethylcellulose (CMC) 0% to about 2%; polymers (*e*.*g*., polycarboxylate or PEG) about 1% to about 5%; enzymes (calculated as pure enzyme protein) 0.0001-0.1%; and minor ingredients (*e.g*., optical brightener, perfume) 0-5%.
10) An aqueous liquid detergent composition comprising linear alkylbenzenesulfonate (calculated as acid) about 15% to about 23%; alcohol ethoxysulfate (*e*.*g*., C₁₂₋₁₅ alcohol, 2-3 EO) about 8% to about 15%; alcohol ethoxylate (*e.g*., C₁₂₋₁₅ alcohol, 7 EO, or C₁₂₋₁₅ alcohol, 5 EO) about 3% to about 9%; soap as fatty acid (*e*.*g*., lauric acid) 0% to about 3%; aminoethanol about 1% to about 5%; sodium citrate about 5% to about 10%; hydrotrope (*e*.*g*., sodium toluensulfonate) about 2% to about 6%; borate (*e*.*g*., B₄O₇) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e*.*g*., polymers, dispersants, perfume, optical brighteners) 0-5%.
11) An aqueous liquid detergent composition comprising linear alkylbenzenesulfonate (calculated as acid) about 20% to about 32%; alcohol ethoxylate (*e*.*g*., C₁₂₋₁₅ alcohol, 7 EO, or C₁₂₋₁₅ alcohol, 5 EO) 6-12%; aminoethanol about 2% to about 6%; citric acid about 8% to about 14%; borate (*e*.*g*., B₄O₇) about 1% to about 3%; polymer (*e*.*g*., maleic/acrylic acid copolymer, anchoring polymer such as, *e.g.,* lauryl methacrylate/acrylic acid copolymer) 0% to about 3%; glycerol about 3% to about 8%; enzymes (calculated as pure enzyme protein) 0.0001-0.1%; and minor ingredients (*e*.*g*., hydrotropes, dispersants, perfume, optical brighteners) 0-5%.
12) A detergent composition formulated as a granulate having a bulk density of at least 600 g/L comprising anionic surfactant (linear alkylbenzenesulfonate, alkyl sulfate, α-olefinsulfonate, α-sulfo fatty acid methyl esters, alkanesulfonates, soap) about 25% to about 40%; nonionic surfactant (*e*.*g*., alcohol ethoxylate) about 1% to about 10%; sodium carbonate (*e*.*g*., Na₂CO₃) about 8% to about 25%; soluble silicates (*e*.*g*., Na₂O, 2SiO₂) about 5% to about 15%; sodium sulfate (*e*.*g*., Na₂SO₄) 0% to about 5%; zeolite (NaA1SiO₄) about 15% to about 28%; sodium perborate (*e*.*g*., NaBO₃·4H₂O) 0% to about 20%; bleach activator (TAED or NOBS) about 0% to about 5%; enzymes (calculated as pure enzyme protein) 0.0001-0.1%; minor ingredients (*e*.*g*., perfume, optical brighteners) 0-3%.
13) Detergent compositions as described in compositions 1)-12) *supra,* wherein all or part of the linear alkylbenzenesulfonate is replaced by (C₁₂-C₁₈) alkyl sulfate.
14) A detergent composition formulated as a granulate having a bulk density of at least 600 g/L comprising (C₁₂-C₁₈) alkyl sulfate about 9% to about 15%; alcohol ethoxylate about 3% to about 6%; polyhydroxy alkyl fatty acid amide about 1% to about 5%; zeolite (*e.g*., NaA1SiO₄) about 10% to about 20%; layered disilicate (*e*.*g*., SK56 from Hoechst) about 10% to about 20%; sodium carbonate (*e*.*g*., Na₂CO₃) about 3% to about 12%; soluble silicate (*e*.*g*., Na₂O, 2SiO₂) 0% to about 6%; sodium citrate about 4% to about 8%; sodium percarbonate about 13% to about 22%; TAED about 3% to about 8%; polymers (*e*.*g*., polycarboxylates and PVP) 0% to about 5%; enzymes (calculated as pure enzyme protein) 0.0001-0.1%; and minor ingredients (*e*.*g*., optical brightener, photobleach, perfume, suds suppressors) 0-5%.
15) A detergent composition formulated as a granulate having a bulk density of at least 600 g/L comprising (C₁₂-C₁₈) alkyl sulfate about 4% to about 8%; alcohol ethoxylate about 11% to about 15%; soap about 1% to about 4%; zeolite MAP or zeolite A about 35% to about 45%; sodium carbonate (as Na₂CO₃) about 2% to about 8%; soluble silicate (*e*.*g*., Na₂O, 2SiO₂) 0% to about 4%; sodium percarbonate about 13% to about 22%; TAED 1-8%; carboxymethylcellulose (CMC) 0% to about 3%; polymers (*e*.*g*., polycarboxylates and PVP) 0% to about 3%; enzymes (calculated as pure enzyme protein) 0.0001-0.1%; and minor ingredients *(e.g.,* optical brightener, phosphonate, perfume) 0-3%.
16) Detergent formulations as described in 1)-15) supra, which contain a stabilized or encapsulated peracid, either as an additional component or as a substitute for already specified bleach systems.
17) Detergent compositions as described supra in 1), 3), 7), 9), and 12), wherein perborate is replaced by percarbonate.
18) Detergent compositions as described supra in 1), 3), 7), 9), 12), 14), and 15), which additionally contain a manganese catalyst. The manganese catalyst for example is one of the compounds described in "Efficient manganese catalysts for low-temperature bleaching," Nature 369: 637-639 (1994).
19) Detergent composition formulated as a non-aqueous detergent liquid comprising a liquid nonionic surfactant such as, *e.g.,* linear alkoxylated primary alcohol, a builder system (*e*.*g*., phosphate), an enzyme(s), and alkali. The detergent may also comprise anionic surfactant and/or a bleach system.

As above, the present amylase polypeptide may be incorporated at a concentration conventionally employed in detergents. It is at present contemplated that, in the detergent composition, the enzyme may be added in an amount corresponding to 0.00001-1.0 mg (calculated as pure enzyme protein) of amylase polypeptide per liter of wash liquor.

The detergent composition may also contain other conventional detergent ingredients, *e*.*g*., deflocculant material, filler material, foam depressors, anti-corrosion agents, soil-suspending agents, sequestering agents, anti-soil redeposition agents, dehydrating agents, dyes, bactericides, fluorescers, thickeners, and perfumes.

The detergent composition may be formulated as a hand (manual) or machine (automatic) laundry detergent composition, including a laundry additive composition suitable for pre-treatment of stained fabrics and a rinse added fabric softener composition, or be formulated as a detergent composition for use in general household hard surface cleaning operations, or be formulated for manual or automatic dishwashing operations.

Any of the cleaning compositions described, herein, may include any number of additional enzymes. In general the enzyme(s) should be compatible with the selected detergent, (e.g., with respect to pH-optimum, compatibility with other enzymatic and non-enzymatic ingredients, and the like), and the enzyme(s) should be present in effective amounts. The following enzymes are provided as examples.

*Proteases:* Suitable proteases include those of animal, vegetable or microbial origin. Chemically modified or protein engineered mutants are included, as well as naturally processed proteins. The protease may be a serine protease or a metalloprotease, an alkaline microbial protease, a trypsin-like protease, or a chymotrypsin-like protease. Examples of alkaline proteases are subtilisins, especially those derived from *Bacillus*, *e*.*g*., subtilisin Novo, subtilisin Carlsberg, subtilisin 309, subtilisin 147, and subtilisin 168 (*see*, *e*.*g*., WO 89/06279). Examples of trypsin-like proteases are trypsin (*e*.*g*., of porcine or bovine origin), and *Fusarium* proteases (*see*, *e*.*g*., WO 89/06270 and WO 94/25583). Examples of useful proteases also include but are not limited to the variants described in WO 92/19729, WO 98/20115, WO 98/20116, and WO 98/34946. Commercially available protease enzymes include but are not limited to: ALCALASE®, SAVINASE®, PRIMASE™, DURALASE™, ESPERASE®, KANNASE™, and BLAZE™ (Novo Nordisk A/S and Novozymes A/S); MAXATASE®, MAXACAL™, MAXAPEM™, PROPERASE®, PURAFECT®, PURAFECT OXP™, FN2™, and FN3™ (Danisco US Inc.). Other exemplary proteases include NprE from *Bacillus amyloliquifaciens* and ASP from *Cellulomonas* sp. strain 69B4.

*Lipases:* Suitable lipases include those of bacterial or fungal origin. Chemically modified, proteolytically modified, or protein engineered mutants are included. Examples of useful lipases include but are not limited to lipases from *Humicola* (synonym *Thermomyces*), *e.g.,* from *H. lanuginosa (T. lanuginosus*) (*see e*.*g*., EP 258068 and EP 305216), from *H. insolens* (*see e*.*g*., WO 96/13580); a *Pseudomonas* lipase (*e*.*g*., from *P. alcaligenes* or *P. pseudoalcaligenes*; *see*, *e.g.,* EP 218 272), *P. cepacia* (*see e*.*g*., EP 331 376), *P. stutzeri* (*see e*.*g*., GB 1,372,034), *P. fluorescens, Pseudomonas* sp. strain SD 705 (*see e*.*g*., WO 95/06720 and WO 96/27002), *P. wisconsinensis* (*see e*.*g*., WO 96/12012); a *Bacillus* lipase *(e.g.,* from *B*. *subtilis*; *see e.g.,* Dartois et al. Biochemica et Biophysica Acta, 1131: 253-360 (1993)), *B. stearothermophilus* (*see e.g*., JP 64/744992), or *B. pumilus* (*see e.g*., WO 91/16422). Additional lipase variants contemplated for use in the formulations include those described for example in: WO 92/05249, WO 94/01541, WO 95/35381, WO 96/00292, WO 95/30744, WO 94/25578, WO 95/14783, WO 95/22615, WO 97/04079, WO 97/07202, EP 407225, and EP 260105. Some commercially available lipase enzymes include LIPOLASE® and LIPOLASE ULTRA™ (Novo Nordisk A/S and Novozymes A/S).

*Polyesterases*: Suitable polyesterases can be included in the composition, such as those described in, for example, WO 01/34899, WO 01/14629, and US6933140.

Amylases: The compositions can be combined with other amylases, such as non-production enhanced amylase. These can include commercially available amylases, such as but not limited to STAINZYME®, NATALASE®, Duramyl®, Termamyl®, Fungamyl® and BAN™ (Novo Nordisk A/S and Novozymes A/S); Rapidase®, POWERASE®, and Purastar® (from Danisco US Inc.).

Cellulases: Cellulases can be added to the compositions. Suitable cellulases include those of bacterial or fungal origin. Chemically modified or protein engineered mutants are included. Suitable cellulases include cellulases from the genera *Bacillus*, *Pseudomonas*, *Humicola*, *Fusarium*, *Thielavia*, *Acremonium*, *e*.*g*., the fungal cellulases produced from *Humicola insolens*, *Myceliophthora thermophila* and *Fusarium oxysporum* disclosed for example in U.S. Patent Nos. 4,435,307; 5,648,263; 5,691,178; 5,776,757; and WO 89/09259. Exemplary cellulases contemplated for use are those having color care benefit for the textile. Examples of such cellulases are cellulases described in for example EP 0495257, EP 0531372, WO 96/11262, WO 96/29397, and WO 98/08940. Other examples are cellulase variants, such as those described in WO 94/07998; WO 98/12307; WO 95/24471; PCT/DK98/00299; EP 531315; U.S. Patent Nos. 5,457,046; 5,686,593; and 5,763,254. Commercially available cellulases include CELLUZYME® and CAREZYME® (Novo Nordisk A/S and Novozymes A/S); CLAZINASE® and PURADAX HA® (Danisco US Inc.); and KAC-500(B)™ (Kao Corporation).

*Peroxidases*/*Oxidases*: Suitable peroxidases/oxidases contemplated for use in the compositions include those of plant, bacterial or fungal origin. Chemically modified or protein engineered mutants are included. Examples of useful peroxidases include peroxidases from *Coprinus*, *e*.*g*., from *C*. *cinereus*, and variants thereof as those described in WO 93/24618, WO 95/10602, and WO 98/15257. Commercially available peroxidases include for example GUARDZYME™ (Novo Nordisk A/S and Novozymes A/S).

The detergent composition can also comprise 2,6-β-D-fructan hydrolase, which is effective for removal/cleaning of biofilm present on household and/or industrial textile/laundry.

The detergent enzyme(s) may be included in a detergent composition by adding separate additives containing one or more enzymes, or by adding a combined additive comprising all of these enzymes. A detergent additive, *i.e.* a separate additive or a combined additive, can be formulated *e*.*g*., as a granulate, a liquid, a slurry, and the like. Exemplary detergent additive formulations include but are not limited to granulates, in particular non-dusting granulates, liquids, in particular stabilized liquids or slurries.

Non-dusting granulates may be produced, *e.g*., as disclosed in U.S. Patent Nos. 4,106,991 and 4,661,452 and may optionally be coated by methods known in the art. Examples of waxy coating materials are poly(ethylene oxide) products (*e*.*g*., polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition may be in any convenient form, *e.g*., a bar, a tablet, a powder, a granule, a paste, or a liquid. A liquid detergent may be aqueous, typically containing up to about 70% water, and 0% to about 30% organic solvent. Compact detergent gels containing about 30% or less water are also contemplated. The detergent composition can optionally comprise one or more surfactants, which may be non-ionic, including semi-polar and/or anionic and/or cationic and/or zwitterionic. The surfactants can be present in a wide range, from about 0.1% to about 60% by weight.

When included therein the detergent will typically contain from about 1% to about 40% of an anionic surfactant, such as linear alkylbenzenesulfonate, α-olefinsulfonate, alkyl sulfate (fatty alcohol sulfate), alcohol ethoxysulfate, secondary alkanesulfonate, α-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (*e*.*g*.,SKS-6 from Hoechst).

The detergent may comprise one or more polymers. Exemplary polymers include carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates *e*.*g*., polyacrylates, maleic/acrylic acid copolymers), and lauryl methacrylate/acrylic acid copolymers.

The enzyme(s) of the detergent composition may be stabilized using conventional stabilizing agents, *e.g*., as polyol (*e*.*g*., propylene glycol or glycerol), a sugar or sugar alcohol, lactic acid, boric acid, or a boric acid derivative (*e.g*., an aromatic borate ester), or a phenyl boronic acid derivative (*e*.*g*., 4-formylphenyl boronic acid). The composition may be formulated as described in WO 92/19709 and WO 92/19708.

It is contemplated that in the detergent compositions, in particular the enzyme variants, may be added in an amount corresponding to about 0.01 to about 100 mg of enzyme protein per liter of wash liquor (*e.g*., about 0.05 to about 5.0 mg of enzyme protein per liter of wash liquor or 0.1 to about 1.0 mg of enzyme protein per liter of wash liquor).

Yet additional exemplary detergent formulations to which the present amylase can be added are described in, *e.g*., WO2010065455, WO2011072099, WO2011130222, WO2011140364, WO2011156297, WO2011156298, WO2011130076, WO2011133381, WO2011156297, WO2011156298, EP1794295B1, US20110195481, US20110212876, US20110257063, WO2010039958, WO2011072117, WO2011098531, WO2011100410, WO2011130076, WO2011133381, WO2011140316, US20070215184, US20070251545, US20090075857, US20090137444, US20090143271, US20100011513, US20100093588, US20110201536, US20110232004, US20110237482, US20110312868, US20120003326, US20120004155, WO2011131585, EP707628B1, US5719115, EP736084B1, US5783545, EP767830B1, US5972668, EP746599B1, US5798328, EP662117B1, US5898025, US6380140, EP898613B1, US3975280, US6191092, US6329333, US6530386, EP1307547B1, US7153818, EP1421169B1, US6979669, EP1529101B1, US7375070, EP1385943B1, US7888104, EP1414977B1, US5855625, EP1921147B1, EP1921148B1, EP701605B1, EP1633469B1, EP1633470B1, EP1794293B1, EP171007B1, US4692260, US7569226, EP1165737B1, US6391838, US6060441, US2009017074, US7320887, EP1737952B1, US7691618, US20070256251, US20050261156, US20050261158, US20100234267, US20110136720, US20110201536, US7811076, US5929017, US5156773, EP2343310A1, WO2011083114, EP214761B1, US4876024, EP675944B1, US5763383, EP517761B1, US6624129, EP1054956B1, US6939702, US6964944, EP832174B1, US20060205628, US20070179076, US20080023031, US20110015110, US20110028372, US4973417, US5447649, US5840677, US5965503, US5972873, US5998344, US6071356, WO9009428, EP1661978A1, EP1698689A1, EP1726636A1, EP1867707A1, EP1876226A1, EP1876227A1, EP0205208A2, EP0206390A2, EP0271152, EP0271154, EP0341999, EP0346136, EP2135934, US20120208734, WO2011127102, WO2012142087, WO2012145062, EP1790713B1, US8066818B2, US8163686B2, US8283300B2, US8354366B2, US20120125374, US3929678, and US5898025.

### 7.6. Methods of Assessing Amylase Activity in Detergent Compositions

Numerous α-amylase cleaning assays are known in the art, including swatch and micro-swatch assays. The appended Examples describe only a few such assays.

In order to further illustrate the compositions and methods, and advantages thereof, the following specific examples are given with the understanding that they are illustrative rather than limiting.

### 8. Brewing Compositions

The present α-amylase may be a component of a brewing composition used in a process of brewing, *i.e*., making a fermented malt beverage. Non-fermentable carbohydrates form the majority of the dissolved solids in the final beer. This residue remains because of the inability of malt amylases to hydrolyze the alpha-1,6-linkages of the starch. The non-fermentable carbohydrates contribute about 50 calories per 12 ounces of beer. An amylase, in combination with a glucoamylase and optionally a pullulanase and/or isoamylase, assist in converting the starch into dextrins and fermentable sugars, lowering the residual non-fermentable carbohydrates in the final beer.

The principal raw materials used in making these beverages are water, hops and malt. In addition, adjuncts such as common corn grits, refined corn grits, brewer's milled yeast, rice, sorghum, refined corn starch, barley, barley starch, dehusked barley, wheat, wheat starch, torrified cereal, cereal flakes, rye, oats, potato, tapioca, and syrups, such as corn syrup, sugar cane syrup, inverted sugar syrup, barley and/or wheat syrups, and the like may be used as a source of starch.

For a number of reasons, the malt, which is produced principally from selected varieties of barley, has the greatest effect on the overall character and quality of the beer. First, the malt is the primary flavoring agent in beer. Second, the malt provides the major portion of the fermentable sugar. Third, the malt provides the proteins, which will contribute to the body and foam character of the beer. Fourth, the malt provides the necessary enzymatic activity during mashing. Hops also contribute significantly to beer quality, including flavoring. In particular, hops (or hops constituents) add desirable bittering substances to the beer. In addition, the hops act as protein precipitants, establish preservative agents and aid in foam formation and stabilization.

Grains, such as barley, oats, wheat, as well as plant components, such as corn, hops, and rice, also are used for brewing, both in industry and for home brewing. The components used in brewing may be unmalted or may be malted, *i.e*., partially germinated, resulting in an increase in the levels of enzymes, including α-amylase. For successful brewing, adequate levels of α-amylase enzyme activity are necessary to ensure the appropriate levels of sugars for fermentation. An amylase, by itself or in combination with another α-amylase(s), accordingly may be added to the components used for brewing.

As used herein, the term "stock" means grains and plant components that are crushed or broken. For example, barley used in beer production is a grain that has been coarsely ground or crushed to yield a consistency appropriate for producing a mash for fermentation. As used herein, the term "stock" includes any of the aforementioned types of plants and grains in crushed or coarsely ground forms. The methods described herein may be used to determine α-amylase activity levels in both flours and stock.

Processes for making beer are well known in the art. *See, e.g*., Wolfgang Kunze (2004) "Technology Brewing and Malting," Research and Teaching Institute of Brewing, Berlin (VLB), 3rd edition. Briefly, the process involves: (a) preparing a mash, (b) filtering the mash to prepare a wort, and (c) fermenting the wort to obtain a fermented beverage, such as beer. Typically, milled or crushed malt is mixed with water and held for a period of time under controlled temperatures to permit the enzymes present in the malt to convert the starch present in the malt into fermentable sugars. The mash is then transferred to a mash filter where the liquid is separated from the grain residue. This sweet liquid is called "wort," and the left over grain residue is called "spent grain." The mash is typically subjected to an extraction, which involves adding water to the mash in order to recover the residual soluble extract from the spent grain. The wort is then boiled vigorously to sterilizes the wort and help develop the color, flavor and odor. Hops are added at some point during the boiling. The wort is cooled and transferred to a fermentor.

The wort is then contacted in a fermentor with yeast. The fermentor may be chilled to stop fermentation. The yeast flocculates and is removed. Finally, the beer is cooled and stored for a period of time, during which the beer clarifies and its flavor develops, and any material that might impair the appearance, flavor and shelf life of the beer settles out. The beer usually contains from about 2% to about 10% v/v alcohol, although beer with a higher alcohol content, *e.g.,* 18% v/v, may be obtained. Prior to packaging, the beer is carbonated and, optionally, filtered and pasteurized.

The brewing composition comprising an amylase, in combination with a glucoamylase and optionally a pullulanase and/or isoamylase, may be added to the mash of step (a) above, *i.e*., during the preparation of the mash. Alternatively, or in addition, the brewing composition may be added to the mash of step (b) above, *i.e*., during the filtration of the mash. Alternatively, or in addition, the brewing composition may be added to the wort of step (c) above, *i.e*., during the fermenting of the wort.

A fermented beverage, such as a beer, can be produced by one of the methods above. The fermented beverage can be a beer, such as full malted beer, beer brewed under the "Reinheitsgebot," ale, IPA, lager, bitter, Happoshu (second beer), third beer, dry beer, near beer, light beer, low alcohol beer, low calorie beer, porter, bock beer, stout, malt liquor, non-alcoholic beer, non-alcoholic malt liquor and the like, but also alternative cereal and malt beverages such as fruit flavored malt beverages, *e*.*g*., citrus flavored, such as lemon-, orange-, lime-, or berry-flavored malt beverages, liquor flavored malt beverages, *e*.*g*., vodka-, rum-, or tequila-flavored malt liquor, or coffee flavored malt beverages, such as caffeine-flavored malt liquor, and the like.

### 9. Reduction of Iodine-Positive Starch

α-amylases may reduce the iodine-positive starch (IPS), when used in a method of liquefaction and/or saccharification. One source of IPS is from amylose that escapes hydrolysis and/or from retrograded starch polymer. Starch retrogradation occurs spontaneously in a starch paste, or gel on ageing, because of the tendency of starch molecules to bind to one another followed by an increase in crystallinity. Solutions of low concentration become increasingly cloudy due to the progressive association of starch molecules into larger articles. Spontaneous precipitation takes place and the precipitated starch appears to be reverting to its original condition of cold-water insolubility. Pastes of higher concentration on cooling set to a gel, which on ageing becomes steadily firmer due to the increasing association of the starch molecules. This arises because of the strong tendency for hydrogen bond formation between hydroxy groups on adjacent starch molecules. *See* J.A. Radley, ed., STARCH AND ITS DERIVATIVES 194-201 (Chapman and Hall, London (1968)).

The presence of IPS in saccharide liquor negatively affects final product quality and represents a major issue with downstream processing. IPS plugs or slows filtration system, and fouls the carbon columns used for purification. When IPS reaches sufficiently high levels, it may leak through the carbon columns and decrease production efficiency. Additionally, it may result in hazy final product upon storage, which is unacceptable for final product quality. The amount of IPS can be reduced by isolating the saccharification tank and blending the contents back. IPS nevertheless will accumulate in carbon columns and filter systems, among other things. The use of α-amylases is expected to improve overall process performance by reducing the amount of IPS.

In order to further illustrate the compositions and methods, and advantages thereof, the following specific examples are given with the understanding that they are illustrative rather than limiting.

### EXAMPLES

### Example 1

### Cloning and expression of PcuAmy1

PcuAmy1 (NCBI accession No.: ZP_07385374.1) is an α-amylase from the bacterium strain *Paenibacillus curdlanolyticus.* An expression plasmid was made to evaluate its expression in *Bacillus subtilis.* An expression construct was synthesized by GeneRay Biotech Co., Ltd (Shanghai, China), which contains an aprE promoter, an aprE signal sequence used to direct target protein secretion in *B. subtilis,* an oligonucleotide named AGK-proAprE that encodes peptide Ala-Gly-Lys to facilitate the secretion of the target protein, and the optimized sequence of PcuAmy1 (SEQ ID NO: 1). The synthetic construct was digested with *Xho*I and *EcoR*I*,* and ligated into a p2JM-based vector digested with the same restriction enzymes to obtain the expression plasmid p2JM706. The ligation mixture was used to transform chemically competent *E. coli* TOP10 cells (Invitrogen Corp.) following the manufacturer's protocol. The transformed cells were plated on Luria Agar plates supplemented with 50 ppm ampicillin and incubated overnight at 37°C. Three transformants were picked from the plate and inoculated into 5 ml Luria Broth supplemented with 50 ppm ampicillin. Cultures were grown overnight at 37°C. The plasmids were extracted and the sequence of the *PcuAmyl* gene was confirmed by DNA sequencing.

The p2JM706 plasmid was then transformed into *B. subtilis* cells (*degU^{Hy}32,* Δ*scoC*) and the transformed cells were spread on Luria Agar plates supplemented with 5ppm Chloramphenicol and 1% starch. Colonies with the largest clear halos on the plates were selected and subjected to fermentation in a 250ml shake flask with Grant's II medium.

The codon-optimized nucleotide sequence of the *PcuAmy1* gene in plasmid p2JM706 is set forth as SEQ ID NO: 1:

GCCGACAACGGCACAATCATGCAGTATTTCGAGTGGTACCTGCCGAACGACGGAGCGCACTGGA
ACAGACTTAATAACGACGCACAAAACCTGAAAAATGTGGGCATCACGGCAGTGTGGATTCCTCC
GGCATACAAGGGCGGCAGCTCAGCAGATGTTGGCTACGGAGTTTACGATACATACGACCTGGGC
GAGTTCAATCAGAAAGGCACGGTCAGAACAAAGTACGGAACGAAGAGCGAACTGATTTCAGCGG
TCAACAATCTTCACGCAAAGGGCATTGCGGTTTACGGCGACGTGGTCCTGAACCATAGAATGAA
TGCGGATGCAACGGAGCTTGTGGATGCGGTTGAGGTGGATCCGAACAACAGAAACGTCGAGACG
ACAAGCACGTATCAGATCCAGGCATGGACGCAATACGATTTCCCGGGCAGAGGCAACACGTACA
GCAGCTTTAAATGGAGATGGTATCACTTCGACGGCGTCGACTGGGACCAGAGCAGAGGCCTGAA
CAGAATCTATAAGCTGAGAGGCGATGGCAAGGATTGGGACTGGGAGGTCGACAGCGAGTACGGC

AACTACGATTACCTGATGGGAGCGGACCTGGACTTCAACCACCCGGATGTGGTTAACGAAACAA
AGACATGGGGCAAATGGTTTGTGAACACGGTGAACCTGGATGGCGTCAGACTGGACGCGGTTAA
GCACATCAAGTTCGACTTCATGAGAGACTGGGTGAACAACGTGAGAAGCACGACGGGCAAGAAC
CTTTTCGCAGTTGGCGAGTATTGGCACTACGACGTGAACAAACTGAACAGCTACATCACGAAGA
CGAATGGCACGATGAGCCTGTTCGACGTGCCGCTGCACTTTAGATTTTATGATGCAAGCAACGG
CGGAGGCGGCTACGACATGAGAAACCTGCTGAATAACACGCTGATGAGCAGCAACCCGATGAAG
GCGGTTACATTCGTTGAGAACCATGACACACAACCGACGCAGGCCCTGCAATCAACGGTCCAAA
GCTGGTTTAAGCCGCTTGCGTATGCTACAATCCTGACGAGAGAGCAAGGCTACCCGTGCGTTTT
CTACGGCGACTATTATGGAACAAGCGACGGCAAAATTAGCAGCTACAAGCCGATCATGGATAAG
CTTCTTAACGCGAGAAAGGTGTACGCCTACGGCACGCAGAGAGATTACTTCGATCATCCGGACA
TCGTTGGCTGGACAAGAGAAGGCGATGCAGCACATGCTGGCTCAGGACTGGCAACGCTTATCAC
AGATGGCCCTGGCGGAAGCAAGTGGATGTATGTTGGAACGTCAAAGGCAGGCCAGGTCTGGACG
GATAAAACAGGAAACAGAAGCGGAACGGTGACGATTGATGCCAATGGCTGGGGAAACTTTTGGG
TTAATGGCGGATCAGTTAGCGTTTGGGCAAAATAA

The amino acid sequence of the PcuAmy1 precursor polypeptide is set forth as SEQ ID NO: 2. The signal peptide is shown in italics and bold.

MFSIDFMKSRKRLISYMVVFAFLAGLVFQPLGATKASAADNGTIMQYFEWYLPNDGAHWNRLNN
DAQNLKNVGITAVWIPPAYKGGSSADVGYGVYDTYDLGEFNQKGTVRTKYGTKSELISAVNNLH
AKGIAVYGDVVLNHRMNADATELVDAVEVDPNNRNVETTSTYQIQAWTQYDFPGRGNTYSSFKW
RWYHFDGVDWDQSRGLNRIYKLRGDGKDWDWEVDSEYGNYDYLMGADLDFNHPDVVNETKTWGK
WFVNTVNLDGVRLDAVKHIKFDFMRDWVNNVRSTTGKNLFAVGEYWHYDVNKLNSYITKTNGTM
SLFDVPLHFRFYDASNGGGGYDMRNLLNNTLMSSNPMKAVTFVENHDTQPTQALQSTVQSWFKP
LAYATILTREQGYPCVFYGDYYGTSDGKISSYKPIMDKLLNARKVYAYGTQRDYFDHPDIVGWT
REGDAAHAGSGLATLITDGPGGSKWMYVGTSKAGQVWTDKTGNRSGTVTIDANGWGNFWVNGGS
VSVWAK

The amino acid sequence of the mature form of PcuAmy1 is set forth as SEQ ID NO: 3:

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLRGDGKDWDWEVDSEYG
NYDYLMGADLDFNHPDVVNETKTWGKWFVNTVNLDGVRLDAVKHIKFDFMRDWVNNVRSTTGKN
LFAVGEYWHYDVNKLNSYITKTNGTMSLFDVPLHFRFYDASNGGGGYDMRNLLNNTLMSSNPMK
AVTFVENHDTQPTQALQSTVQSWFKPLAYATILTREQGYPCVFYGDYYGTSDGKISSYKPIMDK
LLNARKVYAYGTQRDYFDHPDIVGWTREGDAAHAGSGLATLITDGPGGSKWMYVGTSKAGQVWT
DKTGNRSGTVTIDANGWGNFWVNGGSVSVWAK

### Example 2

### Cloning and expression of PcuAmy1 variant PcuAmy1-v1

A PcuAmy1 variant (PcuAmy1-v1) having deletions of Arg-177 and Gly-178 (*i.e*., del (R177, G178), ΔR177-ΔG178, or ΔRG; Suzuki, Y. et al. (1989) J. Biol. Chem. 264:18933-38)) was constructed in an expression plasmid to evaluate its expression in *Bacillus subtilis.* Four primers were designed with engineered restriction sites and overlapping regions based on SEQ ID NO: 1. First, the former partial fragment before Arg-177 and Gly-178 and the latter partial sequence after Arg-177 and Gly-178 were obtained by PCR separately. Then, overlap extension PCR was performed by combining these two fragments to obtain a full length PcuAmy1-v1 gene.

The overlap extension PCR products were digested with *Bss*HII and *Xho*I and ligated into the p2JM vector which was digested with the same restriction enzymes to obtain the expression plasmid p2JM754. The ligation mixture was used to transform *E. coli* TOP10 chemically competent cells (Invitrogen Corp.) following the manufacturer's protocol. The transformed cells were plated on Luria Agar plates supplemented with 50 ppm ampicillin and incubated overnight at 37°C. Three transformants were picked from the plate and inoculated into 5 ml Luria Broth supplemented with 50 ppm ampicillin. Cultures were grown overnight at 37°C, plasmid DNA extracted and the correct sequence of the *PcuAmy1-v1* gene was confirmed by DNA sequencing.

The p2JM754 plasmid was then transformed into *B. subtilis* cells and the transformed cells were spread on Luria Agar plates supplemented with 5ppm Chloramphenicol and 1% starch. Colonies with the largest clear halos on the plates were selected and subjected to fermentation in a 250ml shake flask with Grant's II medium.

The nucleotide sequence of the *PcuAmy1-v1* gene of plasmid p2JM754 is set forth as SEQ ID NO: 4:

GCCGACAACGGCACAATCATGCAGTATTTCGAGTGGTACCTGCCGAACGACGGAGCGCACTGGA
ACAGACTTAATAACGACGCACAAAACCTGAAAAATGTGGGCATCACGGCAGTGTGGATTCCTCC
GGCATACAAGGGCGGCAGCTCAGCAGATGTTGGCTACGGAGTTTACGATACATACGACCTGGGC
GAGTTCAATCAGAAAGGCACGGTCAGAACAAAGTACGGAACGAAGAGCGAACTGATTTCAGCGG
TCAACAATCTTCACGCAAAGGGCATTGCGGTTTACGGCGACGTGGTCCTGAACCATAGAATGAA
TGCGGATGCAACGGAGCTTGTGGATGCGGTTGAGGTGGATCCGAACAACAGAAACGTCGAGACG
ACAAGCACGTATCAGATCCAGGCATGGACGCAATACGATTTCCCGGGCAGAGGCAACACGTACA
GCAGCTTTAAATGGAGATGGTATCACTTCGACGGCGTCGACTGGGACCAGAGCAGAGGCCTGAA
CAGAATCTATAAGCTGGATGGCAAGGATTGGGACTGGGAGGTCGACAGCGAGTACGGCAACTAC

GATTACCTGATGGGAGCGGACCTGGACTTCAACCACCCGGATGTGGTTAACGAAACAAAGACAT
GGGGCAAATGGTTTGTGAACACGGTGAACCTGGATGGCGTCAGACTGGACGCGGTTAAGCACAT
CAAGTTCGACTTCATGAGAGACTGGGTGAACAACGTGAGAAGCACGACGGGCAAGAACCTTTTC
GCAGTTGGCGAGTATTGGCACTACGACGTGAACAAACTGAACAGCTACATCACGAAGACGAATG
GCACGATGAGCCTGTTCGACGTGCCGCTGCACTTTAGATTTTATGATGCAAGCAACGGCGGAGG
CGGCTACGACATGAGAAACCTGCTGAATAACACGCTGATGAGCAGCAACCCGATGAAGGCGGTT
ACATTCGTTGAGAACCATGACACACAACCGACGCAGGCCCTGCAATCAACGGTCCAAAGCTGGT
TTAAGCCGCTTGCGTATGCTACAATCCTGACGAGAGAGCAAGGCTACCCGTGCGTTTTCTACGG
CGACTATTATGGAACAAGCGACGGCAAAATTAGCAGCTACAAGCCGATCATGGATAAGCTTCTT
AACGCGAGAAAGGTGTACGCCTACGGCACGCAGAGAGATTACTTCGATCATCCGGACATCGTTG
GCTGGACAAGAGAAGGCGATGCAGCACATGCTGGCTCAGGACTGGCAACGCTTATCACAGATGG
CCCTGGCGGAAGCAAGTGGATGTATGTTGGAACGTCAAAGGCAGGCCAGGTCTGGACGGATAAA
ACAGGAAACAGAAGCGGAACGGTGACGATTGATGCCAATGGCTGGGGAAACTTTTGGGTTAATG
GCGGATCAGTTAGCGTTTGGGCAAAATAA

The amino acid sequence of the mature form of PcuAmy1-v1 is set forth as SEQ ID NO: 5:

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWEVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPTQALQSTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNGGSVSVWAK

### Example 3

### Proteolytic cleavage of PcuAmy1 and variants

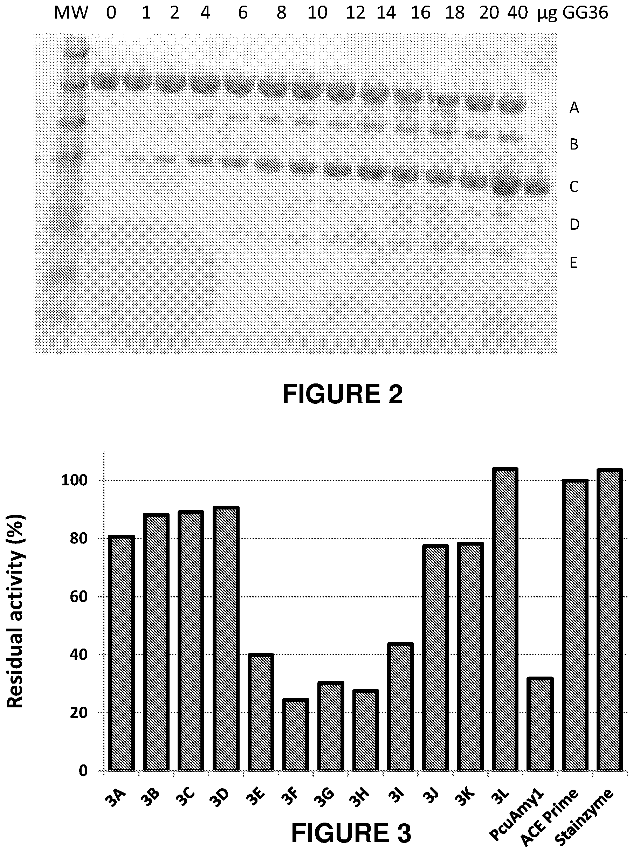

Incubation of wild-type PcuAmy1 amylase, or the PcuAmy-vl variant, with subtilisin proteases leads to cleavage of the proteins, as observed when the reaction products are subjected to SDS/PAGE electrophoresis. Figure 2 is an image of an SDS/PAGE gel showing the cleavage of 20 µg of PcuAmy1-v1 in the presence of increasing amounts of GG36 protease, (from 0 to 40 µg as indicated above the gel). The letters on the right side of the gel indicate (A) intact full-length PcuAmy1-v1, (B) a first cleavage product of PcuAmy1-v1, (C) GG36 protease, (D) a contaminant in the GG36 protein preparation, and (E) a second cleavage product of PcuAmy1-v1. The main degradation products observed after incubation of PcuAmy-vl amylase with a subtilisin protease have a molecular weight of about 38 and 16 kDa (B and E, respectively). The amount of proteolytic degradation is dependent on the concentration of protease used. This makes PcuAmy1 amylase suboptimal for inclusion in enzyme detergent formulations that contain commonly-used subtilisin proteases.

A sample of PcuAmy-vl protein was incubated with GG36 protease (*Bacillus lentis* subtilisin) and the reaction products were analyzed by mass spectroscopy. The results were consistent with hydrolysis occurring between residues Q334 and L336 (not shown).

### Example 4

### PcuAmy1 variants resistant to proteolytic cleavage

In this example, the construction of *Bacillus subtilis* strains expressing PcuAmy1-v3 and further variants 3A to 3L are described. PcuAmy1-v3 is a variant of PcuAmy1 with the mutations E186P, G472K and lacking R177 and G178. The substitution E186P and the deletions at R177 and G178 increase the detergent stability of PcuAmy1. The substitution G472K improves cleaning performance. None of these mutations has any effect on protease sensitivity (data not shown). Therefore, including these mutations in variants made to explore the effect of other mutations on protease stability does not interfere with the results.

For expression of PcuAmy1-v3, a PCR with the primers PSV_FW (SEQ ID NO: 6) and PSV3_RV (SEQ ID NO: 7) was performed on pHP024T02-PcuAmy1-v1 plasmid DNA (described, above).

Primer PSV_FW (SEQ ID NO: 6):

ACCCCCCTCGAGGCTTTTCTTTTGGAAGAAAATATAGGGAAAATGGTACTTGTTAAAAATTCGG
AATATTTATACAATATCATATGACAGAATAGTCTTTTAAGTAAGTCTACTCTGAATTTTTTTAA
AAGGAGAGGGTAAAGAATGAAACAACAAAAACGGCTTTACGCCCGATTGCTGACGCTGTTATTT
GCGCTCATCTTCTTGCTGCCTCATTCTGCAGCTTCAGCAGCCGACAACGGCACAATCATGC

Primer PSV3_RV (SEQ ID NO: 7):

Primer PSV3_RV (SEQ ID NO: 7):
CTCGATGCGGCCGCAGCTGTTTTATCCTTTACCTTGTCTCCAAGCTTAAAATAAAAAAACGGAT
TTCCTTCAGGAAATCCGTCCTCTGTTAACTTATTTTGCCCAAACGCTAACTGATCCTTTATTAA
CCCAAAAGTTTCCCCAGCC

PCR was performed with Platinum Taq DNA Polymerase High Fidelity according to the protocol provided by the supplier (Invitrogen, Life Technologies). The PCR fragment was purified, digested with *XhoI* - *Not*I, ligated into vector pICatH and transformed into *B. subtilis* cells as described in US7968691. *B. subtilis* transformants were selected on agar plates containing heart infusion agar (Difco) and 10 mg/L neomycin sulfate and 5 mg/L chloramphenicol. One transformant with starch hydrolyzing activity was selected and the amylase gene was sequence verified by DNA sequencing (BaseClear, The Netherlands). This *B. subtilis* clone contains plasmid pICatH-PcuAmy1-v3 and expresses PcuAmy1-v3 amylase. The DNA sequence of the PcuAmy1-v3 gene (encoding the mature form of PcuAmy1-v3) is shown in SEQ ID NO: 8. The protein sequence of PcuAmy1-v3 is depicted in SEQ ID NO: 9.

Nucleic acid sequence encoding PcuAmy1-v3 (SEQ ID NO: 8):

GCCGACAACGGCACAATCATGCAGTATTTCGAGTGGTACCTGCCGAACGACGGAGCGCACTGGA
ACAGACTTAATAACGACGCACAAAACCTGAAAAATGTGGGCATCACGGCAGTGTGGATTCCTCC
GGCATACAAGGGCGGCAGCTCAGCAGATGTTGGCTACGGAGTTTACGATACATACGACCTGGGC
GAGTTCAATCAGAAAGGCACGGTCAGAACAAAGTACGGAACGAAGAGCGAACTGATTTCAGCGG
TCAACAATCTTCACGCAAAGGGCATTGCGGTTTACGGCGACGTGGTCCTGAACCATAGAATGAA
TGCGGATGCAACGGAGCTTGTGGATGCGGTTGAGGTGGATCCGAACAACAGAAACGTCGAGACG
ACAAGCACGTATCAGATCCAGGCATGGACGCAATACGATTTCCCGGGCAGAGGCAACACGTACA
GCAGCTTTAAATGGAGATGGTATCACTTCGACGGCGTCGACTGGGACCAGAGCAGAGGCCTGAA
CAGAATCTATAAGCTGGATGGCAAGGATTGGGACTGGCCGGTCGACAGCGAGTACGGCAACTAC
GATTACCTGATGGGAGCGGACCTGGACTTCAACCACCCGGATGTGGTTAACGAAACAAAGACAT
GGGGCAAATGGTTTGTGAACACGGTGAACCTGGATGGCGTCAGACTGGACGCGGTTAAGCACAT
CAAGTTCGACTTCATGAGAGACTGGGTGAACAACGTGAGAAGCACGACGGGCAAGAACCTTTTC
GCAGTTGGCGAGTATTGGCACTACGACGTGAACAAACTGAACAGCTACATCACGAAGACGAATG
GCACGATGAGCCTGTTCGACGTGCCGCTGCACTTTAGATTTTATGATGCAAGCAACGGCGGAGG
CGGCTACGACATGAGAAACCTGCTGAATAACACGCTGATGAGCAGCAACCCGATGAAGGCGGTT
ACATTCGTTGAGAACCATGACACACAACCGACGCAGGCCCTGCAATCAACGGTCCAAAGCTGGT
TTAAGCCGCTTGCGTATGCTACAATCCTGACGAGAGAGCAAGGCTACCCGTGCGTTTTCTACGG
CGACTATTATGGAACAAGCGACGGCAAAATTAGCAGCTACAAGCCGATCATGGATAAACTGCTT
AACGCGAGAAAGGTGTACGCCTACGGCACGCAGAGAGATTACTTCGATCATCCGGACATCGTTG
GCTGGACAAGAGAAGGCGATGCAGCACATGCTGGCTCAGGACTGGCAACGCTTATCACAGATGG
CCCTGGCGGAAGCAAGTGGATGTATGTTGGAACGTCAAAGGCAGGCCAGGTCTGGACGGATAAA
ACAGGAAACAGAAGCGGAACGGTGACGATTGATGCCAATGGCTGGGGAAACTTTTGGGTTAATA
AAGGATCAGTTAGCGTTTGGGCAAAATAA

Mature form of PcuAmy1-v3 (SEQ ID NO:9):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF

AVGEYWHYDVNKLNSYITKTNGTMSLFDVPLHFRFYDASNGGGYDMRNLLNNTLMSSNPMKAV
TFVENHDTQPTQALQSTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

To identify the reason for PcuAmy1 protease sensitivity, the amino acid sequence of PcuAmy1 was compared to that of other CAZy Family GH-13 amylases which show protease-resistance, such as PURASTAR® ST (*B. licheniformis* amylase or AmyL), SPEZYME® XTRA (*Geobacillus stearothermophilus* amylase or AmyS), ACE-QK (WO2010/115021), and STAINZYME® (Novozymes). Based on observed difference in sequence, PcuAmy1 variants PcuAmy1-v3A to PcuAmy1-v3L (*i*.*e*., 3A-3L) were designed and tested for protease resistance. The mutations present in each variant are listed in Table 2. They were introduced into PcuAmy1-v3 (SEQ ID NO: 9), described above.

**Table 2. List of mutations introduced in PcuAmy1-v3 resulting in variants 3A to 3L**

| **Position** | **wt** | **3A** | **3B** | **3C** | **3D** | **3E** | **3F** | **3G** | **3H** | **3I** | **3J** | **3K** | **3L** |
|---|---|---|---|---|---|---|---|---|---|---|---|---|---|
| **319** | M | | | | | | | | | T | | | |
| **333** | T | G | G | G | G | | | | | | | G | G |
| **335** | A | | S | S | S | | S | | | | | S | S |
| **337** | Q | | E | E | E | | | E | | | | | E |
| **339** | T | | | | | | | | | | | W | |
| **341** | Q | | | | | | | | | | | E | |
| **342** | S | | | P | P | | | | P | | | P | T |
| **351** | T | | | F | | F | | | | | W | | |

To generate PcuAmy1-v3 variants 3A to 3L (*i*.*e*., PcuAmy1-v3A - PcuAmy1-v3L), fusion PCR was performed using plasmid pICatH-PcuAmy1-v3 with primers shown in Table 3. PCR were performed with Taq DNA Polymerase High Fidelity as described above. In a first PCR, primer PSTI_FW and one of primers 3A_RV to 3L-RV were used (12 separate reactions respectively for 3A to 3L). In a second PCR, primer ECORV_RV and one of primers 3A_FW to 3L_FW were used (also 12 separate reactions for 3A to 3L). The PCR fragments obtained by the first and second PCR were fused in a third PCR (12 reactions for 3A to 3L) using the primers PSTI_FW and ECORV_RV. The 12 PCR fusion products were purified, digested with *Pst*I and *EcoRV* and ligated individually into vector pHPLT (Solingen et al. (2001) Extremophiles 5:333-341, US Patent Application 20100021587) and transformed into *Bacillus subtilis.* Transformants were selected on agar plates containing heart infusion agar and 10 mg/L neomycin sulphate and starch azure (Sigma). For each variant, one halo-positive clone was selected and the sequence of the amylase gene verified. Selective growth of *B. subtilis* transformants harboring the pHPLT-amylase expression plasmids was performed in shake flasks containing MBD medium (a MOPS based defined medium), 5 mM CaCl₂ and 10 mg/L neomycin. MBD medium was made essentially as known in the art (See, Neidhardt et al. (1974) J. Bacteriol., 119:736-747), except that NH₄Cl₂, FeSO₄, and CaCl₂ were omitted from the base medium, 3 mM K₂HPO₄ was used, and the base medium was supplemented with 60 mM urea, 75 g/L glucose, and 1% soytone. The micronutrients were made up as a 100X stock solution containing in one liter, 400 mg FeSO₄ 7H₂O, 100 mg MnSO₄ .H₂O, 100 mg ZnSO₄ 7H₂O, 50 mg CuCl₂ 2H₂O, 100 mg CoCl₂ 6H₂O, 100 mg NaMoO₄ 2H₂O, 100 mg Na₂B₄O₇ 10H₂O, 10 ml of 1M CaCl₂, and 10 ml of 0.5 M sodium citrate. Growth resulted in the production of secreted amylase with starch hydrolyzing activity for all 12 variants (3A to 3L).

**Table 3. DNA primers used to construct PcuAmy1 variants 3A to 3L**

| **Primer** | **Sequence (5'-3')** | **SEQ ID NO** |
|---|---|---|
| PSTI_FW | GCTGCCTCATTCTGCAGCTTCAGCAGCCGACAACGGCAC | 10 |
| ECORV_RV | CGTCCTCTGATATCTTATTTTGCCCAAACGCTAACTG | 11 |
| 3A_FW | GAGAACCATGACACACAACCGGGCCAGGCCCTGCAATCAACG GTCCAAAGC | 12 |
| 3A_RV | GCTTTGGACCGTTGATTGCAGGGCCTGGCCCGGTTGTGTGTC ATGGTTCTC | 13 |
| 3B_FW | GAGAACCATGACACACAACCGGGGCAGAGTCTGGAAAGCACG GTCCAAAGCTGG | 14 |
| 3B_RV | CCAGCTTTGGACCGTGCTTTCCAGACTCTGCCCCGGTTGTGT GTCATGGTTCTC | 15 |
| 3C_FW | GAGAACCATGACACACAACCGGGGCAGAGTCTGGAAAGCACG GTCCAACCCTGGTTTAAGCCGCTTGCGTATGCTTTCATCCTG ACGAGAGAGCAAGG | 16 |
| 3C_RV | CCTTGCTCTCTCGTCAGGATGAAAGCATACGCAAGCGGCTTA AACCAGGGTTGGACCGTGCTTTCCAGACTCTGCCCCGGTTGT GTGTCATGGTTCTC | 17 |
| 3D_FW | GAGAACCATGACACACAACCGGGGCAGAGTCTGGAAAGCACG GTCCAACCCTGGTTTAAGCCGCTTGCGTATGC | 18 |
| 3D_RV | GCATACGCAAGCGGCTTAAACCAGGTTGGACCGTGCTTTCC AGACTCTGCCCCGGTTGTGTGTCATGGTTCTC | 19 |
| 3E_FW | GGTTTAAGCCGCTTGCGTATGCTTTCATCCTGACGAGAGAGC AAGGC | 20 |
| 3E_RV | GCCTTGCTCTCTCGTCAGGATGAAAGCATACGCAAGCGGCTT AAACC | 21 |
| 3F_FW | CCATGACACACAACCGACGCAGTCCCTGCAATCAACGGTCCA AAGC | 22 |
| 3F_RV | GCTTTGGACCGTTGATTGCAGGGACTGCGTCGGTTGTGTGTC ATGG | 23 |
| 3G_FW | CACAACCGACGCAGGCCCTGGAATCAACGGTCCAAAGCTGGT TTAAGCCGC | 24 |
| 3G_RV | GCGGCTTAAACCAGCTTTGGACCGTTGATTCCAGGGCCTGCG TCGGTTGTG | 25 |
| 3H_FW | GGCCCTGCAATCAACGGTCCAACCCTGGTTTAAGCCGCTTGC GTATGCT | 26 |
| 3H_RV | AGCATACGCAAGCGGCTTAAACCAGGGTTGGACCGTTGATTG CAGGGCC | 27 |
| 3I_FW | CACGCTGATGAGCAGCAACCCGACGAAGGCGGTTACATTCGT TGAG | 28 |
| 3I_RV | CTCAACGAATGTAACCGCCTTCGTCGGGTTGCTGCTCATCAG CGTG | 29 |
| 3J_FW | GGTTTAAGCCGCTTGCGTATGCTTGGATCCTGACGAGAGAGC AAGGC | 30 |
| 3J_RV | GCCTTGCTCTCTCGTCAGGATCCAAGCATACGCAAGCGGCTT AAACC | 31 |
| 3K_FW | GAGAACCATGACACACAACCGGGCCAAAGCCTTCAAAGCTGG GTCGAACCGTGGTTTAAGCCGCTTGCGTATGC | 32 |
| 3K_RV | GCATACGCAAGCGGCTTAAACCACGGTTCGACCCAGCTTTGA AGGCTTTGGCCCGGTTGTGTGTCATGGTTCTC | 33 |
| 3L_FW | GAGAACCATGACACACAACCGGGGCAATCGCTTGAGTCGACT GTCCAAACATGGTTTAAGCCGCTTGCGTATGC | 34 |
| 3L_RV | GCATACGCAAGCGGCTTAAACCATGTTTGGACAGTCGACTCA AGCGATTGCCCCGGTTGTGTGTCATGGTTCTC | 35 |

The amino acid sequences of PcuAmy1-v3 variants 3A to 3L are shown below as SEQ ID NOs: 36-47.

Amino acid sequence of mature PcuAmy1-v3A (SEQ ID NO: 36):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQALQSTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3B (SEQ ID NO:37):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQSLESTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequenceof mature PcuAmy1-v3C (SEQ ID NO:38)

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQSLESTVQPWFKPLAYAFILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3D (SEQ ID NO:39)

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQSLESTVQPWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3E (SEQ ID NO:40):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPTQALQSTVQSWFKPLAYAFILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3F (SEQ ID NO:41):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPTQSLQSTVQSWFKPLAYATILTREQGYPCVFYGDYYGTSDGKISSYKPIMDKLL

NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3G (SEQ ID NO:42):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPTQALESTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3H (SEQ ID NO:43):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPTQALQSTVQPWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3I (SEQ ID NO:44):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPTKAV
TFVENHDTQPTQALQSTVQSWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3J (SEQ ID NO:45):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV

TFVENHDTQPTQALQSTVQSWFKPLAYAWILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3K (SEQ ID NO:46):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQSLQSWVEPWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

Amino acid sequence of mature PcuAmy1-v3L (SEQ ID NO:47):

ADNGTIMQYFEWYLPNDGAHWNRLNNDAQNLKNVGITAVWIPPAYKGGSSADVGYGVYDTYDLG
EFNQKGTVRTKYGTKSELISAVNNLHAKGIAVYGDVVLNHRMNADATELVDAVEVDPNNRNVET
TSTYQIQAWTQYDFPGRGNTYSSFKWRWYHFDGVDWDQSRGLNRIYKLDGKDWDWPVDSEYGNY
DYLMGADLDFNHPDVVNETKTWGKWFVNTVNLDGVRLDAVKHIKFDFMRDWVNNVRSTTGKNLF
AVGEYWHYDVNKLNSYITKTNGTMSLFDVPLHFRFYDASNGGGGYDMRNLLNNTLMSSNPMKAV
TFVENHDTQPGQSLESTVQTWFKPLAYATILTREQGYPCVFYGDYYGTSDGKISSYKPIMDKLL
NARKVYAYGTQRDYFDHPDIVGWTREGDAAHAGSGLATLITDGPGGSKWMYVGTSKAGQVWTDK
TGNRSGTVTIDANGWGNFWVNKGSVSVWAK

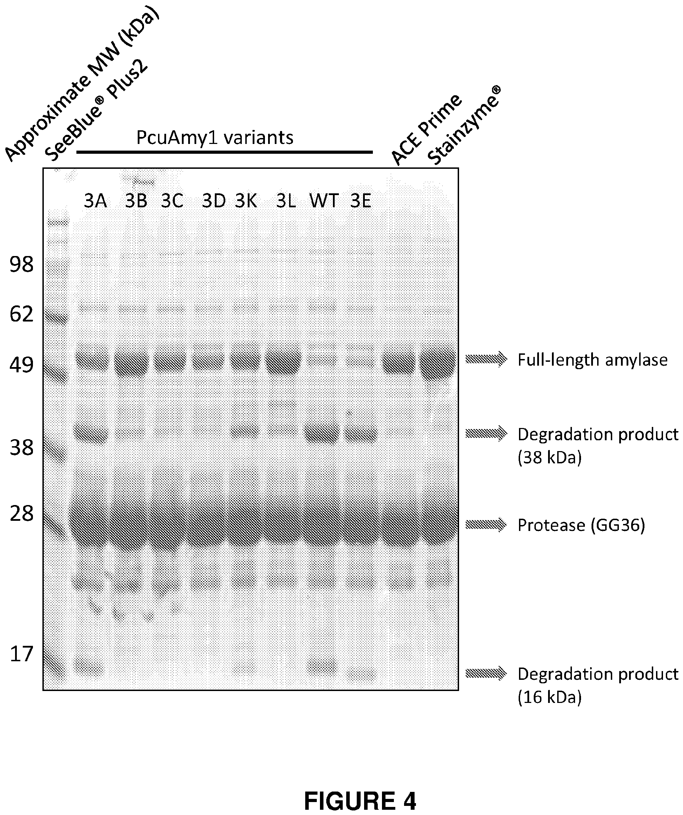

The 12 PcuAmy1 variants were expressed in *B. subtilis* as described, above. 40 µL filtered supernatant from each PcuAmy1 variant culture broth was incubated with 100 µg GG36 protease for 6 hours at room temperature, and subsequently analyzed for remaining amylase activity using the Megazyme Ceralpha substrate assay (Megazyme International Ireland, Co. Wicklow, Ireland). Residual activity after protease incubation was compared to the amylase activity of each sample after incubation with buffer alone. The results are shown in Figure 3. A subset of the samples was also analyzed by SDS-PAGE (Figure 4), with the protein standard, SeeBlue® Plus2 (Invitrogen). The commercially available amylases were included for comparison.

PcuAmy1 variants 3A, 3B, 3C, 3D, and 3L maintained >80% of their enzymatic activity after incubation with GG36 protease. PcuAmy1 variants 3J and 3K maintained >75% of their enzymatic activity after incubation with GG36 protease. PcuAmy1 variants 3E, 3F, 3G, 3H, and 3I did not show an appreciable increase of stability compared to the wild-type enzyme. Samples of the 3B, 3C, 3D and 3L incubations were analyzed by SDS/PAGE and showed significant reduction in degradation products when incubated with GG36 protease, confirming that the increase in residual amylase activity was due to decreased proteolytic cleavage.

These results of the small-scale experiment indicate that the introduction of a mutation at position T333 significantly reduces the proteolytic cleavage of PcuAmyl, and that additional mutations at A335, Q337, and S342 further reduce proteolytic cleavage. The T351W mutation but not the T351F mutation, also appears to reduce the proteolytic cleavage of PcuAmy1.

### Example 5

### Further categorization of the effect of individual and combined mutations

The results of the small-scale experiment described in Example 4 suggest that a mutation at position T333 significantly reduces the proteolytic cleavage of PcuAmyl, and that further benefit is obtained from mutations at A335, Q337, and S342. The T351W mutation also reduces the proteolytic cleavage of PcuAmy1. To better characterize the relative contributions of these mutations to protease resistance, the following additional variants were made, in a manner similar to that described in Example 4:
PcuAmy1-v10: Δ R177-ΔG178-E186P-T333G-Q337E-G472K
PcuAmy1-v11: Δ R177-ΔG178-E186P-T333G-A335S-G472K
PcuAmy1-v12: Δ R177-ΔG178-E186P-A335S-Q337E-G472K
PcuAmy1-v13: Δ R177-ΔG178-E186P-T333G-A335S-Q337E-T351W-G472K

Variants PcuAmy1-v10, PcuAmy1-v11, and PcuAmy1-v12 include pair-wise combination of mutations at positions T333, A335, and Q337. PcuAmy1-v3-v13 includes mutations at all the aforementioned positions and includes the additional mutation T351W. These variants were compared in a large scale detergent stability assay to the following variants from Example 4:
PcuAmy1-v3B: ΔR177-ΔG178 -E186P-T333G-A335S-Q337E-G472K
PcuAmy1-v3L: ΔR177-ΔG178 -E186P-T333G-A335S-Q337E-S342T-G472K

The commercial detergents Total Color (MIFA Ag Frenkendorf, Switzerland) and Omo (Unilever, London, UK) were heat inactivated at 95°C for 3 hours to eliminate existing enzyme activities. Enzyme activity in the heat inactivated detergents was measured using the Suc-AAPF-pNA and Ceralpha assays for measuring protease and amylase activity, respectively. To prepare the stability samples, 2% w/w protease (PURAFECT® Prime 4000L, Danisco US Inc.) and 0.5% w/w amylase were added to each detergent sample and mixed. Samples were stored in a CO₂ incubator (Sanyo) at 37°C for 14 days. Aliquots were taken from each reaction sample at various time points, diluted in 50 mM MOPS, pH 7.15 buffer with 1% BSA added, and alpha-amylase activity was measured using the Ceralpha substrate (Megazyme, Inc). The activity for each sample was determined using a Arena 20XT Photometric Analyzer (Thermo Scientific) using a calibrated standard. The remaining activity at each time point was reported as a percent (%) of the total activity determined at time zero.

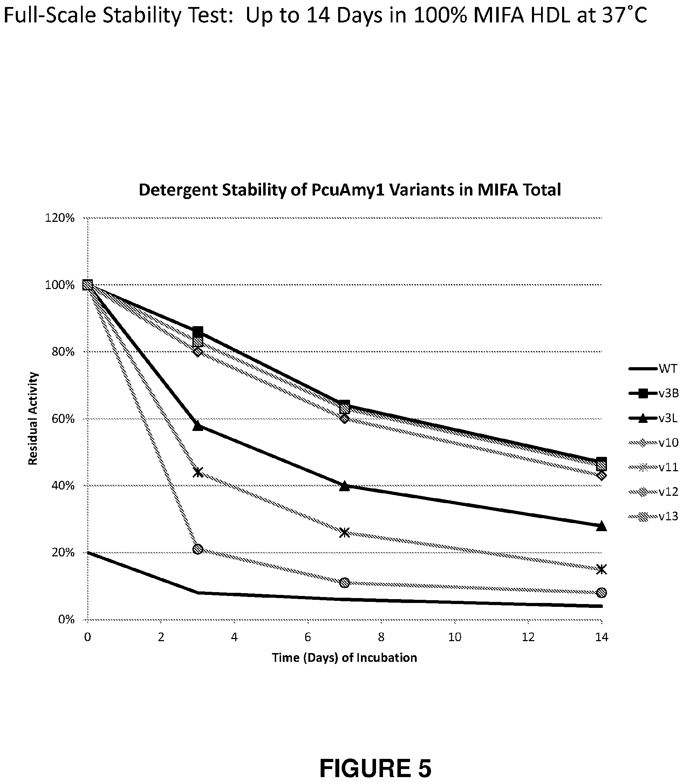

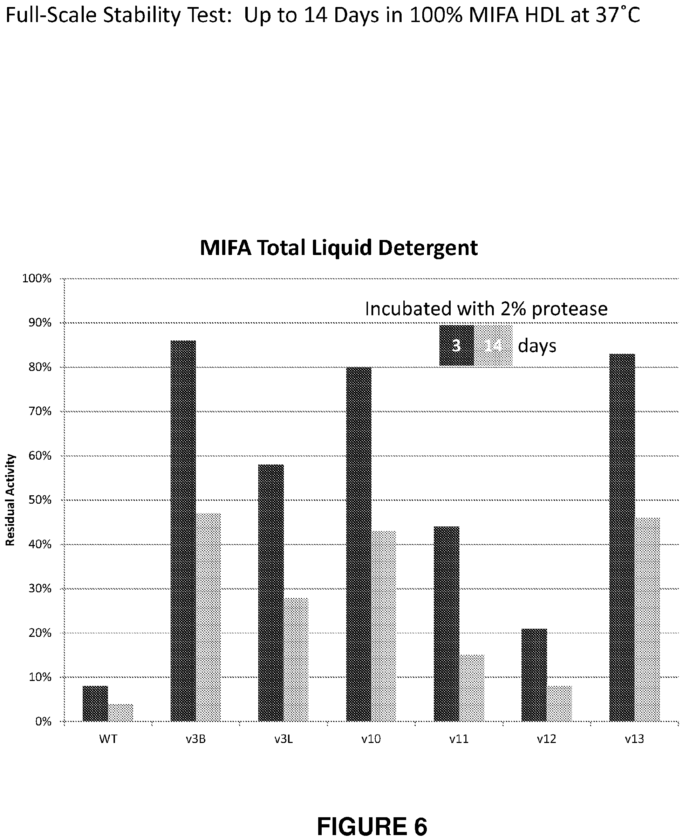

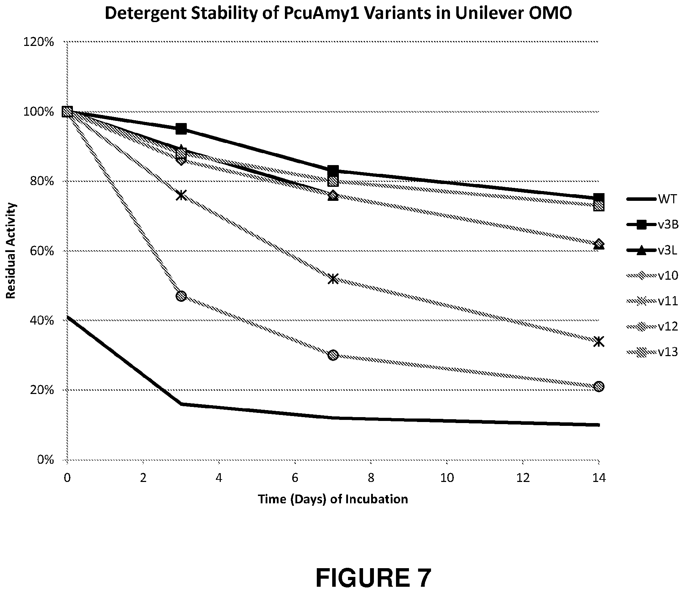

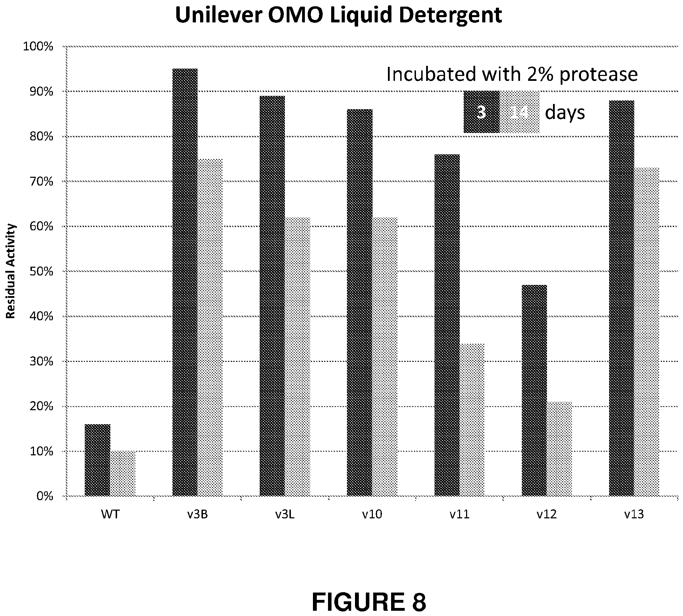

The results of the detergent stability assays performed in MIFA Total (Mifa Ag Frenkendorf, Switzerland) and Unilever OMO (Unilever, London, UK) are shown in Figures 5-8. Figures 5 and 7 show the residual activity of PcuAmy1 variants over time in MIFA Total and Unilever Omo, respectively, supplemented with FNA protease. Figures 6 and 8 summarize the data for the 3 and 14 day time points. PcuAmy1 variants that include the T333 mutation, *i*.*e*., 3B, 3L,v10, v13, and to a lesser degree, v11 were the most stable. Variants that did not include the T333 mutation, *i.e.,* V1 and v12, were the least stable. As evidenced by v10 and v11, the presence of a mutation at Q337E further improves stability.

### Example 6

### Cleaning performance of PcuAmy1 variants

The cleaning performance of purified PcuAmy1-v3B and PcuAmy1-v3L was analyzed in a microswatch cleaning assay. CFT CS-28 rice starch on cotton swatches (Center for Testmaterials, BV, Vlaardingen, Netherlands) containing an indicator dye bound to the starch were punched to form discs measuring 5.5 mm in diameter. Two discs were placed in each well of three flat-bottom non-binding 96-well assay plates.

Both enzymes and two commercial amylase products: PURASTAR® ST (alpha-amylase from *Bacillus* licheniformis; DuPont Industrial Biosciences, Palo Alto, California, USA), and STAINZYME® (Novozymes, Copenhagen, Denmark) were diluted to 0.5 mg/mL in dilution buffer (50 mM MOPS, pH 7.2, 0.005% Tween), and then further diluted to 2 ppm in a microtiter plate. 200 µL of these samples were transferred into the first row of each of three swatch plates. 100 µL of HEPES buffer (25 mM HEPES, pH 8.0 with 2 mM CaCl₂ and 0.005% Tween-80) was then added to each well of the next five rows of the swatch plates, and serial dilutions were made to result in final enzyme concentrations of 2, 1, 0.5, 0.25, and 0.125 ppm as well as a row of blank (buffer only) wells with 200 µL in every well. Plates were incubated at 25 °C with agitation at 1150 rpm for 15 minutes. The wash liquor was transferred to fresh microtiter plates and enzyme performance was judged by the amount of color released into the wash liquor. Color release was quantified spectrophotometrically at 488 nm, and triplicate reads were blank-subtracted and averaged.

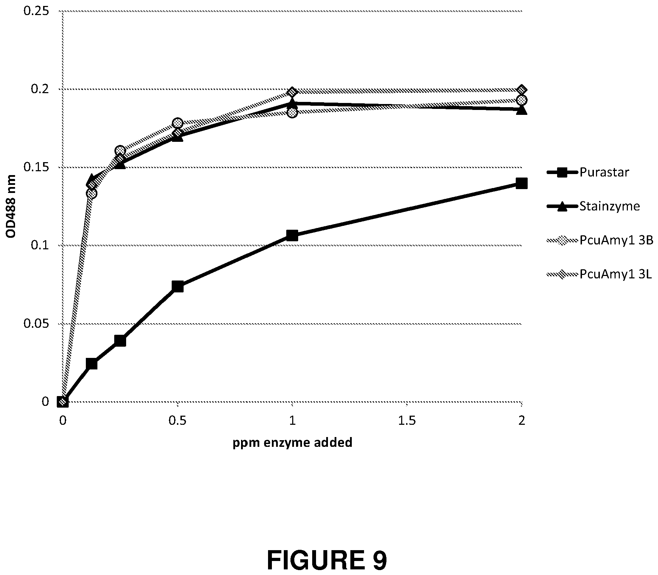

The results are shown in Figure 9. Both PcuAmy1-v3B and PcuAmy1-v3L demonstrated excellent cleaning performance.

### Example 7

### Thermal Stability of PcuAmy1 variants in liquid detergent

Commercial detergent Persil Universal Gel Gold (Henkel, Düsseldorf, Germany) was heat inactivated at 95°C for 3 hours to eliminate existing enzyme activities. Following inactivation, enzyme activity in the heat inactivated detergents was measured using the Suc-AAPF-pNA substrate-based and Ceralpha assay (Megazyme, Wicklow, Ireland) to ensure that any protease and amylase activities, respectively, had been abolished. A 10% solution of detergent was then made in water.

100 µL of each of the enzyme stocks (at 0.5 mg/mL) were added to 400 µL of the 10% detergent solutions. The enzymes tested were Purastar, Stainzyme®, PcuAmy1-v3B and PcuAmy-v3L. 50 µL of enzyme stock solution was added to PCR tubes and incubated at either 60, 70, 80, or 90°C for 15 minutes. Prior to incubation, 10 µL was removed and incubated at room temperature throughout the duration of the experiment to serve as the "unstressed" samples. Following incubation, an additional 1:10 dilution of each sample was made in dilution buffer. Samples were then transferred to microtiter plates in triplicate, and alpha-amylase activity was measured on all unstressed and stressed samples using the Ceralpha assay. Residual activity was calculated by dividing the activity of each amylase after the thermal stress by the activity of the unstressed amylase.

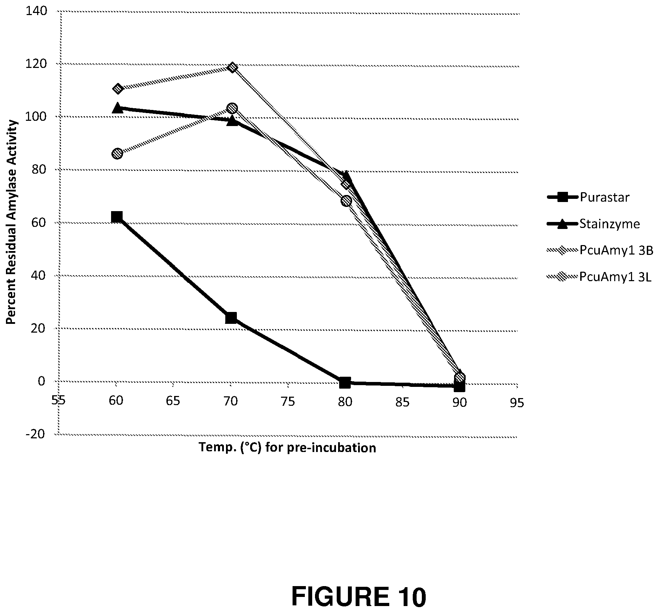

The results are shown in Figure 10. PcuAmy1-v3B and PcuAmy-v3L demonstrated similar thermostability compared to STAINZYME®, and significantly better stability than PURASTAR®.

### Example 8

### Combinatorial PcuAmy1 variants with improved performance

Having produced PcuAmy1 variants resistant to proteolytic cleavage, the effect of performance-enhancing mutations was explored. Combinations of mutations were made at positions N125, F152, E186, N205, G472, and G473, in combination with the deletion at positions R177 and G178 (using SEQ ID NO: 3 for numbering). The tested variants are shown in Table 4.

**Table 4. Combinatorial variants of PcuAmy1 amylase**

| **Variant** | **Mutations** |
|---|---|
| PcuAmy1-v1A | del (R177, G178) + N125Y + E186P + T333G + A335S + Q337E + G472K |
| PcuAmyl-v6 | del (R177, G178) + N125Y + F152W + E186P + T333G + A335S + Q337E + G472K |
| PcuAmyl-v8 | del (R177, G178) + N125Y + F152W + E186P + T333G + A335S + Q337E + G472R + G473R |
| PcuAmyl-v16 | del (R177, G178) + N125Y + F152W + E186P + N205D + T333G + A335S + Q337E + G472K |

The amino acid sequence of the mature PcuAmy1-v1A polypeptide is shown below as SEQ ID NO: 60:

ADNGTIMQYFEWYLPNDGAHWNRLNNDAQNLKNVGITAVWIPPAYKGGSSADVGYGVYD
TYDLGEFNQKGTVRTKYGTKSELISAVNNLHAKGIAVYGDVVLNHRMNADATELVDAVE
VDPNNRYVETTSTYQIQAWTQYDFPGRGNTYSSFKWRWYHFDGVDWDQSRGLNRIYKLD
GKDWDWPVDSEYGNYDYLMGADLDFNHPDVVNETKTWGKWFVNTVNLDGVRLDAVKHIK
FDFMRDWVNNVRSTTGKNLFAVGEYWHYDVNKLNSYITKTNGTMSLFDVPLHFRFYDAS
NGGGGYDMRNLLNNTLMSSNPMKAVTFVENHDTQPGQSLESTVQSWFKPLAYATILTRE
QGYPCVFYGDYYGTSDGKISSYKPIMDKLLNARKVYAYGTQRDYFDHPDIVGWTREGDA
AHAGSGLATLITDGPGGSKWMYVGTSKAGQVWTDKTGNRSGTVTIDANGWGNFWVNKGS
VSVWAK

The amino acid sequence of the mature PcuAmy1-v6 polypeptide is shown below as SEQ ID NO: 61:

ADNGTIMQYFEWYLPNDGAHWNRLNNDAQNLKNVGITAVWIPPAYKGGSSADVGYGVYD
TYDLGEFNQKGTVRTKYGTKSELISAVNNLHAKGIAVYGDVVLNHRMNADATELVDAVE
VDPNNRYVETTSTYQIQAWTQYDFPGRGNTYSSWKWRWYHFDGVDWDQSRGLNRIYKLD
GKDWDWPVDSEYGNYDYLMGADLDFNHPDVVNETKTWGKWFVNTVNLDGVRLDAVKHIK
FDFMRDWVNNVRSTTGKNLFAVGEYWHYDVNKLNSYITKTNGTMSLFDVPLHFRFYDAS
NGGGGYDMRNLLNNTLMSSNPMKAVTFVENHDTQPGQSLESTVQSWFKPLAYATILTRE
QGYPCVFYGDYYGTSDGKISSYKPIMDKLLNARKVYAYGTQRDYFDHPDIVGWTREGDA
AHAGSGLATLITDGPGGSKWMYVGTSKAGQVWTDKTGNRSGTVTIDANGWGNFWVNKGS
VSVWAK

The amino acid sequence of the mature PcuAmy1-v8 polypeptide is shown below as SEQ ID NO: 62:

ADNGTIMQYFEWYLPNDGAHWNRLNNDAQNLKNVGITAVWIPPAYKGGSSADVGYGVYD
TYDLGEFNQKGTVRTKYGTKSELISAVNNLHAKGIAVYGDVVLNHRMNADATELVDAVE
VDPNNRYVETTSTYQIQAWTQYDFPGRGNTYSSWKWRWYHFDGVDWDQSRGLNRIYKLD
GKDWDWPVDSEYGNYDYLMGADLDFNHPDVVNETKTWGKWFVNTVNLDGVRLDAVKHIK
FDFMRDWVNNVRSTTGKNLFAVGEYWHYDVNKLNSYITKTNGTMSLFDVPLHFRFYDAS
NGGGGYDMRNLLNNTLMSSNPMKAVTFVENHDTQPGQSLESTVQSWFKPLAYATILTRE
QGYPCVFYGDYYGTSDGKISSYKPIMDKLLNARKVYAYGTQRDYFDHPDIVGWTREGDA

AHAGSGLATLITDGPGGSKWMYVGTSKAGQVWTDKTGNRSGTVTIDANGWGNFWVNRRS
VSVWAK

The amino acid sequence of the mature PcuAmy1-v16 polypeptide is shown below as SEQ ID NO: 63:

ADNGTIMQYFEWYLPNDGAHWNRLNNDAQNLKNVGITAVWIPPAYKGGSSADVGYGVYD
TYDLGEFNQKGTVRTKYGTKSELISAVNNLHAKGIAVYGDVVLNHRMNADATELVDAVE
VDPNNRYVETTSTYQIQAWTQYDFPGRGNTYSSWKWRWYHFDGVDWDQSRGLNRIYKLD
GKDWDWPVDSEYGNYDYLMGADLDFDHPDVVNETKTWGKWFVNTVNLDGVRLDAVKHIK
FDFMRDWVNNVRSTTGKNLFAVGEYWHYDVNKLNSYITKTNGTMSLFDVPLHFRFYDAS
NGGGGYDMRNLLNNTLMSSNPMKAVTFVENHDTQPGQSLESTVQSWFKPLAYATILTRE
QGYPCVFYGDYYGTSDGKISSYKPIMDKLLNARKVYAYGTQRDYFDHPDIVGWTREGDA
AHAGSGLATLITDGPGGSKWMYVGTSKAGQVWTDKTGNRSGTVTIDANGWGNFWVNKGS
VSVWAK

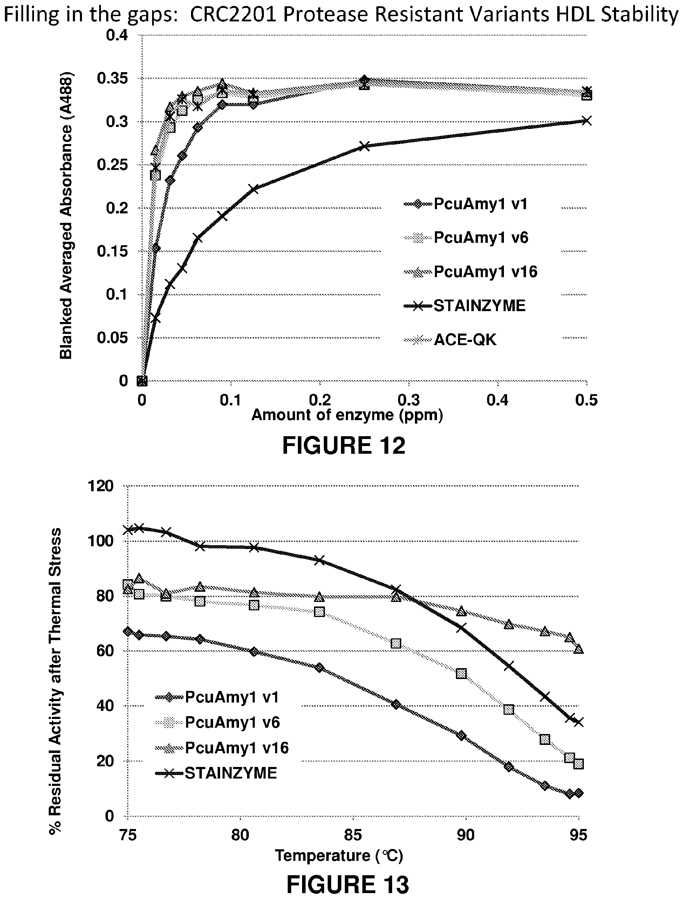

The cleaning performance of PcuAmy1-v1, PcuAmy1-v6, and PcuAmy1-v16, compared to STAINZYME® and ACE-QK was determined using the microswatch assay performed in Example 6. The results are shown in the graph in Figure 12. The PcuAmy1-v6 and PcuAmy1-v16 outperformed PcuAmy1-v1 and STAINZYME® at low doses *(e.g.,* 0.1 ppm enzyme or less).

The thermal stability of PcuAmy1-v1, PcuAmy1-v6, and PcuAmy1-v16, compared to STAINZYME® was determined using the assay described in Example 7, with some slight modifications. The results are shown in the graph in Figure 13. The assays were performed at higher temperatures (75 to 95 degrees C) as PcuAmy1-v1, PcuAmy1-v6, and PcuAmy1v16 were more thermostable than the other tested molecules using the same detergents and enzyme doses.

Although the foregoing compositions and methods have been described in some detail by way of illustration and examples for purposes of clarity of understanding, it will be apparent to those skilled in the art that certain changes and modifications may be made. Therefore, the description should not be construed as limiting the scope of the invention, which is delineated by the appended claims.

## Claims

1. A method for reducing the susceptibility to proteolytic cleavage of a parent α-amylase having a TIM barrel structure and having at least 70% amino acid sequence identity to SEQ ID NO: 3, wherein the parent α-amylase has a residue other than glycine or glutamic acid at a position corresponding to position 333 of SEQ ID NO: 3 by alignment using ClustalW with default parameters, the method comprising substituting the residue at the position corresponding to position 333 to glycine or glutamic acid to produce a variant α-amylase, wherein the variant α-amylase has reduced susceptibility to cleavage compared to the parent α-amylase.

2. The method of claim 1, wherein the parent α-amylase has a threonine at the position corresponding to position 333.

3. The method of claim 1 or claim 2, further comprising substituting the amino acid residue present at a position corresponding to position 335 of SEQ ID NO: 3 by alignment using ClustalW with default parameters in the parent α-amylase to a different amino acid residue.

4. The method of claim 3, wherein the parent α-amylase has an amino acid residue other than serine at the position corresponding to position 335 and wherein the amino acid residue at the position corresponding to position 335 is substituted to a serine.

5. The method of any of the preceding claims, further comprising making one or more of the following mutations in the parent α-amylase:
(i) deleting one or more amino acid residues at positions corresponding to positions 177, 178, 179, and 180 of SEQ ID NO: 3 by alignment using ClustalW with default parameters, e.g. deleting the amino acid residues at positions corresponding to positions 177 and 178;
(ii) substituting the amino acid residue present at a position corresponding to position 186 of SEQ ID NO: 3 by alignment using ClustalW with default parameters, e.g. with a proline; or
(iii) substituting the amino acid residue present at a position corresponding to position 472 of SEQ ID NO: 3 by alignment using ClustalW with default parameters, e.g. with an arginine or a lysine;
wherein the resulting variant has increased detergent stability and/or increased cleaning performance in a detergent composition compared to the parent.

6. The method of any of claims 1-5, further comprising making one or more mutations at positions corresponding to Nl25, Fl52, N205, and G473 of SEQ ID NO: 3 by alignment using ClustalW with default parameters, whereby the variant comprises one of the following combinations of mutations:
(i) N125Y + E186P + T333G + A335S + Q337E + G472K;
(ii) N125Y + F152W + E186P + T333G + A335S + Q337E + G472K;
(iii) N125Y + F152W + E186P + T333G + A335S + Q337E + G472R + G473R;
(iv) N125Y + F152W + E186P + N205D + T333G + A335S + Q337E + G472K;
(v) N125Y + E186P + T333G + A335S + G472K;
(vi) N125Y + F152W + E186P + T333G + A335S + G472K;
(vii) N125Y + F152W + E186P + T333G + A335S + G472R + G473R;
(viii) N125Y + F152W + E186P + N205D + T333G + A335S + G472K;
(ix) N125Y + E186P + T333G + G472K;
(x) N125Y + F152W + E186P + T333G + G472K;
(xi) N125Y + F152W + E186P + T333G + G472R + G473R; or
(xii) N125Y + F152W + E186P + N205D + T333G + G472K.

7. The method of any of the preceding claims, wherein the parent or the variant α-amylase has an amino acid sequence having at least 80%, or at least 90% amino acid sequence identity to SEQ ID NO: 3.

8. A variant α-amylase, wherein the variant has at least 80% amino acid sequence identity to SEQ ID NO: 3 and comprises a glycine or glutamic acid residue at a position corresponding to position 333 of SEQ ID NO: 3 by alignment using ClustalW with default parameters, wherein the variant α-amylase has reduced susceptibility to proteolytic cleavage compared to a parent α-amylase which differs from said variant α-amylase only by having a residue other than glycine or glutamic acid at said position corresponding to position 333 of SEQ ID NO: 3.

9. The variant of claim 8, wherein the parent α-amylase has a threonine at the position corresponding to position 333.

10. A variant α-amylase, wherein the variant has at least 80% amino acid sequence identity to SEQ ID NO: 3 and comprises a glycine or glutamic acid residue at a position corresponding to position 333 of SEQ ID NO: 3 by alignment using ClustalW with default parameters, and comprises a serine residue at a position corresponding to position 335 of SEQ ID NO: 3 by alignment using ClustalW with default parameters, wherein the variant α-amylase has reduced susceptibility to proteolytic cleavage compared to a parent α-amylase which differs from said variant α-amylase only by having a residue other than glycine or glutamic acid at said position corresponding to position 333 of SEQ ID NO: 3 and a residue other than serine at said position corresponding to position 335 of SEQ ID NO: 3.

11. The variant of any of claims 8-10, wherein the variant has an amino acid sequence having at least 90% amino acid sequence identity to SEQ ID NO: 3.

12. A composition comprising the α-amylase of any of claims 8-11 and optionally further comprising a surfactant.

13. The composition of claim 12, further comprising one or more additional enzymes selected from the group consisting of protease, hemicellulase, cellulase, peroxidase, lipolytic enzyme, metallolipolytic enzyme, xylanase, lipase, phospholipase, esterase, perhydrolase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, laccase, metalloproteinase, amadoriase and an α-amylase other than PcuAmyl, or a variant thereof.

14. A recombinant polynucleotide encoding an α-amylase of any of claims 8-11 and optionally having at least 80% nucleic acid sequence identity to the polynucleotide of SEQ ID NO: 1.

15. A host cell comprising the recombinant polynucleotide of claim 14 which is capable of expressing the α-amylase.

## Patentansprüche

1. Verfahren zum Vermindern der Anfälligkeit zu einer proteolytischen Spaltung einer Ausgangs-α-Amylase, die eine TIM-Barrel-Struktur aufweist und mindestens 70% Aminosäuresequenzidentität mit SEQ ID No: 3 aufweist, wobei die Ausgangs-α-Amylase einen anderen Rest als Glycin oder Glutaminsäure an einer Position entsprechend Position 333 von SEQ ID No: 3 durch Alignment unter Verwendung von ClustalW mit Standardparametern aufweist, wobei das Verfahren Substituieren des Rests an der Position entsprechend Position 333 zu Glycin oder Glutaminsäure umfasst, um eine variante α-Amylase zu erzeugen, wobei die variante α-Amylase im Vergleich zu der Ausgangs-α-Amylase verminderte Anfälligkeit für Spaltung aufweist.

2. Verfahren nach Anspruch 1, wobei die Ausgangs-α-Amylase an der Position entsprechend Position 333 ein Threonin aufweist.

3. Verfahren nach Anspruch 2 oder Anspruch 3, umfassend das Substituieren des Aminosäurerests, der sich an einer Position entsprechend der Position 333 von SEQ ID No: 3 befindet, durch Alignment unter Verwendung von ClustalW mit Standardparametern in der Ausgangs-α-Amylase zu einem andere Aminosäurerest.

4. Verfahren nach Anspruch 3, wobei die Ausgangs-α-Amylase einen anderen Rest als Serin an der Position entsprechend der Position 335 aufweist und wobei der Aminosäurerest an der Position entsprechend Position 335 zu einem Serin substituiert wird.

5. Verfahren nach einem der vorhergehenden Ansprüche, ferner umfassend das Erzeugen von einer oder mehreren der folgenden Mutationen in der Ausgangs-α-Amylase:
(i) Deletieren von einem oder mehreren Aminosäureresten an Positionen, die den Positionen 177, 178, 179 und 180 von SEQ ID No: 3 entsprechen, durch Alignment unter Verwendung von ClustalW mit Standardparametern, z.B. Deletieren der Aminosäurereste an Positionen, die den Positionen 177 und 178 entsprechen;
(ii) Substituieren des Aminosäurerests, der sich an einer Position befindet, die Position 186 von SEQ ID No: 3 entspricht, durch Alignment unter Verwendung von ClustalW mit Standardparametern, z.B. mit einem Prolin; oder
(iii) Substituieren des Aminosäurerests, der sich an einer Position befindet, die der Position 472 von SEQ ID No: 3 entspricht, durch Alignment unter Verwendung von ClustalW mit Standardparametern, z.B. mit einem Arginin oder einem Lysin;
wobei die resultierende Variante eine erhöhte Waschmittelstabilität und/oder eine erhöhte Reinigungsleistung in einer Waschmittelzusammensetzung im Vergleich zu dem Ausgangsteil aufweist.

6. Verfahren nach einem der Ansprüche 1 bis 5, ferner umfassend das Erzeugen von einer oder mehreren Mutationen an Positionen, die N125, F152, N205 und G473 von SEQ ID No: 3 entsprechen, durch Alignment unter Verwendung von ClustalW mit Standardparametern, wodurch die Variante eine der folgenden Kombinationen von Mutationen umfasst:
(i) N125Y + E186P + T333G + A335S + Q337E + G472K;
(ii) N125Y + F152W + E186P + T333G + A335S + Q337E + G472K;
(iii) N125Y + F152W + E186P + T333G + A335S + Q337E + G472R + G473R;
(iv) N125Y + F152W + E186P + N205D + T333G + A335S + Q337E + G472K;
(v) N125Y + E186P + T333G + A335S + G472K;
(vi) N125Y + F152W + E186P + T333G + A335S + G472K;
(vii) N125Y + F152W + E186P + T333G + A335S + G472R + G473R;
(viii) N125Y + F152W + E186P + N205D + T333G + A335S + G472K;
(ix) N125Y + E186P + T333G + G472K;
(x) N125Y + F152W + E186P + T333G + G472K;
(xi) N125Y + F152W + E186P + T333G + G472R + G473R; oder
(xii) N125Y + F152W + E186P + N205D + T333G + G472K.

7. Verfahren nach einem der vorhergehenden Ansprüche, wobei der Ausgangsteil oder die variante α-Amylase eine Aminosäuresequenz mit mindestens 80% oder mindestens 90% Aminosäuresequenzidentität zu SEQ ID NO: 3 aufweist.

8. Variante α-Amylase, wobei die Variante mindestens 80% Aminosäuresequenzidentität zu SEQ ID NO: 3 aufweist und einen Glycin- oder Glutaminsäure-Rest an einer Position aufweist, die Position 333 von SEQ ID No: 3 entspricht, durch Alignment unter Verwendung von ClustalW mit Standardparametern, wobei die variante α-Amylase im Vergleich zu einer Ausgangs-α-Amylase, die sich von der varianten α-Amylase lediglich dadurch unterscheidet, dass sie einen anderen Rest als Glycin oder Glutaminsäure an der Position aufweist, die Position 333 von SEQ ID No: 3 entspricht, verminderte Anfälligkeit für proteolytische Spaltung aufweist.

9. Variante nach Anspruch 8, wobei die Ausgangs-α-Amylase ein Threonin an der Position aufweist, die Position 333 entspricht.

10. Variante α-Amylase, wobei die Variante mindestens 80% Aminosäuresequenzidentität zu SEQ ID NO: 3 aufweist und einen Glycin- oder Glutaminsäure-Rest an einer Position umfasst, die Position 333 von SEQ ID No: 3 entspricht, durch Alignment unter Verwendung von ClustalW mit Standardparametern, und einen Serin-Rest an einer Position umfasst, die Position 335 von SEQ ID No: 3 entspricht, durch Alignment unter Verwendung von ClustalW mit Standardparametern, wobei die variante α-Amylase im Vergleich zu einer Ausgangs-α-Amylase, die sich von der varianten α-Amylase lediglich dadurch unterscheidet, dass sie einen anderen Rest als Glycin oder Glutaminsäure an der Position, die Position 333 von SEQ ID No: 3 entspricht, sowie einen anderen Rest als Serin an der Position, die Position 335 von SEQ ID No: 3 entspricht, aufweist, verminderte Anfälligkeit für proteolytische Spaltung aufweist.

11. Variante nach einem der Ansprüche 1 bis 10, wobei die Variante eine Aminosäuresequenz aufweist, die mindestens 90% Aminosäuresequenzidentität zu SEQ ID No: 3 aufweist.

12. Zusammensetzung, umfassend die α-Amylase nach einem der Ansprüche 8 bis 11 und gegebenenfalls ferner umfassend ein Tensid.

13. Zusammensetzung nach Anspruch 12, ferner umfassend ein oder mehrere zusätzliche Enzyme, die ausgewählt sind aus der Gruppe bestehend aus Protease, Hemicellulase, Cellulase, Peroxidase, lipolytischem Enzym, metallolipolytischem Enzym, Xylanase, Lipase, Phospholipase, Esterase, Perhydrolase, Cutinase, Pektinase, Pektatlyase, Mannanase, Keratinase, Reduktase, Oxidase, Phenoloxidase, Lipoxygenase, Ligninase, Pullulanase, Tannase, Pentosanase, Malanase, β-Glucanase, Arabinosidase, Hyaluronidase, Chondroitinase, Laccase, Metalloproteinase, Amadoriase und eine andere α-Amylase als PcuAmyl, oder eine Variante davon.

14. Rekombinantes Polynucleotid, codierend eine α-Amylase nach einem der Ansprüche 8 bis 11 und gegebenenfalls aufweisend mindestens 80% Aminosäuresequenzidentität zu dem Polynucleotid von SEQ ID No: 1.

15. Wirtszelle, umfassend das rekombinante Polynucleotid nach Anspruch 14, das zum Exprimieren der α-Amylase in der Lage ist.

## Revendications

1. Procédé pour réduire la sensibilité à un clivage protéolytique d'une α-amylase parente ayant une structure de tonneau TIM et ayant au moins 70% d'identité de séquence d'acides aminés avec la SEQ ID NO: 3, dans lequel l'a-amylase parente possède un résidu autre que la glycine ou l'acide glutamique à une position correspondant à la position 333 de la SEQ ID NO: 3 par alignement en utilisant ClustalW avec des paramètres par défaut, le procédé comprenant la substitution du résidu à la position correspondant à la position 333 de la SEQ ID NO: 3 en glycine ou acide glutamique pour produire une α-amylase variante, dans lequel l'a-amylase variante a une sensibilité réduite à un clivage comparée à l'a-amylase parente.

2. Procédé selon la revendication 1, dans lequel l'a-amylase parente possède une thréonine à la position correspondant à la position 333.

3. Procédé selon la revendication 1 ou la revendication 2, comprenant en outre la substitution du résidu d'acide aminé présent à une position correspondant à la position 335 de la SEQ ID NO: 3 par alignement en utilisant ClustalW avec des paramètres par défaut dans l'a-amylase parente en un résidu d'acide aminé différent.

4. Procédé selon la revendication 3, dans lequel l'a-amylase parente possède un résidu d'acide aminé autre qu'une sérine à la position correspondant à la position 335 et dans lequel le résidu d'acide aminé à la position correspondant à la position 335 est substitué en une sérine.

5. Procédé selon l'une quelconque des revendications précédentes, consistant en outre à faire une ou plusieurs des mutations suivantes dans l'a-amylase parente:
(i) délétion d'un ou de plusieurs résidus d'acide aminé à des positions correspondant aux positions 177, 178, 179 et 180 de la SEQ ID NO: 3 par alignement en utilisant ClustalW avec des paramètres par défaut, par ex. délétion des résidus d'acide aminé à des positions correspondant aux positions 177 et 178;
(ii) substitution du résidu d'acide aminé présent à une position correspondant à la position 186 de la SEQ ID NO: 3 par alignement en utilisant ClustalW avec des paramètres par défaut, par ex. avec une proline; ou
(iii) substitution du résidu d'acide aminé présent à une position correspondant à la position 472 de la SEQ ID NO: 3 par alignement en utilisant ClustalW avec des paramètres par défaut, par ex. avec une arginine ou une lysine;
dans lequel le variant résultant a une stabilité de détergent augmentée et/ou une performance de nettoyage augmentée dans une composition de détergent comparé au parent.

6. Procédé selon l'une quelconque des revendications 1-5, consistant en outre à faire une ou plusieurs mutations aux positions correspondant à N125, F152, N205 et G473 de la SEQ ID NO: 3 par alignement en utilisant ClustalW avec des paramètres par défaut, en conséquence de quoi le variant comprend une des combinaisons de mutations suivantes:
(i) N125Y + E186P + T333G + A335S + Q337E + G472K;
(ii) N125Y + F152W + E186P + T333G + A335S + Q337E + G472K;
(iii) N125Y + F152W + E186P + T333G + A335S + Q337E + G472R + G473R;
(iv) N125Y + F152W + E186P + N205D + T333G + A335S + Q337E + G472K;
(v) N125Y + E186P + T333G + A335S + G472K;
(vi) N125Y + F152W + E186P + T333G + A335S + G472K;
(vii) N125Y + F152W + E186P + T333G + A335S + G472R + G473R;
(viii) N125Y + F152W + E186P + N205D + T333G + A335S + G472K;
(ix) N125Y + E186P + T333G + G472K;
(x) N125Y + F152W + E186P + T333G + G472K;
(xi) N125Y + F152W + E186P + T333G + G472R + G473R; ou
(xii) N125Y + F152W + E186P + N205D + T333G + G472K.

7. Procédé selon l'une quelconque des revendications précédentes, dans lequel l'a-amylase parente ou variante possède une séquence d'acides aminés ayant au moins 80% ou au moins 90% d'identité de séquence d'acides aminés avec la SEQ ID NO: 3.

8. α-amylase variante, où le variant a au moins 80% d'identité de séquence d'acides aminés avec la SEQ ID NO: 3 et comprend un résidu de glycine ou d'acide glutamique à une position correspondant à la position 333 de la SEQ ID NO: 3 par alignement en utilisant ClustalW avec des paramètres par défaut, où l'a-amylase variante a une sensibilité réduite à un clivage protéolytique comparée à une α-amylase parente qui diffère de ladite α-amylase variante uniquement par le fait d'avoir un résidu autre qu'une glycine ou un acide glutamique à ladite position correspondant à la position 333 de la SEQ ID NO: 3.

9. Variant selon la revendication 8, où l'a-amylase parente possède une thréonine à la position correspondant à la position 333.

10. α-amylase variante, où le variant a au moins 80% d'identité de séquence d'acides aminés avec la SEQ ID NO: 3 et comprend un résidu de glycine ou d'acide glutamique à une position correspondant à la position 333 de la SEQ ID NO: 3 par alignement en utilisant ClustalW avec des paramètres par défaut et comprend un résidu de sérine à une position correspondant à la position 335 de la SEQ ID NO: 3 par alignement en utilisant ClustalW avec des paramètres par défaut, où l'a-amylase variante a une sensibilité réduite à un clivage protéolytique comparée à une α-amylase parente qui diffère de ladite α-amylase variante uniquement par le fait d'avoir un résidu autre qu'une glycine ou un acide glutamique à ladite position correspondant à la position 333 de la SEQ ID NO: 3 et un résidu autre qu'une sérine à ladite position correspondant à la position 335 de la SEQ ID NO: 3.

11. Variant selon l'une quelconque des revendications 8-10, où le variant possède une séquence d'acides aminés ayant au moins 90% d'identité de séquence d'acides aminés avec la SEQ ID NO: 3.

12. Composition comprenant l'a-amylase selon l'une quelconque des revendications 8-11 et optionnellement comprenant en outre un tensioactif.

13. Composition selon la revendication 12, comprenant en outre une ou plusieurs enzymes supplémentaires choisies dans le groupe constitué de: protéase, hémicellulase, cellulase, peroxydase, enzyme lipolytique, enzyme métallolipolytique, xylanase, lipase, phospholipase, estérase, perhydrolase, cutinase, pectinase, pectate lyase, mannanase, kératinase, réductase, oxydase, phénoloxydase, lipoxygénase, ligninase, pullulanase, tannase, pentosanase, malanase, β-glucanase, arabinosidase, hyaluronidase, chondroïtinase, laccase, métalloprotéinase, amadoriase et une α-amylase autre que PcuAmy1 ou un variant de celle-ci.

14. Polynucléotide recombinant codant pour une α-amylase selon l'une quelconque des revendications 8-11 et ayant optionnellement au moins 80% d'identité de séquence d'acides nucléiques avec le polynucléotide de la SEQ ID NO: 1.

15. Cellule hôte comprenant le polynucléotide recombinant selon la revendication 14 qui est capable d'exprimer l'a-amylase.
